(19) Europäisches Patentamt
European Patent Office
Office européen des brevets

(11) EP 2 880 181 B1

(12) EUROPEAN PATENT SPECIFICATION

(45) Date of publication and mention of the grant of the patent:
**19.09.2018 Bulletin 2018/38**

(21) Application number: **13745418.7**

(22) Date of filing: **05.08.2013**

(51) Int Cl.:
***C12Q 1/6883*** *(2018.01)*

(86) International application number:
**PCT/EP2013/066422**

(87) International publication number:
**WO 2014/023704 (13.02.2014 Gazette 2014/07)**

# (54) PROGNOSIS BIOMARKERS IN CARTILAGE DISORDERS

PROGNOSEBIOMARKER BEI KNORPELERKRANKUNGEN

BIOMARQUEURS POUR LE PRONOSTIC DES TROUBLES DU CARTILAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.08.2012 EP 12179393**
**07.08.2012 US 201261680493 P**
**13.03.2013 US 201361778950 P**

(43) Date of publication of application:
**10.06.2015 Bulletin 2015/24**

(73) Proprietor: **Merck Patent GmbH**
**64293 Darmstadt (DE)**

(72) Inventors:
- **LADEL, Christoph, Hubertus**
  **64291 Darmstadt (DE)**
- **BERTON, Alix, Anne, Simone**
  **88437 Sulmingen (DE)**
- **VALSESIA, Armand**
  **1022 Chavannes-Près-Renens (CH)**
- **FARMER, Pierre, Jacques**
  **74500 Evian-les-Bains (FR)**

(74) Representative: **Merck Serono S.A.**
**Intellectual Property**
**Terre Bonne Business Park A2**
**Route de Crassier 15**
**1162 Eysins (CH)**

(56) References cited:
**WO-A2-2008/023063 WO-A2-2009/135218**
**US-A1- 2012 115 137**

- **M. ATTUR ET AL: "Radiographic severity of knee osteoarthritis is conditional on interleukin 1 receptor antagonist gene variations", ANNALS OF THE RHEUMATIC DISEASES, vol. 69, no. 5, 23 November 2009 (2009-11-23), pages 856-861, XP055040487, ISSN: 0003-4967, DOI: 10.1136/ard.2009.113043**
- **KERKHOF H J M ET AL: "Large-scale meta-analysis of interleukin-1 beta and interleukin-1 receptor antagonist polymorphisms on risk of radiographic hip and knee osteoarthritis and severity of knee osteoarthritis", OSTEOARTHRITIS AND CARTILAGE, BAILLIERE TINDALL, LONDON, GB, vol. 19, no. 3, 2 December 2010 (2010-12-02), pages 265-271, XP028152870, ISSN: 1063-4584, DOI: 10.1016/J.JOCA.2010.12.003 [retrieved on 2010-12-10]**
- **ATTUR M ET AL: "391 ASSOCIATION OF INTERLEUKIN-1 RECEPTOR ANTAGONIST (IL-IRN) TTC HAPLOTYPE WITH RADIOGRAPHIC KNEE OA SEVERITY IN META-ANALYSIS", OSTEOARTHRITIS AND CARTILAGE, BAILLIERE TINDALL, LONDON, GB, vol. 18, 1 October 2010 (2010-10-01), page S172, XP027316947, ISSN: 1063-4584 [retrieved on 2010-09-29]**
- **ATTUR M ET AL: "392 INTERLEUKIN-1 RECEPTOR ANTAGONIST GENE VARIATIONS PREDICT THE SEVERITY AND PROGRESSION OF KNEE OSTEOARTHRITIS", OSTEOARTHRITIS AND CARTILAGE, BAILLIERE TINDALL, LONDON, GB, vol. 18, 1 October 2010 (2010-10-01), page S172, XP027316948, ISSN: 1063-4584 [retrieved on 2010-09-29]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KERKHOF H J ET AL: "377 LARGE SCALE META-ANALYSIS OF INTERLEUKIN-1 BETA AND INTERLEUKIN-1 RECEPTOR ANTAGONIST POLYMORPHISMS ON RISK OF RADIOGRAPHIC HIP AND KNEE OSTEOARTHRITIS AND SEVERITY OF KNEE OSTEOARTHRITIS", OSTEOARTHRITIS AND CARTILAGE, BAILLIERE TINDALL, LONDON, GB, vol. 18, 1 October 2010 (2010-10-01), page S166, XP027316933, ISSN: 1063-4584 [retrieved on 2010-09-29]
- WU X ET AL: "390 PROGRESSION OR INITIATION OF RADIOGRAPHIC KNEE OSTEOARTHRITIS AND THE INTERLEUKIN-1 RECEPTOR ANTAGONIST GENE: THE JOHNSTON COUNTY OSTEOARTHRITIS PROJECT", OSTEOARTHRITIS AND CARTILAGE, BAILLIERE TINDALL, LONDON, GB, vol. 18, 1 October 2010 (2010-10-01), pages S171-S172, XP027316946, ISSN: 1063-4584 [retrieved on 2010-09-29]
- BUKOWSKI J F ET AL: "A49II-1 RN POLYMORPHISMS ARE ASSOCIATED WITH RADIOGRAPHIC SEVERITY IN OSTEOARTHRITIS", OSTEOARTHRITIS AND CARTILAGE, BAILLIERE TINDALL, LONDON, GB, vol. 16, 1 September 2008 (2008-09-01), page S34, XP025689762, ISSN: 1063-4584, DOI: 10.1016/S1063-4584(08)60095-3 [retrieved on 2008-09-01]
- ELLSWORTH J L ET AL: "Fibroblast growth factor-18 is a trophic factor for mature chondrocytes and their progenitors", OSTEOARTHRITIS AND CARTILAGE, BAILLIERE TINDALL, LONDON, GB, vol. 10, no. 4, 1 April 2002 (2002-04-01), pages 308-320, XP002374609, ISSN: 1063-4584, DOI: 10.1053/JOCA.2002.0514

## Description

### Field of Invention

The present invention relates, generally, to pharmacogenetics, more specifically to genetic markers associated with severity of a cartilage disorder or progression of said cartilage disorder. The present invention more particularly relates to human genes, which can be used for the diagnosis and treatment of cartilage disorders.

### Background of the invention

Cartilage disorders broadly refer to diseases characterized by degeneration of metabolic abnormalities in the connective tissues which are manifested by pain, stiffness and limitation of motion of the affected body parts. These disorders can be due to pathology or can be the result of trauma or injury. Among others, cartilage disorders include osteoarthritis (OA) and cartilage injury (inclusive sports injuries of cartilage and joint, or surgical injuries such as microfracture(s)). Mature cartilage has limited ability to repair itself, notably because mature chondrocytes have little potential for proliferation and due to the absence of blood vessels. In addition, cartilage is not well nutrified and has a low oxygen pressure. Replacement of damaged cartilage, in particular articular cartilage, caused either by injury or disease is a major challenge for physicians, and available surgical treatment procedures are considered not completely predictable and effective for only a limited time. Therefore, the majority of younger patients either does not seek treatment or are counseled to postpone treatment for as long as possible. When treatment is required, the standard procedure is age dependent and varies between total joint replacement, transplantation of pieces of cartilage or marrow stimulating technique (such as microfracture). Microfracture is a common procedure that involves penetration of the subchondral bone to stimulate cartilage deposition by bone marrow derived stem cells. However, it has been shown that this technique does not repair sufficiently the chondral defect and the new cartilage formed is mainly fibrocartilage, resulting in inadequate or altered function and biomechanics. Indeed, fibrocartilage does not have the same durability and may not adhere correctly to the surrounding hyaline cartilage. For this reason, the newly synthesized fibrocartilage may breakdown more easily (expected time frame: 5-10 years).

For patients with osteoarthritis, non-surgical treatment consists notably of physical therapy, lifestyle modification (e.g. reducing activity), supportive devices, oral and injected drugs (e.g. non-steroidal anti-inflammatory drugs), and medical management. Once these treatments fail, surgery, such as joint replacement, is the main option for the patients. Such an option can provide a reduction in symptoms that are generally only short lived. Tibial or femoral osteotomies (cutting the bone to rebalance joint wear) may reduce symptoms, help to maintain an active lifestyle, and delay the need for total joint replacement. Total joint replacement can provide relief for the symptom of advanced osteoarthritis, but generally requires a change in a patient's lifestyle and/or activity level.

At that time, drug treatments on the market are mainly directed to pain relief. There is not yet commercially available treatment that restores the cartilage damages (see Lotz, 2010).

Fibroblast Growth factor 18 (FGF-18) is a member of the FGF family of proteins, closely related to FGF-8 and FGF-17. It has been shown that FGF-18 is a proliferative agent for chondrocytes and osteoblasts (Ellsworth et al., 2002; Shimoaka et al., 2002). FGF-18 has been proposed for the treatment of cartilage disorder such as osteoarthritis and cartilage injury either alone (WO2008/023063) or in combination with hyaluronic acid (WO2004/032849).

Sprifermin, which is a truncated form of human FGF-18, is being investigated in clinical trials for treatment of both osteoarthritis and cartilage injury (for more details see for instance NCT01033994, NCT00911469 and NCT01066871). The current dosing regimen for spriferminе is once weekly for 3 weeks (one treatment cycle), the drug being administered via intraarticular injections. This treatment cycle can be repeated. This dosing regimen has been described in WO2008023063.

At that time, OA and cartilage injury treatments with any drug, notably anabolic drug such as sprifermin, during clinical trials, are provided to patients without predictive information on the response, *i.e.* without knowledge on whether the treatment will likely be highly effective, moderately effective or show only little or no effect. Currently, for instance, numerous treated patient population exhibit an intermediate/high response to treatment according to the WOMAC scores with FGF18 after at least one treatment cycle, however, some others either do not respond to said treatment or respond while presenting high WOMAC score compared to control.

The present invention describes genetic markers that are prognostic of the disorder severity or of the disorder progression. Such markers are useful for identifying, through genetic screening prior to the treatment, subgroups of patients that are more likely to exhibit less severe form of cartilage disorder. Such prognostic information may thus be clinically useful to guide medical decisions.

**Summary of the invention**

The present invention is directed to a method of prognosing disorder severity, or disorder progression, in a subject having osteoarthritis, the method comprising the steps of:

a. Determining, from a nucleic acid sample, the presence of the genotype G/G at IL-1RN rs9005 and T/T at IL-1RN rs31595;
b. Prognosing from the result of step a. a less severe form of osteoarthritis.

The present invention also describes a method of prognosing disorder severity, or disorder progression, in a subject having osteoarthritis, the method comprising the steps of:

a. Determining, from a nucleic acid sample, the absence of the genotype G/G at IL-1RN rs9005 and T/T at IL-1RN rs31595;
b. Prognosing from the result of step a. a more severe form of osteoarthritis.

It is to be understood that in any of the methods or uses mentioned herein, before determining the genotype at one locus, it is needed to obtain a nucleic acid sample (or a test sample) of said subject, for instance by blood or saliva collecting. Alternatively the test sample is selected from bucall cells, urine or stool. Preferably, the nucleic acid sample is a DNA sample. Further, it is also to be understood that any of the methods or uses mentioned herein are performed in vitro, and not on the animal or human body.

**Definitions**

- The term "drug" or "therapeutic compound" means a compound that is currently used or that could be used in therapy of a cartilage disorder.
- The term "anabolic compound" or "anabolic drug" is to be understood as a compound or a drug that has anabolic effect on the cartilage, preferably leading to cartilage repair. Among such compound can be listed FGF-18 compound (as defined herein), BMP-2 (e.g. reference Uniprot P12643), BMP-7 (e.g. reference Uniprot P18075), GDF-5 (e.g. reference Uniprot P43026), FGFβ (e.g. reference Uniprot P09038), FGF-8 (e.g. reference Uniprot P55075), FGF-9 (e.g. reference Uniprot P31371), SOX-9 enhancers (e.g. reference Uniprot P48436 for SOX-9) or TGFβ (e.g. reference Uniprot P01137) and any variants thereof.
- The term "FGF-18 compound" or "FGF-18", as used herein, is intended to be a protein maintaining at least one biological activity of the human FGF-18 protein. FGF-18 may be native, in its mature form, or a truncated form thereof. Biological activities of the human FGF-18 protein include notably the increase in osteoblastic activity (see WO98/16644) or in cartilage formation (see WO2008/023063). Native, or wild-type, human FGF-18 is a protein expressed by chondrocytes of articular cartilage. Human FGF-18 was first designated zFGF-5 and is fully described in WO98/16644. SEQ ID NO:1 corresponds to the amino acid sequence of the native human FGF-18, with a signal peptide consisting of amino acid residues 1 (Met) to 27(Ala). The mature form of human FGF-18 corresponds to the amino acid sequence from residue 28(Glu) to residue 207(Ala) of SEQ ID NO: 1 (180 amino acids). The term also includes fusion protein, wherein FGF-18 protein is coupled with a heterologous protein or a chemical compound. FGF-18 may be produced by recombinant methods, such as taught by the application WO2006/063362. Depending on the expression systems and conditions, FGF-18 is expressed in a recombinant host cell with a starting Methionine (Met) residue or with a signal sequence for secretion. When expressed in prokaryotic host, such as in E. coli, FGF-18 contains an additional Met residue in N-terminal of its sequence. For instance, the amino acid sequence of human FGF-18, when expressed in E.coli, starts with a Met residue in N-term (position 1) followed by residues 28 (Glu) to residue 207 (Ala) of SEQ ID NO: 1.
- The term "truncated form" of FGF18, as used herein, refers to a protein which comprises or consists of residues 28(Glu) to 196(Lys) of SEQ ID NO: 1. Preferably, the truncated form of FGF-18 protein is the polypeptide designated "trFGF-18" (170 amino acids), which starts with a Met residue (in N-terminal) followed by amino acid residues 28 (Glu) -196 (Lys) of the wild-type human FGF-18. The amino acid sequence of trFGF-18 is shown in SEQ ID NO:2 (amino acid residues 2 to 170 of SEQ ID NO:2 correspond to amino acid residues 28 to 196 of SEQ ID NO:1). trFGF-18 is a recombinant truncated form of human FGF-18, produced in E.coli (see WO2006/063362). The International Nonproprietary Name (INN) for this particular form of FGF-18 is sprifermin. Sprifermin has been shown to display similar activities as the mature human FGF-18, e.g. it increases chondrocyte proliferation and cartilage deposition leading to repair and reconstruction for a variety of cartilaginous tissues (see WO2008/023063).

- "Cartilage disorder", as used herein, encompasses disorders resulting from damages due to injury, such as traumatic injury, chondropathy or arthritis. Examples of cartilage disorders that may be treated by the administration of the FGF-18 formulation described herein include, but are not restricted to, arthritis, such as osteoarthritis, cartilage injury, fractures affecting joint cartilage or surgical procedures with impact on joint cartilage (e.g. Microfracture). Degenerative diseases/disorders of the cartilage or of the joint, such as chondrocalcinosis, polychondritis, relapsing polychondritis, ankylosing spondylitis or costochondritis are also encompassed by this wording. The International Cartilage Repair Society has proposed an arthroscopic grading system to assess the severity of the cartilage defect: grade 0: (normal) healthy cartilage, grade 1: the cartilage has a soft spot or blisters, grade 2: minor tears visible in the cartilage, grade 3: lesions have deep crevices (more than 50% of cartilage layer) and grade 4: the cartilage tear exposes the underlying (subchronal) bone (see for instance page 13 of http://www.cartilage.org/_files/contentmanagement/ICRS_evaluation.pdf).
- The term "Osteoarthritis" is used to intend the most common form of arthritis. The term "osteoarthritis" encompasses both primary osteoarthritis and secondary osteoarthritis (see for instance The Merck Manual, 17th edition, page 449). The most common way of classifying/grading osteoarthritis is the use of the Kellgren-Lawrence radiographic grading scale (see table below). Osteoarthritis may be caused by the breakdown of cartilage. Bits of cartilage may break off and cause pain and swelling in the joint between bones. Over time, the cartilage may wear away entirely, and the bones will rub together. Osteoarthritis can affect any joint but usually concerns hands and weight-bearing joints such as hips, knees, feet, and spine. In a preferred example, the osteoarthritis may be knee osteoarthritis or hip osteoarthritis. Osteoarthritis is one of the preferred cartilage disorders that can be treated by administering the FGF-18 compounds.

Kellgren-Lawrence Radiographic Grading Scale of Osteoarthritis is described as follow:

| Grade of Osteoarthritis | Description |
|---|---|
| 0-None | No radiographic findings of osteoarthritis |
| 1-Doubtful | Doubtful narrowing of joint space and possible osteophytic lipping |
| 2-Minimal | Definite osteophytes, definite narrowing of joint space |
| 3-Moderate | Moderate multiple osteophytes, definite narrowing of joints space, some sclerosis and possible deformity of bone contour |
| 4-Severe | Large osteophytes, marked narrowing of joint space, severe sclerosis and definite deformity of bone contour |

- The term "cartilage injury" as used herein is a cartilage disorder or cartilage damage resulting notably from a trauma. Cartilage injuries can occur notably after traumatic mechanical destruction, notably further to an accident or surgery (for instance microfracture surgery). This term "cartilage injury" also includes chondral or osteochondral fracture, damage to meniscus, and the term microfracture. Also considered within this definition is sport-related injury or sport- related wear of tissues of the joint.
- The term "disease severity" is related to the grade of the cartilage disorder: the higher, the more severe. For instance, a grade 3 according to Kellgren-Lawrence grading system is more severe than a grade 2 according to the same grading system.
- The term AIR (acute inflammatory reaction) as used herein is defined as follow. Within 1 to 7 day-period, preferably within 3 day-period, following the intra-articular injection of FGF-18 compound in the target knee both, the following criteria must be fulfilled:

  - Self-reported swelling (synovial fluid effusion)
  - Pain increase by 30 mm on 100 mm Visual Analogue Scale (VAS)

- An "allele" is a particular form of a gene, genetic marker or other genetic locus, that is distinguishable from other forms of the gene, genetic marker or other genetic locus; e.g. without limitation by its particular nucleotide sequence. The term allele also includes for example without limitation one form of a single nucleotide polymorphism (SNP). An individual can be homozygous for a certain allele in diploid cells; i.e. the allele on both paired chromosomes is identical; or heterozygous for said allele; i.e. the alleles on both paired chromosomes are not identical.
- The term a "genetic marker", "biomarker" or "marker" refers to an identifiable polymorphic (genetic) locus. An example without limitation of a genetic marker is a single nucleotide polymorphism (SNP).
- A "single nucleotide polymorphism (SNP)" is a DNA sequence variation occurring when a single nucleotide - A (for Adenine), T (for Thymine), C (for Cytosine), or G (for Guanine)- in the genome (or other sequence shared between individuals of a species) differs between individuals of a species (or between paired chromosomes in an individual). A SNP is frequently preceded by and followed by highly conserved sequences in the population of interest and thus the location of a SNP is typically made in reference to a consensus nucleic acid sequence of thirty to sixty nucleotides that bracket the genetic marker locus, which is sometimes referred to as a context sequence for the SNP. The SNPs that were analyzed by the present inventors in connection with treatment of cartilage disorder with sprifermin are those shown in Table 1.

- A "genotype" as used herein refers to the combination of both alleles of a genetic marker, e.g. without limitation of a SNP, on a single genetic locus on paired (homologous) chromosomes in an individual. "Genotype" as used herein also refers to the combination of alleles of more than one genetic loci, e.g. without limitation of SNPs, on a pair or more than one pair of homologous chromosomes in an individual.
- The term "Haplotype" refers to variants or alleles from distinct markers (e.g. SNPs) that are co-located on the same chromosome. SNP genotype data, as measured from SNP arrays or Taqman assays, are unphased (i.e. the chromosome's parent of origin is unknown for each allele). Computational methods (Browning et Browning, 2011) use information across individuals to estimate (i.e. infer) haplotype phase from genotype data.
- The term "Genotyping" refers to a process for determining a genotype of an individual, either for a single SNP or many SNPs.
- "Locus" or "genetic locus" refers to a specific location on a chromosome or other genetic material. For instance, IL-1RN rs9005 is a locus and can be called, in the frame of the present invention, either "IL-1 RN rs9005" or "locus IL-1 RN rs9005". The same applies to IL-1 RN rs315952. As self evident for the skilled person, from NCBI database for these SNPs, the genotype to be determined at both IL-1RN rs9005 and IL-1RN rs315952, is the one in position 27 of each of these loci, i.e. position 27 of SEQ ID NO:6 and position 27 of SEQ ID NO:7.
- The term "SNP1" in the context of the present invention, is position 27 of SEQ ID NO: 6, also identified as rs9005 in NCBI database. SEQ ID NO. 6 is a portion of genomic nucleic acid sequence of interleukin 1 receptor antagonist (IL-1RN). The terms "IL-1RN rs9005", "rs9005" or "SNP1" are used interchangeably.
- The term "SNP2" refers to position 27 of SEQ ID NO. 7 identified as being rs315952 in NCBI database. SEQ ID NO. 7 is a portion of genomic nucleic acid sequence of IL-1RN. The terms "IL-1RN rs315952", "rs315952" or "SNP2" are used interchangeably.
- The term "probe" or "primer" refers to an oligonucleotide, i.e. a nucleic acid or a nucleic acid derivative; including without limitation a locked nucleic acid (LNA), peptide nucleic acid (PNA) or bridged nucleic acid (BNA); that is usually between 5 and 100 contiguous bases in length, and most frequently between 5-40, 5-35, 5-30, 5-25, 5-20, 5-15, 5-10, 10-50, 10-40, 10-30, 10-25, 10-20, 15-50, 15-40, 15-30, 15-25, 15-20, 20-50, 20-40, 20-30 or 20-25 contiguous bases in length. The sequence of a probe/a primer can be designed to specifically hybridize to one of the allelic forms of a genetic marker; such oligonucleotides are referred to as allele-specific probes. If the genetic marker is a SNP, the complementary allele for that SNP can occur at any position within an allele-specific probe. Other probes/primers useful in practicing the invention specifically hybridize to a target region adjacent to a SNP with their 3' terminus located one to less than or equal to about 10 nucleotides from the genetic marker locus, preferably ≤ about 5 nucleotides. Such probes/primers hybridizing adjacent to a SNP are useful in polymerase-mediated primer extension methods and are referred to herein as "primer-extension oligonucleotides." In a preferred embodiment, the 3'-terminus of a primer-extension oligonucleotide is a deoxynucleotide complementary to the nucleotide located immediately adjacent a SNP.
- The term "Polymorphism" refers of two or more alternate forms (alleles) in a population of a genetic locus that differ in nucleotide sequence or have variable numbers of repeated nucleotide units. Polymorphisms occur in coding regions (exons), non-coding regions of genes or outside of genes (intergenic regions). The different alleles of a polymorphism typically occur in a population at different frequencies, with the allele occurring most frequently in a selected population sometimes referenced as the "major" or "wild type" allele. Diploid organisms may be homozygous or heterozygous for the different alleles that exist. A biallelic polymorphism has two alleles.
- The term "Epistasis" is generally used to define the interaction between genes. Epistasis was first defined by Bateson (Bateson et Mendel, 1909) to describe a masking effect whereby a variant or allele at one locus prevents the variant at another locus from manifesting its effect. However the scientific literature provides many different definitions (Phillips, 1998; Cordell, 2002). Herein, epistasis was tested as the statistical interaction between genotypes from two distinct SNPs. This is similar to the definition proposed by Fisher in 1918 (Fisher, 1918), i.e. a deviation from additivity in the effect of alleles at different loci with respect to their contribution to a phenotype.
- "WOMAC total scores" or "WOMAC scores" ("WOMAC" for "Western Ontario and McMaster Universities Osteoarthritis Index") measure pain (WOMAC pain score), function (WOMAC function score) and stiffness (WOMAC stiffness score). When applied to assessing pain and dysfunction associated with cartilage injury, it consists of a questionary containing 24 items divided into 3 subscales (5 items for Pain, 2 items for Stiffness and 17 items for Physical Function)(see Bellamy et al., 1988; Wolfe, 1999). It is a well-known instrument, widely used notably in assessment of the OA severity.

- Cartilage volume measurements were performed through magnetic resonance imaging (MRI) measurements, including Total volume of cartilage (LFTC + MFTC), Lateral volume of cartilage (also referred as LFTC: lateral femoro-tibial compartment), Medial volume of cartilage (also referred as MFTC: medial femoro-tibial compartment), and new total average cartilage thickness.
- The term "baseline" means before treatment (*i.e.* at study entry). It refers notably to clinical variables, such as, but not limited to, the cartilage volume and WOMAC total score of one given patient at study entry (*i.e.* before treatment with FGF-18 compound or placebo).
- "Sensitives" are patients that exhibit a response to treatment of a cartilage disorder with a drug, preferably an anabolic drug, such as FGF-18. Preferably, sensitive patients (or patients showing sensitivity to treatment) exhibit notably a higher increase in total cartilage volume than placebo treated subjects, i.e. they show cartilage repair. In addition, sensitive patients exhibit at least similar improvement in WOMAC total scores than placebos. The terms "Super-sensitives", "intermediate-sensitives" and "Non-sensitives" refer to the different groups of patients depending notably on the increase of the cartilage volume following drug treatment. Super-sensitive display a high response (i.e. high cartilage repair) to treatment with said drug, intermediate-sensitive display a good or intermediate response (i.e. good or intermediate cartilage repair) to treatment with said drug, and non-sensitives display no or low response to treatment with said drug. Both super-sensitive and sensitive subjects have similar improvement in WOMAC total score than placebos. Conversely non-responders have significantly smaller improvement in WOMAC total score than placebos. The term "super-sensitives" or "high-sensitives" are used interchangeably. It is noted that super-sensitives have been shown to present higher risk of AIR events.

More particularly, the terms "Intermediate-sensitives", "Super-sensitives", and "Non-sensitives" include, but are not limited to, the different groups of patients depending on the increase of the cartilage volume and improvement of WOMAC total score, following drug treatment.

The proposed criteria for sensitives are the following:

1. Positive cartilage increase (between +10 and +100mm$^3$) compared to baseline,
2. Cartilage increase change significantly higher than change in placebo (e.g. as tested with a linear model adjusting for BMI, KL grade, sex and age and with alpha = 5%),
3. WOMAC score improvement, i.e. diminution, (e.g. more than 5 points reduction) compared to baseline,
4. WOMAC score change not significantly higher than change in placebo (e.g. as tested with a linear model adjusting for BMI, KL grade, sex and age and with alpha = 5%).

The proposed criteria for super-sensitives are the same than for sensitives, but with cartilage increase greater than 100mm$^3$ (criterion #1) compared to baseline.

Non-sensitives can be defined as subjects not fulfilling criteria #1 or #2 and not fulfilling criteria #3 or #4.

Intermediate sensitives display a good or intermediate response (or a good or intermediate sensitivity) to treatment with said drug (see above criteria; for instance, median change when treated with an FGF-18 compound: +84.81mm$^3$ total cartilage volume increases compared to baseline; median change: -20 points on the WOMAC total score compared to baseline; and non-significant difference in WOMAC total score compared to placebos). Super-sensitives display a high response to treatment with said drug (see above criteria; for instance, median change when treated with an FGF-18 compound: +119.46mm$^3$ total cartilage volume increase compared to baseline, representing a +40.85% increase (i.e. benefit) compared to sensitive subjects, median change: -10 points on the WOMAC total score, both compared to baseline, and non-significant difference in WOMAC total score compared to placebos). Non-sensitives display no or low response to treatment with said drug (see above criteria; for instance, median change when treated with an FGF-18 compound: significantly smaller increase in total cartilage volume compared to placebos (difference between medians: -106.64 mm$^3$);total cartilage; little improvement (median change: -1 point) in WOMAC total scores compared to baseline, and significant difference in WOMAC total score compared to placebos).

- The "response", or "sensitivity" to a drug treatment is to be understood as 1 year after the first injection and measured as 1) increase of cartilage volume, measured owing to MRI or X-Ray for instance, 2) decrease of WOMAC total scores, and 3) changes in WOMAC total scores not significantly higher than those from placebos (refer also to the definition of "sensitive").
- A "prognostic biomarker" is informative about the subject condition, including and not limited to disease evolution, disease severity or disease outcome, regardless of any therapy. A "predictive biomarker" is informative about the effect of a received therapy, including and not limited to efficacy and safety outcome. The prognostic and predictive definitions are not mutually exclusive thus a biomarker can be both prognostic and predictive.
- As used herein, the term "MAD" means Multiple Ascending Dose. When this acronym is followed by a figure, the figure corresponds to the dose at which the drug has been injected during treatment. For instance MAD100 refers to a treatment during which a patient received 100 mcg of said drug (e.g. FGF-18 compound) per injection. The acronym "PL" (and "MADPL") refers to placebo.

- The term "storage device", as used herein, is intended to include any suitable computing or processing apparatus or other device configured or adapted for storing data or information. Examples of electronic apparatus include stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media.
- As used herein, the term "stored" refers to a process for encoding information on the storage device. Those skilled in the art can readily adopt any of the presently known methods for recording information on known media to generate manufactures comprising expression level information.

## Detailed description of the invention

There is a need to prognosing disease severity, or disease progression, in a subject having a cartilage disorder. Biomarkers that predict disease susceptibility, disease severity, or disease progression, are therefore important. These biomarkers are typically referred as prognostic biomarkers and are independent of treatment received by patients. Prognostic biomarkers can also be predictive of the response to drug treatment and conversely predictive biomarkers can also be prognostic.

To optimize the treatment of said patients, it is important to identify biomarkers that could be used as predictors of the disease severity. Said predictors could also be useful to identify high-risk groups either being non-sensitives or on the contrary super-sensitives to a drug treatment. For instance, if one patient having osteoarthritis is known to be at high risk for non-responding to a given drug, preferably an anabolic drug, the physician may decide not to propose said drug to said patient. On the contrary, if one patient having osteoarthritis is known to be at high risk for being super-sensitive to the drug treatment, the physician may decide to adapt the dose regimen, in order to lower the dose of said drug to be administered to said patient. Such predictive information may be clinically useful to guide decisions, and notably on the timing of joint replacement surgery if needed.

The surprising finding of the present invention is based on a study aimed at identifying potential biomarkers associated with disease severity, as well as with the risk of being sensitive or non-sensitive to a drug treatment, preferably an anabolic drug such as sprifermin. The biomarkers used in this study were composed of both candidate genetic markers (see Table 1) and less than 1 million SNPs covering the human genome with a median marker spacing of 680 bases. The association between genetic markers and disease severity or clinical response variables was assessed. The rationale behind this type of analysis was to identify biomarkers that could be prognostic of disease severity, in a patient having a cartilage disorder or predictive of the clinical outcome, for a patient to be treated with a drug, preferably an anabolic drug. These SNPs could be used to stratify and target specific patient populations.

The inventors have surprisingly found an association with certain biomarkers (or SNPs) and disease severity or outcome as well adverse effects of drug therapy. Of special interest are the SNPs IL-1RN rs9005 and rs315952, both located in the IL1RN gene (see Figure 1).

These biomarkers have been described in the literature, as being possibly related to disease severity and progression in OA patients (see for instance WO2009/135218, US2012115137 or Attur et al., 2010), using a haplotype (so-called C-T-A haplotype) that includes rs419598 (C), rs315952 (T) and rs9005 (A). WO2009/135218 also discloses that subject having at least the genotype (or pair of alleles) G/G at IL-1RN rs9005 and/or T/T or C/C at IL-1RN rs315952 may be predisposed to severe disease progression, whereas subject having at least the genotype T/T at IL-1RN rs315952 and/or A/A or G/A at IL-1RN rs9005 may be protected from progression to severe disease. Similarly, US2012115137 proposes that subject having the genotype T at IL-1RN rs419598, G/G at IL-1RN rs9005 and T/T at IL-1RN rs315952 may be predisposed to severe disease progression. Interestingly, although the literature shows that the C-T-A haplotype is needed to possibly predict the risk of having and developing a severe disease, it is a finding of the present invention that only two of these biomarkers, i.e. rs9005 and rs315952, are sufficient to identify patients having less risk of having and developing a severe form of OA, but are also strongly correlated with responsiveness to a drug treatment, such as an anabolic drug. They are not only sufficient to identify such patients, but also more efficient for said prognosis, than the C-T-A haplotype. The third SNP, i.e. rs419598, does not appear being further involved in the observed phenotype, although described, in the prior art, as being linked to the two other SNPs. Another difference between the C-T-A haplotype and the combination from rs9005 and rs315952, is that the former corresponds to additive effects from allele located on the same phase (same inherited chromosome) while the latter corresponds to epistatic effects (i.e. interaction between alleles from either the same phase or different phase).

In particular, it was found that a genotype T/T of the biomarker rs315952 together with G/G of the biomarker rs9005 is associated with a less severe form of osteoarthritis osteoarthritis (i.e. less severe OA form at baseline and less severe OA development). Subjects from a placebo group, bearing this genotype combination, have significantly higher cartilage growth and significantly higher improvement in WOMAC scores compared to placebos from other genotype combinations. Additional analyses, at baseline, demonstrated that subjects (from any dose regimen during treatment with sprifermin, including placebos) bearing this IL-1RN rs9005 G/G and IL-1RN rs315259 T/T genotype were protected against being classified as Kellgren-Lawrence grade 3, thus protected as having a more advanced/severe osteoarthritis condition.

The present invention is thus directed to a method of prognosing disorder severity, in a subject having osteoarthritis, the method comprising the steps of:

a. Determining, from a nucleic acid sample, the genotype at both loci IL-1RN rs9005 and IL-1RN rs315952;
b. Prognosing from the result of step a disorder severity.

Before determining the genotype at one locus, it is needed to obtain a nucleic acid sample of said subject, for instance by blood or saliva collecting. Preferably, the nucleic acid sample is a DNA sample. Thus, the present invention is directed to a method of prognosing disorder severity, in a subject having osteoarthritis the method comprising the steps of:

a. Determining, from a nucleic acid sample, the genotype at both loci IL-1RN rs9005 and IL-1RN rs315952;
b. Prognosing from the result of step b disorder severity.

According to said method, the presence of the genotype G/G at IL-1RN rs9005 and T/T at IL-1RN rs315952 is predictive of a less severe form of osteoarthritis. It follows that patients having this genotype will also be predisposed to less severe disease/disorder progression. To the contrary, the absence of the genotype G/G at IL-1RN rs9005 and T/T at IL-1RN rs315952 is predictive of a more severe form of osteoarthritis.

The present disclosure is also directed to a method of determining disorder severity, or disorder progression, in a human subject having a cartilage disorder, the method comprising the steps of: (a) subjecting a test sample from said subject, diagnosed as having a cartilage disorder, to at least one genotyping assay that determines the genotypes of at least two loci, wherein said at least two loci are: (i) SNP1 and (ii) SNP2, (b) determining the genotypes of said at least two loci; (c) determining from the result of steps (a) and (b) disorder severity, or disorder progression for said subject.

According to said method, the presence of the genotype G/G at IL-1RN rs9005 (SNP1) and T/T at IL-1RN rs315952 (SNP2) is predictive of a less severe form of cartilage disorder. It follows that patients having this genotype will also be predisposed to less severe disease/disorder progression. Herein also disclosed is an assay to determine disorder severity, or disorder progression, in a human subject having a cartilage disorder, the assay comprising: (a) subjecting a test sample from said human subject, diagnosed as having a cartilage disorder, to at least one genotyping assay that determines the genotypes of at least two loci, wherein said at least two loci are: (i) SNP1 and (ii) SNP2, (b) determining the genotypes of said at least two loci; (c) determining from the result of steps (a) and (b) disorder severity, or disorder progression for said subject.

According to said method, the presence of the genotype G/G at IL-1RN rs9005 (SNP1) and T/T at IL-1RN rs315952 (SNP2) is predictive of a less severe form of cartilage disorder. It follows that patients having this genotype will also be predisposed to less severe disease/disorder progression. Surprisingly, it has also been found by the present inventors that the alleles "A" of the biomarker rs9005 together with "C" of the biomarker rs315952 are associated with a better response to treatment with a drug, preferably an anabolic drug, such as sprifermin, in subjects afflicted with cartilage disorder. These subjects are called super-sensitives or high-sensitives.

On the contrary, it has also surprisingly been found by the present inventors that the genotype rs315952 T/T together with rs9005 G/G is associated with an absence of, or low, response to treatment with a drug, preferably an anabolic drug, such as sprifermin, in subjects afflicted with cartilage injury. These subjects are called non-sensitives.

Therefore, it is a finding of the inventors that polymorphic loci IL-1RN rs9005 and IL-1RN rs315952 can be used in combination as predictive biomarkers of responsiveness of one subject to a drug treatment. In a particular embodiment, the subject will be predicted to be non-sensitive to the drug treatment if he has the genotype IL-1RN rs9005 G/G together with IL-1RN rs315952 T/T. On the contrary, the subject will be predicted to be super-sensitive (or a high-sensitive) to the drug treatment if he has the genotype IL-1RN rs9005 A/G or A/A together with IL-1RN rs315952 T/C or C/C. In any other case (i.e. G/G at rs9005 together with T/C or C/C at rs315952 or A/G or A/A at rs9005 and T/T at rs315952), the patient will be predicted to be sensitive (or intermediate-sensitive) to the drug treatment.

These two biomarkers rs315952 and rs9005 (also called bi-markers when in combination) are therefore not only prognostic but more important predictive of the response of the patients to a drug treatment.

Also disclosed herein is a method of predicting sensitivity to a drug prior to drug administration in a subject having a cartilage disorder, the method comprising the steps of:

a. Determining, from a nucleic acid sample, the genotype at both loci IL-1RN rs9005 and IL-1RN rs315952; and
b. Predicting from the result of step a high, intermediate, low or no sensitivity of said subject to said drug.

Before determining the genotype at one locus, it is needed to obtain a nucleic acid sample of said subject, for instance by blood or saliva collecting. Preferably, the nucleic acid sample is a DNA sample. Thus, herein disclosed is a method of predicting sensitivity to a drug prior to drug administration in a subject having a cartilage disorder, the method comprising the steps of:

a. Obtaining a nucleic acid sample of said subject
b. Determining, from said a nucleic acid sample, the genotype at both loci IL-1RN rs9005 and IL-1RN rs315952;
c. Predicting from the result of step b high, intermediate, low or no sensitivity of said subject to said drug.

According to said method, the presence of the genotype G/G at IL-1RN rs9005 and T/T at IL-1RN rs315952 is predictive of low or no sensitivity to said drug. The patient will thus be predicted to be non-sensitive. On the contrary, the presence of the genotype(s) selected from the group consisting of (1) IL-1RN rs9005 G/G and IL-1RN rs315952 T/C or C/C, and (2) IL-1RN rs9005 A/G or A/A and IL-1 RN rs315952 T/T, T/C or C/C is predictive of sensitivity to said drug. In particular, the presence of the genotype A/A or A/G at IL-1RN rs9005 and C/C or C/T at IL-1 RN rs315952; is predictive of high sensitivity (high response) to said drug. These patients will thus be predicted to be super-sensitive. It follows from said prediction, that the doctor can easily select only those patients that are predicted to be sensitives, including super-sensitives.

Also disclosed herein is an assay to determine sensitivity to a drug treatment or to determine a treatment regimen with a drug treatment, the assay comprising: (a) subjecting a test sample from a human subject, diagnosed as having a cartilage disorder, to at least one genotyping assay that determines the genotypes of at least two loci, wherein said at least two loci are: (i) SNP1 and (ii) SNP2, (b) determining the genotypes of said at least two loci; (c) selecting a patient as being sensitive to a treatment with said drug when at least one of the following combinations of SNPs is determined to be present: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7, or (iii) SNP1 genotype A/G or A/A, or T/C or T/T in the complement of SEQ ID NO:6 and SNP2 genotype T/C or C/C, or A/G or G/G in the complement of SEQ ID No:7 and (d) optionally treating the patient selected in step (c) with said drug.

When the above assay is performed to determine a treatment regimen with a drug, step (c) is optional, whereas step (d) is preferably performed, or is performed.

Herein further disclosed is an assay to determine non-sensitivity to a drug treatment, the assay comprising: (a) subjecting a test sample from a human subject, diagnosed as having a cartilage disorder, to at least one genotyping assay that determines the genotypes of at least two loci, wherein said at least two loci are: (i) SNP1 and (ii) SNP2, (b) determining the genotypes of said at least two loci; (c) selecting a patient as being non-sensitive to a treatment with said drug when the following combinations of SNPs is determined to be present: SNP1 genotype G/G, or C/C in the complement of the SEQ ID NO: 6 ; and SNP2 genotype T/T, or A/A in the complement of the SEQ ID NO: 7, and (d) optionally treating the patient selected in step (c) with a therapeutic compound other than said drug.

Before determining the genotype at one locus, in the above disclosed assays, it is needed to obtain a nucleic acid (or test) sample of said subject, for instance by blood or saliva collecting.

Also further disclosed is method for selecting patients having a cartilage disorder for inclusion in or exclusion from treatment, or clinical trial, with a drug, based on the likelihood of their response to said treatment, comprising:

a. Determining, from a nucleic acid sample, the genotype at both loci IL-1RN rs9005 and IL-1 RN rs315952, wherein the patient's genotype with respect to said loci is predictive about the patient's risk for being sensitive or non-sensitive to said drug, and
b. Selecting the sensitive patients as being suitable for said treatment or clinical trial.

Before determining the genotype at one locus, it is needed to obtain a nucleic acid sample of said subject, for instance by blood or saliva collecting. Preferably, the nucleic acid sample is a DNA sample. Thus, herein reported is a method for selecting patients a cartilage disorder for inclusion in or exclusion from treatment, or clinical trial, with a drug, based on the likelihood of their response to said treatment, comprising:

a. Obtaining a nucleic acid sample of said subject,
b. Identifying, from said nucleic acid sample, the patient's nucleic acid at both of the polymorphic loci consisting of IL-1RN rs9005 and IL-1RN rs315952, wherein the patient's genotype with respect to said loci is predictive about the patient's risk for being sensitive or non-sensitive to said treatment, and
c. Selecting the sensitive patients as being suitable for said treatment or clinical trial.

According to said method, patients having the genotype IL-1RN rs9005 G/G and IL-1RN rs315952 T/T, who are predicted being non-sensitives, are preferably excluded from the drug treatment or from clinical trial. The others patients (the sensitive patients, including super-sensitive), can be selected as suitable for the treatment, or clinical trial, and thus can be treated with the drug.

Alternatively, the method for selecting a patient having a cartilage disorder for inclusion in or exclusion from treatment or clinical trial with a drug based on the likelihood of the patient's sensitivity to said drug, comprised the steps of: (a) subjecting a test sample from a human subject, who is diagnosed as having cartilage disorder, to at least one genotyping assay adapted to determine the genotypes of at least two loci, wherein said at least two loci are: (i) SNP1 SNP2, wherein SNP2 is position 27 of SEQ ID NO. 7 identified by rs315952, wherein the SEQ ID NO. 7 is a portion of genomic nucleic acid sequence of interleukin 1 receptor antagonist (IL-1RN); and (b) detecting from the genotypes of said at least two loci the presence of a genotype combination selected from: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7; or (iii) SNP1 genotype G/G, or C/C in the complement of the SEQ ID NO: 6 and SNP2 genotype T/T, or A/A in the complement of the SEQ ID NO: 7; and (c) selecting a patient for inclusion in treatment or clinical trial with said drug when conditions (i) or (ii) are detected based on the recognition that the genotype combinations (i) and (ii) are associated with a response to said drug, and excluding the patient from treatment or clinical trial with said drug when condition (iii) is detected based on the recognition that the genotype combination (iii) is associated with inadequate response to treatment with said drug.

The method for selecting a human subject for a clinical trial for testing a drug, may alternatively comprises the steps of: (a) assaying a biological sample from a human subject diagnosed with a cartilage disorder for at least the following two single nucleotide polymorphisms: (i) SNP1 and (ii) SNP2, (b) determining the genotypes of the SNPs; (c) selecting for the clinical trial the human subject who carries one of the following genotypes in said SNPs: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7; or (iii) a human subject who does not carry SNP1 genotype G/G, or C/C in the complement of the SEQ ID NO: 6 and SNP2 genotype T/T, or A/A in the complement of the SEQ ID NO: 7.

Herein disclosed is also a method of excluding a human subject from a clinical trial testing a drug, the method comprising the steps of: (a) assaying a biological sample from a human subject diagnosed with a cartilage disorder for at least the following two single nucleotide polymorphisms: (i) SNP1 and (ii) SNP2; (b) determining the genotypes of the SNPs; (c) excluding from the clinical trial the human subject who carries the following genotype in said SNPs: SNP1 genotype G/G, or C/C in the complement of the SEQ ID NO: 6 and SNP2 genotype T/T, or A/A in the complement of the SEQ ID NO: 7; or excluding from the clinical trial the human subject who does not carry either of the following SNP genotypes: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7.

Besides the finding that as a function of his/her genotype, the subject could be classified as super-sensitive, sensitive or non-sensitive, it has surprisingly been found that the same genotype is also predictive of adverse events, such as AIRs. Indeed, further investigations and analysis of the SNP polymorphisms demonstrated a relation between the markers rs9005 and rs315952, in combination, with adverse events in the clinic, with MRI data concerning structural benefit and with symptomatic benefit as determined using the WOMAC questionnaire. Not only these SNPs can be used as predictive tool of the patient's response to a treatment with a drug at cartilage volume level, but can also be used as predictive tool of his/her risk to develop adverse events such as AIRs. Thus, the profile: "structural benefit vs. potential adverse effects" of drug therapy would be useful to determine a better risk/benefit ratio, i.e. better outcome with lower risk of side effects in the patients.

This is indeed based on the finding that the super-sensitives have higher WOMAC scores and higher likelihood for having an AIR event, notably for instance when a drug, such as and FGF-18 compound, is used at a dose of 100 mcg, compared to patients treated with the placebo. Similarly, the non-sensitives also have high WOMAC scores, at any dose, compared to patients treated with the placebo. It has also been shown that contrary to the results a dose of 100 mcg, super-sensitives treated with said drug (such as FGF-18 compound) at a lower dose, for instance 30 mcg, have lower WOMAC scores (i.e. better WOMAC improvement) and lower likelihood of having an AIR event. In view of these results, it can be useful to select the patients based on their likelihood to respond/not respond to a drug treatment in combination with their risk level to present adverse events: the non-sensitives could be excluded from a treatment that is likely not working for them (see above method of selection), and the super-sensitives may be subjects to an alternative treatment regimen.

Thus also disclosed is a method for selecting patients having a cartilage disorder for an alternative therapeutic regimen with a drug, based on their likelihood of being super sensitives to said drug treatment, comprising identifying the patient's nucleic acid at both of the polymorphic loci selected from the group consisting of IL-1RN rs9005 and IL-1RN rs315952, wherein the patient's genotype with respect to said loci is predictive about the subject's risk for being super sensitive to a treatment with said drug and allows the selection of said patient for an alternative therapeutic regimen that would be suitable to said patient, in which alternative therapeutic regimen the dose of the drug that is to be administered is reduced compared to the dose of the drug to be administered to a patient who is predicted to be sensitive but not super-sensitive to the said drug treatment.

Also described herein is a method for selecting a patient having a cartilage disorder for a modified treatment regimen with a drug based on the likelihood of said patient of having Acute Inflammatory Reaction (AIR) events when treated with said compound, the method comprising the steps of (a) detecting from a nucleic acid sample obtained from the patient the genotype of (i) SNP1 and (ii) SNP2; and (b) selecting a modified treatment regimen for a patient when a combination of SNP1 genotype A/G or AA, or T/C or T/T/ in the complement of the SEQ ID NO: 6; and SNP2 genotype T/T, or A/A in the complement of the SEQ ID NO: 7 is detected.

Accordingly, patients having the genotype IL-1RN rs9005 A/G or A/A and IL-1RN rs315952 T/C or C/C, who are predicted being super-sensitives, are preferably selected for an alternative therapeutic regimen in which one the dose of the drug to be administered is reduced.

Also described herein is a method for selecting patients having a cartilage disorder for an alternative therapeutic regimen with a drug, based on their likelihood of having AIR events when treated with said drug, comprising determining, from a nucleic acid sample, the genotype at both loci IL-1RN rs9005 and IL-1RN rs315952, wherein the patient's genotype with respect to said loci is predictive about the subject's risk for developing AIR events in response to treatment with said drug, and allows the selection of said patient for an alternative therapeutic regimen that would be suitable to said patient, in which alternative therapeutic regimen the dose of drug that is to be administered is reduced compared to the dose of drug to be administered to a patient who (1) is predicted to be sensitive and (2) does not present a risk for developing AIR events.

Accordingly, patients having the genotype A/G or A/A at IL-1RN rs9005 and T/C or C/C at IL-1RN rs315952, who are predicted being super-sensitives, are preferably selected for an alternative therapeutic regimen in which one the dose of drug to be administered is reduced, compared to the normal therapeutic regimen, i.e. the one for a patient who is predicted to be sensitive to drug treatment but who does not present a risk for developing AIR events.

Further herein described is an assay for selecting a treatment regimen for a human subject with a cartilage disorder, the assay comprising: (a) subjecting a test sample from the human subject, who is diagnosed as having cartilage disorder, to at least one genotyping assay that determines the genotypes of at least two loci, wherein said at least two loci are: (i) SNP1 and (ii) SNP2; (b) detecting from the genotypes of said at least two loci the presence of a genotype combination selected from: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7; or (iii) SNP1 genotype G/G, or C/C in the complement of the SEQ ID NO: 6 and SNP2 genotype T/T, or A/A in the complement of the SEQ ID NO: 7; and (c) selecting, and optionally administering, a treatment regimen comprising an effective amount of a drug when condition (i) or (ii) is detected based on the recognition that the genotype combinations (i) and (ii) are associated with a response to said drug, and excluding the treatment regimen comprising said drug when condition (iii) is detected based on the recognition that the genotype combination (iii) is associated with inadequate response to treatment with said drug.

Also described is a method for treating a human subject with cartilage disorder, comprising administering a composition comprising an effective amount of a drug to a human subject, who is diagnosed to have cartilage disorder, and who is further determined to carry the combination of the single nucleotide polymorphisms (SNPs) selected from: (i) SNP1 genotype G/G, or C/C in the complement of the SEQ ID NO: 6, wherein SNP1 is position X of SEQ ID NO: 6 identified by rs9007, wherein the SEQ ID NO. 6 is a portion of genomic nucleic acid sequence of interleukin 1 receptor antagonist (IL-1RN); and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7, wherein SNP2 is position X of SEQ ID NO. 7 identified by rs317972, wherein the SEQ ID NO. 7 is a portion of genomic nucleic acid sequence of interleukin 1 receptor antagonist (IL-1RN).

Further disclosed is a method for treating a human subject with a cartilage disorder, comprising (a) assaying a biological sample of a subject, who is diagnosed as having the cartilage disorder for at least the following two SNP loci: (i) SNP1, and (ii) SNP2; and (b) administering a treatment regimen comprising a composition comprising an effective amount of an FGF-18 compound to the subject if one of the following conditions is detected: (i) SNP1 genotype G/G, or C/C in the complement of the SEQ ID NO: 6 ; and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7.

Alternatively, the method for treating a human subject with a cartilage disorder, comprises the steps of: (a) assaying a biological sample of a subject, who is diagnosed as having the cartilage disorder for at least the following two SNP loci: (i) SNP1, and (ii) SNP2 and (b) administering a treatment regimen comprising a composition comprising an effective amount of an FGF-18 compound to the subject if SNP1 genotype G/G, or C/C in the complement of the SEQ ID NO: 6 and SNP2 genotype T/T, or A/A in the complement of the SEQ ID NO: 7 is not detected.

In yet an other alternative, the method for selecting in a subject having a cartilage disorder, wherein said a cartilage disorder is susceptible to treatment with a drug, comprises: (a) obtaining a biological sample from the subject with a cartilage disorder with the objective to determine whether the cartilage disorder in the subject is susceptible to treatment with said drug; (b) contacting the biological sample with at least two oligonucleotides capable of interrogating whether or not the biological sample comprises the combination of the single nucleotide polymorphisms (SNPs) selected from (i) SNP1 genotype G/G, or C/C in the complement of the SEQ ID NO: 6, and SNP2 genotype T/C or CC, or A/G or GG in the complement of the SEQ ID NO: 7; or (ii) SNP1 genotype A/G or AA, or T/C or T/T/ in the complement of the SEQ ID NO: 6; and SNP2 genotype T/T, or A/A in the complement of the SEQ ID NO: 7; (c) identifying the cartilage disorder in the subject as susceptible for treatment with said drug when either the combination of (i) or (ii) is detected in the biological sample and identifying the cartilage disorder in the subject as poorly or non-responsive to treatment with said drug when neither (i) nor (ii) is detected in the biological sample.

Also disclosed herein is a method for selecting a treatment regimen for a subject with a cartilage disorder, comprising: (a) obtaining a test sample from the human subject diagnosed as having depression; (b) subjecting the test sample to at least one analysis to determine parameters of at least two single nucleotide polymorphisms (SNPs), wherein the at least two SNPs comprise the following: (i) SNP1, and (ii) SNP2, (c) detecting using the SNPs, the presence of at least one condition of the following or a combination thereof: i. SNP1 genotype G/G, or C/C in the complement of the SEQ ID NO: 6 ; and SNP2 genotype T/C or CC, or A/G or GG in the complement of the SEQ ID NO: 7; or ii. SNP1 genotype A/G or AA, or T/C or T/T/ in the complement of the SEQ ID NO: 6; and SNP2 genotype T/T, or A/A in the complement of the SEQ ID NO: 7; or iii. SNP1 genotype G/G, or C/C in the complement of the SEQ ID NO: 6 and SNP2 genotype T/T, or A/A in the complement of the SEQ ID NO: 7 (d) providing a result output setting forth whether at least one of said condition is detected from the test sample and when condition (i) or (ii) is detected, then selecting and optionally administering a treatment regimen comprising a drug to the human subject, and when condition (iii) is detected, then not selecting or administering a treatment regimen comprising said drug to the human subject

In the above mentioned methods, and assay, the patients having the genotype A/G or A/A at IL-1RN rs9007 (SNP1) and T/C or C/C at IL-1RN rs317972 (SNP2), who are predicted being super-sensitives, are preferably selected for an alternative therapeutic regimen in which one the dose of the drug to be administered is reduced, compared to the normal therapeutic regimen, i.e. the one for a patient who is predicted to be sensitive to the drug treatment but who does not present a risk for developing AIR events.

In another aspect of the disclosure, also provided are systems (and computer readable media for causing computer systems) for obtaining data. Said data can be used notably for assessing suitability of a treatment with a drug in a subject, for assessing the subject's risk of developing AIR when treated with said drug, or monitoring treatment efficacy of a subject with said drug. Said systems can be used during clinical trials, notably when a treament with a drug for treating a cartilage disorder has to be envisaged or when a treatment with said compound is already ongoing.

Therefore, in an aspect of the present disclosure is included a computer system for obtaining data from at least one test sample obtained from at least one subject with a cartilage disorder, the system comprising: (a) at least one determination module configured to receive said at least one test sample and perform at least one analysis on said at least one test sample to determine the presence or absence of the following conditions: (i) SNP1 genotype G/G, or C/C in the complement of the SEQ ID NO: 6, and SNP2 genotype T/C or CC, or A/G or GG in the complement of the SEQ ID NO: 7 or (ii) SNP1 genotype A/G or AA, or T/C or T/T/ in the complement of the SEQ ID NO: 6; and SNP2 genotype T/T, or A/A in the complement of the SEQ ID NO: 7,; or (iii) SNP1 genotype G/G, or C/C in the complement of the SEQ ID NO: 6, and SNP2 genotype T/T, or A/A in the complement of the SEQ ID NO: 7; (b) at least one storage device configured to store data output from said determination module; and (c) at least one display module for displaying a content based in part on the data output from said determination module, wherein the content comprises a signal indicative of the presence of at least one of these conditions, and optionally the absence of any one of these conditions.

Also described is a computer system for obtaining data from at least one test sample obtained from at least one subject, the system comprising: (a) a determination module configured to receive said at least one test sample and perform at least one genotyping analysis on said at least one test sample to determine the genotypes of at least two loci, wherein said at least two loci comprise: (i) SNP1, and (ii) SNP2, (b) a storage device configured to store output data from said determination module; (c) a computing module comprising specifically-programmed instructions to determine from the output data the presence of any of the combinations of polymorphisms selected from the following: i. SNP1 genotype G/G, or C/C in the complement of the SEQ ID NO: 6 ; and SNP2 genotype T/C or CC, or A/G or GG in the complement of the SEQ ID NO: 7; or ii. SNP1 genotype A/G or AA, or T/C or T/T/ in the complement of the SEQ ID NO: 6; and SNP2 genotype T/T, or A/A in the complement of the SEQ ID NO: 7; or iii. SNP1 genotype G/G, or C/C in the complement of the SEQ ID NO: 6 and SNP2 genotype T/T, or A/A in the complement of the SEQ ID NO: 7; and (d) a display module for displaying a content based in part on the data output from said computing module, wherein the content comprises a signal indicative of the presence of the combination (i), (ii), or (iii) of the SNPs, and optionally the absence of any one or more or the combinations (i), (ii), and (iii) of the SNPs.

The computer readable medium can have computer readable instructions recorded thereon to define software modules for implementing a method on a computer. In such a case, said computer readable storage medium may comprise: (a) instructions for comparing the data stored on a storage device with reference data to provide a comparison result, wherein the comparison identifies the presence or absence of at least one of the following conditions: (i) SNP1 genotype G/G, or C/C in the complement of the SEQ ID NO: 6, and SNP2 genotype T/C or CC, or A/G or GG in the complement of the SEQ ID NO: 7, or (ii) SNP1 genotype A/G or AA, or T/C or T/T/ in the complement of the SEQ ID NO: 6; and SNP2 genotype T/T, or A/A in the complement of the SEQ ID NO: 7; or (iii) SNP1 genotype G/G, or C/C in the complement of the SEQ ID NO: 6, and SNP2 genotype T/T, or A/A in the complement of the SEQ ID NO: 7; and (b) instructions for displaying a content based in part on the data output from said determination module, wherein the content comprises a signal indicative of the presence of at least one of the conditions, and optionally the absence of one or more of the conditions.

The computer readable storage media can be any available tangible media that can be accessed by a computer. Computer readable storage media includes volatile and nonvolatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (eraseable programmable read only memory), EEPROM (electrically eraseable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable media may define instructions, for example, as part of one or more programs that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable media on which such instructions are embodied may reside on one or more of the components of either of a system, or a computer readable storage medium described herein, may be distributed across one or more of such components.

The computer-readable media may be transportable such that the instructions stored thereon can be loaded onto any computer resource to implement the aspects of the present invention discussed herein.

The information determined in the determination module can be read by the storage device. The storage device is adapted or configured for having recorded thereon expression level or protein level information. Such information may be provided in digital form that can be transmitted and read electronically, e.g., via the Internet, on diskette, via USB (universal serial bus) or via any other suitable mode of communication.

It is to be understood that in the context of the present disclosure as a whole, e.g. in the context of any one of the methods, uses, assays or kits according to the present disclosure, before determining the genotype at one locus, it is needed to obtain a nucleic acid sample (or a test sample) of one subject, for instance by blood or saliva collecting. Preferably, the nucleic acid sample is a DNA sample.

In the context of the present disclosure as a whole, e.g. in the context of any one of the methods, uses, computer system or kits according to the present invention, the preferred drug is an anabolic drug, i.e. which have an anabolic effect on cartilage. In particular said anabolic drug is selected from the group consisting of an FGF-18 compound, BMP-2, BMP-7, GDF-5, FGF$\beta$, FGF-8, FGF-9, SOX-9 enhancers, or TGF$\beta$ and any variants thereof. More preferably the anabolic drug is a truncated FGF-18 compound, such as sprifermin.

Also in the context of the present disclosure as a whole, the cartilage disorder is preferably selected from the group consisting of osteoarthritis, cartilage injury, fractures affecting joint cartilage or surgical procedures with impact on joint cartilage (e.g. Microfracture).

An individual afflicted with a cartilage disorder and to be tested and/or treated according to any of the methods and uses described herein is a human subject that is a candidate for treatment with a drug, preferably an anabolic drug, such as sprifermin. In a preferred embodiment, the individual has been diagnosed with cartilage disorder, or exhibits a symptom of cartilage disorder. Said cartilage disorder is preferably selected from the group consisting of, but not limited to, osteoarthritis, cartilage injury, fractures affecting joint cartilage or surgical procedures with impact on joint cartilage (e.g. Microfracture).

It is also to be understand that in the context of the disclosure as a whole, determination can be performed on the complemantary sequence of IL1-RN rs9005 and IL1-RN rs315952. It thus follows that according to the present disclosure as a whole, e.g. in the context of any one of the methods, uses, assays, computer system or kits according to the present disclosure, the presence of the genotype C/C on the complementary sequence to IL-1RN rs9005 and A/A on the complementary sequence of IL-1RN rs315952 is predictive of no response or low response (i.e. non-sensitivity) to treatment with an FGF-18 compound. On the contrary, the presence of the genotype T/C or T/T on the complementary sequence at IL-1RN rs9005 and A/G or G/G on the complementary sequence of IL-1RN rs315952 is predictive of high response (high-sensitivity) to treatment with an FGF-18 compound. Said genotype will also be a marker of likelihood for a patient of developing AIRs events when treated with said FGF-18 compound. The other genotypes at these loci are predictive of intermediate sensitivity (i.e. C/C in the complement of IL-1RN rs9005 and A/G or G/G in the complement of IL-1RN rs315952 or T/C or T/T in the complement of IL-1RN rs9005 and A/A in the complement of IL-1RN 315952).

Also herein disclosed is a kit comprising means for performing the methods described above and instructions for use. In particular, the kit comprises at least a couple of specific primers or probes for detecting the presence or absence of the alleles. Preferably, it comprises two couples of specific primers or probes for genotyping the alleles at loci IL-1RN rs9005 and IL-1RN rs315952.

The kit may comprise an oligonucleotide array affixed with a plurality of oligonucleotide probes that interrogate no more than 20 single nucleotide polymorphisms (SNPs), said SNPs comprising: (i) SNP1 genotype G/G, or C/C in the complement of the SEQ ID NO: 6, and SNP2 genotype T/C or CC, or A/G or GG in the complement of the SEQ ID NO: 7,; or (ii) SNP1 genotype A/G or AA, or T/C or T/T/ in the complement of the SEQ ID NO: 6 and SNP2 genotype T/T, or A/A in the complement of the SEQ ID NO: 7,; or (iii) SNP1 genotype G/G, or C/C in the complement of the SEQ ID NO: 6, and SNP2 genotype T/T, or A/A in the complement of the SEQ ID NO: 7; an optional container containing a detectable label to be conjugated to a nucleotide molecule derived from a test sample of a subject diagnosed as having a cartilage disorder; and at least one reagent.

Alternatively, the oligonucleotide array affixed with a plurality of oligonucleotide probes interrogates no more than 17 single nucleotide polymorphisms (SNPs), no more than 10 single nucleotide polymorphisms (SNPs) or no more than 7 single nucleotide polymorphisms (SNPs).

Also described in the context of this disclosure is a kit comprising: a plurality of oligonucleotide primers or sets of primers that each bind to interrogate no more than one specific allele of no more than 20 single nucleotide polymorphisms (SNPs), wherein each subset of oligonucleotide primers that bind to a specific allele of a SNP is labeled with a distinct reporter, and wherein said SNPs comprise the following SNPs: i. SNP1 genotype G/G, or C/C in the complement of the SEQ ID NO: 6, and SNP2 genotype T/C or CC, or A/G or GG in the complement of the SEQ ID NO: 7; or ii. SNP1 genotype A/G or AA, or T/C or T/T/ in the complement of the SEQ ID NO: 6 and SNP2 genotype T/T, or A/A in the complement of the SEQ ID NO: 7; or iii. SNP1 genotype G/G, or C/C in the complement of the SEQ ID NO: 6, and SNP2 genotype T/T, or A/A in the complement of the SEQ ID NO: 7; and at least one reagent.

Alternatively, the plurality of oligonucleotide primers or sets of primers that each bind to interrogate no more than one specific allele of no more than 17 single nucleotide polymorphisms (SNPs), or no more than one specific allele of no more than 10 single nucleotide polymorphisms (SNPs) or no more than one specific allele of no more than 7 single nucleotide polymorphisms (SNPs).

A kit is also disclosed for selecting a treatment regimen for a subject with a cartilage disorder, comprising at least one reagent for determining in a test sample of a human subject diagnosed as having the cartilage disorder, the presence or absence of the following SNPs : i. SNP1 genotype G/G, or C/C in the complement of the SEQ ID NO: 6, and SNP2 genotype T/C or CC, or A/G or GG in the complement of the SEQ ID NO: 7; or ii. SNP1 genotype A/G or AA, or T/C or T/T/ in the complement of the SEQ ID NO: 6 and SNP2 genotype T/T, or A/A in the complement of the SEQ ID NO: 7; or iii. SNP1 genotype G/G, or C/C in the complement of the SEQ ID NO: 6 and SNP2 genotype T/T, or A/A in the complement of the SEQ ID NO: 7.

In some aspects, the oligonucleotides in the kit are either allele-specific probes or allele-specific primers. In other embodiments, the kit comprises primer-extension oligonucleotides. In still further aspects, the set of oligonucleotides is a combination of allele-specific probes, allele-specific primers, or primer-extension oligonucleotides.

The composition and length of each oligonucleotide in a kit of the invention will depend on the nature of the genomic region containing the genetic marker of the disclosure as well as the type of assay to be performed with the oligonucleotide and is readily determined by the skilled artisan. For example, the polynucleotide to be used in the assay may constitute an amplification product, and thus the required specificity of the oligonucleotide is with respect to hybridization to the target region in the amplification product rather than in genomic DNA isolated from the individual.

In preferred aspects, each oligonucleotide in the kit is a perfect complement of its target region. An oligonucleotide is said to be a "perfect" or "complete" complement of another nucleic acid molecule if every nucleotide of one of the molecules is complementary to the nucleotide at the corresponding position of the other molecule. While perfectly complementary oligonucleotides are preferred for detecting polymorphisms, departures from complete complementarity are contemplated where such departures do not prevent the molecule from specifically hybridizing to the target region as defined above. For example, an oligonucleotide primer may have a non-complementary fragment at its 5' end, with the remainder of the primer being completely complementary to the target region. Alternatively, non-complementary nucleotides may be interspersed into the probe or primer as long as the resulting probe or primer is still capable of specifically hybridizing to the target region.

In some preferred aspects, each oligonucleotide in the kit specifically hybridizes to its target region under stringent hybridization conditions. Stringent hybridization conditions are sequence-dependent and vary depending on the circumstances. Generally, stringent conditions are selected to be about 5°C lower than the thermal melting point ($T_m$) for the specific sequence at a defined ionic strength and pH. The $T_m$ is the temperature (under defined ionic strength, pH, and nucleic acid concentration) at which 50% of the probes complementary to the target sequence hybridize to the target sequence at equilibrium. As the target sequences are generally present in excess, at $T_m$, 50% of the probes are occupied at equilibrium. Typically, stringent conditions include a salt concentration of at least about 0.01 to 1.0 M Na ion concentration (or other salts) at pH 7.0 to 8.3 and the temperature is at least about 25°C for short oligonucleotide probes (e.g., 10 to 50 nucleotides). Stringent conditions can also be achieved with the addition of destabilizing agents such as formamide. For example, conditions of 5 x SSPE (750 mM NaCl, 50 mM NaPhosphate, 5 mM EDTA, pH 7.4) and a temperature of 25-30°C are suitable for allele-specific probe hybridizations.

The oligonucleotides in kits of the disclosure may be comprised of any phosphorylation state of ribonucleotides, deoxyribonucleotides, and acyclic nucleotide derivatives, and other functionally equivalent derivatives. Alternatively, the oligonucleotides may have a phosphate-free backbone, which may be comprised of linkages such as carboxymethyl, acetamidate, carbamate, polyamide [peptide nucleic acid (PNA)] and the like. The oligonucleotides may be prepared by chemical synthesis using any suitable methodology known in the art, or may be derived from a biological sample, for example, by restriction digestion. The oligonucleotides may contain a detectable label, according to any technique known in the art, including use of radiolabels, fluorescent labels, enzymatic labels, proteins, haptens, antibodies, sequence tags and the like. The oligonucleotides in the kit may be manufactured and marketed as analyte specific reagents (ASRs) or may be constitute components of an approved diagnostic device.

In other preferred aspects, the kit includes an instruction manual that describes the various ways the kit may be used to detect the presence or absence of a genetic marker of the disclosure.

In a preferred aspects, the set of oligonucleotides in the kit are allele-specific oligonucleotides. As used herein, the term allele-specific oligonucleotide (ASO) means an oligonucleotide that is able, under sufficiently stringent conditions, to hybridize specifically to one allele of a genetic marker, at a target region containing the genetic marker while not hybridizing to the same region containing a different allele. As understood by the skilled artisan, allele-specificity will depend upon a variety of readily optimized stringency conditions, including salt and formamide concentrations, as well as temperatures for both the hybridization and washing steps.

Typically, an ASO will be perfectly complementary to one allele while containing a single mismatch for another allele. In ASO probes, the single mismatch is preferably within a central position of the oligonucleotide probe as it aligns with the genetic marker in the target region (e.g., approximately the 7th or 8th position in a 15mer, the 8th or 9th position in a 16mer, and the 10th or 11th position in a 20mer). The single mismatch in ASO primers is located at the 3' terminal nucleotide, or preferably at the 3' penultimate nucleotide. ASO probes and primers hybridizing to either the coding or non-coding strand are contemplated by the invention.

In other preferred aspects, the kit comprises a pair of allele-specific oligonucleotides for a genetic marker of the invention to be assayed, with one member of the pair being specific for one allele and the other member being specific for another allele. In such embodiments, the oligonucleotides in the pair may have different lengths or have different detectable labels to allow the user of the kit to determine which allele-specific oligonucleotide has specifically hybridized to the target region, and thus determine which allele is present in the individual at the assayed marker locus.

In still other preferred aspects, the oligonucleotides in the kit are primer-extension oligonucleotides. Termination mixes for polymerase-mediated extension from any of these oligonucleotides are chosen to terminate extension of the oligonucleotide at the genetic marker of interest, or one base thereafter, depending on the alternative nucleotides present at the marker locus.

The methods and kits according to the present disclosure are useful in clinical diagnostic applications. However, as used herein, the term "diagnostic" is not limited to clinical or medical uses, and the diagnostic methods and kits of the invention claimed herein are also useful in any research application, and during clinical trials, for which it is desirable to test a subject for the presence or absence of any genetic marker described herein.

In the context of the disclosure, the presence or absence of a particular allele or pair of alleles at the locus of a genetic marker of the invention in an individual may be detected by any technique known per se to the skilled artisan, including sequencing, pyrosequencing, selective hybridization, selective amplification and/or mass spectrometry including matrix-assisted laser desorption/ionization time-of-flight mass spectrometry (MALDI-TOF MS). In a particular embodiment, the alteration is detected by selective nucleic acid amplification using one or several specific primers. The alteration is detected by selective hybridization using one or several specific probes.

Further techniques include gel electrophoresis-based genotyping methods such as PCR coupled with restriction fragment length polymorphism (RFLP) analysis, multiplex PCR, oligonucleotide ligation assay, and minisequencing; fluorescent dye-based genotyping technologies such as marker, at a target region containing the genetic marker while not hybridizing to the same region containing a different allele. As understood by the skilled artisan, allele-specificity will depend upon a variety of readily optimized stringency conditions, including salt and formamide concentrations, as well as temperatures for both the hybridization and washing steps.

Typically, an ASO will be perfectly complementary to one allele while containing a single mismatch for another allele. In ASO probes, the single mismatch is preferably within a central position of the oligonucleotide probe as it aligns with the genetic marker in the target region (e.g., approximately the 7th or 8th position in a 15mer, the 8th or 9th position in a 16mer, and the 10th or 11th position in a 20mer). The single mismatch in ASO primers is located at the 3' terminal nucleotide, or preferably at the 3' penultimate nucleotide. ASO probes and primers hybridizing to either the coding or non-coding strand are contemplated by the invention.

In other preferred embodiments, the kit comprises a pair of allele-specific oligonucleotides for a genetic marker of the invention to be assayed, with one member of the pair being specific for one allele and the other member being specific for another allele. In such embodiments, the oligonucleotides in the pair may have different lengths or have different detectable labels to allow the user of the kit to determine which allele-specific oligonucleotide has specifically hybridized to the target region, and thus determine which allele is present in the individual at the assayed marker locus.

In still other preferred embodiments, the oligonucleotides in the kit are primer-extension oligonucleotides. Termination mixes for polymerase-mediated extension from any of these oligonucleotides are chosen to terminate extension of the oligonucleotide at the genetic marker of interest, or one base thereafter, depending on the alternative nucleotides present at the marker locus.

The methods and kits according to the present disclosure are useful in clinical diagnostic applications.

However, as used herein, the term "diagnostic" is not limited to clinical or medical uses, and the diagnostic methods and kits of the invention claimed herein are also useful in any research application, and during clinical trials, for which it is desirable to test a subject for the presence or absence of any genetic marker described herein.

In the context of the invention, the presence or absence of a particular allele or pair of alleles at the locus of a genetic marker of the invention in an individual may be detected by any technique known per se to the skilled artisan, including sequencing, pyrosequencing, selective hybridization, selective amplification and/or mass spectrometry including matrix-assisted laser desorption/ionization time-of-flight mass spectrometry (MALDI-TOF MS). In a particular embodiment, the alteration is detected by selective nucleic acid amplification using one or several specific primers. The alteration is detected by selective hybridization using one or several specific probes.

Further techniques include gel electrophoresis-based genotyping methods such as PCR coupled with restriction fragment length polymorphism (RFLP) analysis, multiplex PCR, oligonucleotide ligation assay, and minisequencing; fluorescent dye-based genotyping technologies such as oligonucleotide ligation assay, pyrosequencing, single-base extension with fluorescence detection, homogeneous solution hybridization such as TaqMan, and molecular beacon genotyping; sequencing-based technologies such as Sanger sequencing and next-generation sequencing platforms; rolling circle amplification and Invader assays as well as DNA chip-based microarray and mass spectrometry genotyping technologies. Protein expression analysis methods are known in the art and include 2-dimensional gel- electrophoresis, mass spectrometry and antibody microarrays. Sequencing can be carried out using techniques well known in the art, e.g. using automatic sequencers. The sequencing may be performed on the complete gene or, more preferably, on specific domains thereof, typically those known or suspected to carry deleterious mutations or other alterations.

Amplification may be performed according to various techniques known in the art, such as by polymerase chain reaction (PCR), ligase chain reaction (LCR) and strand displacement amplification (SDA). These techniques can be performed using commercially available reagents and protocols. A preferred technique is allele-specific PCR.

Other embodiments of the invention within the scope of the claims herein will be apparent to one skilled in the art from consideration of the specification or practice of the invention as disclosed herein. It is intended that the specification, together with the examples, be considered exemplary only, with the scope of the invention being indicated by the claims that follow the examples.

**Description of the Figures:**

**General notes:** in the figures, 1) the terms TT, CC, GG or AA are to be understood as being T/T, C/C, G/G or A/A, and 2) the term CTA is to be understood as C-T-A.

**Figure 1:** Organization of the IL1R1-IL1A-IL1B-IL1RN gene cluster. Both rs315952 and rs9005 are located in the last IL1RN exon. Although there is only 1107 bp between them, these SNPs are not inherited together (i.e. not in Linkage Disequilibrium). IL1RN-rs9005 is within the 3' UTR region and overlaps both a transcription factor (ChIP-seq sequence: FOSL2) and a DNAse cluster (regulatory regions and promoter tend to be DNAse sensitive). IL1 RN-rs315952 is a coding silent SNP (i.e. does not lead to an amino acid change).

**Figure 2:** Stratification of the patients as a function of presence or absence (from at least one copy) of the C-T-A haplotype. The Y axis shows change at Week 52 in total cartilage volume (unit: $mm^3$). Each point corresponds to a subject, circle indicates a subject without AIR while cross indicates a subject with AIRs. Indicated p-value was obtained from a non-parametric univariate test (ranksum test).

**Figure 3:** Stratification of the patients as a function of presence or absence (from at least one copy) of the C-T-A haplotype. The Y axis shows change at Week 52 in WOMAC total score. Each point corresponds to a subject, circle indicates a subject without AIR event while cross indicates a subject with AIRs. Indicated p-value was obtained from a non-parametric univariate test (ranksum test).

**Figure 4:** Change in total cartilage volume ($mm^3$) at Week 52 stratified by dose regimen and stratified by their genotype at both rs315952 and rs9005. Each point corresponds to a subject, circle indicates a subject without AIR while cross indicates a subject with AIRs. MRI data from the MAD010 cohort showed aberrant variability and were not included in any analyses.

**Figure 5:** Change in WOMAC total score at Week 52 stratified by dose regimen and stratified by their genotype at both rs315952 and rs9005. Each point corresponds to a subject, circle indicates a subject without AIR while cross indicates a subject with AIRs.

**Figure 6:** Stratification of the patients as a function of presence or absence of the 'rs9005 G/G rs315952 T/T' genotype. The Y axis shows absolute WOMAC total score at baseline. Each point corresponds to a subject, circle indicates a subject with Kellgren-Lawrence grade equals to 2 while cross indicates a subject with Kellgren-Lawrence grade equals to 3. Indicated p-value was obtained from a non-parametric univariate test (ranksum test).

**Figure 7:** Stratification of the patients as a function of presence or absence of the' rs9005 A carriers rs315952 C carriers' genotype. The Y axis shows absolute WOMAC total score at baseline. Each point corresponds to a subject, circle indicates a subject with Kellgren-Lawrence grade equals to 2 while cross indicates a subject with Kellgren-Lawrence grade equals to 3. Indicated p-value was obtained from a non-parametric univariate test (ranksum test).

**Figure 8:** Stratification of the patients as a function of presence or absence of the 'rs9005 G/G rs315952 T/T' genotype. The Y axis shows absolute total cartilage volume ($mm^3$) at baseline. Each point corresponds to a subject, circle indicates a subject with Kellgren-Lawrence grade equals to 2 while cross indicates a subject with Kellgren-Lawrence grade equals to 3. Indicated p-value was obtained from a non-parametric univariate test (ranksum test).

**Figure 9:** Stratification of the patients as a function of presence or absence of the' rs9005 A carriers rs315952 C carriers' genotype. The Y axis shows absolute total cartilage volume ($mm^3$) at baseline. Each point corresponds to a subject, circle indicates a subject with Kellgren-Lawrence grade equals to 2 while cross indicates a subject with Kellgren-Lawrence grade equals to 3. Indicated p-value was obtained from a non-parametric univariate test (ranksum test).

**Figure 10:** Change from baseline in WOMAC total score for all subjects irrespectively of their genotypes. Lines correspond to the mean change from baseline and error bars correspond to standard error of mean.

**Figure 11:** Change from baseline in WOMAC total score for subjects identified as sensitives or super-sensitives based on their rs9005 and rs315952 genotypes. The 'treated' group corresponds to subjects from the MAD100 cohort having the genotype identifying sensitive subjects. Subjects from the MAD030 cohort having the genotype identifying super- sensitive subjects are also included in this 'treated' group. The 'placebo' group includes placebos subjects with genotype corresponding to either the sensitives or to the super-sensitives. Lines correspond to the mean change from baseline and error bars correspond to standard error of mean.

**Figure 12:** Change from baseline in WOMAC total score for subjects having the genotype corresponding to the non-sensitives. Lines correspond to the mean change from baseline and error bars correspond to standard error of mean.

**Figure 13:** Change from baseline in total cartilage volume ($mm^3$) for all subjects irrespectively of their genotypes. Lines correspond to the mean change from baseline and error bars correspond to standard error of mean. MRI data from the MAD010 cohort showed aberrant variability and were not included in any analyses.

**Figure 14:** Change from baseline in total cartilage volume ($mm^3$) for subjects identified as sensitives or super-sensitives based on their rs9005 and rs315952 genotypes. The 'treated' group corresponds to subjects from the MAD100 cohort having the genotype identifying sensitives. Subjects from the MAD030 cohort having the genotype identifying super- sensitives are also included in this 'treated' group. The 'placebo' group includes placebos subjects with genotype corresponding to either the sensitives or to the super-sensitives. Lines correspond to the mean change from baseline and error bars correspond to standard error of mean. MRI data from the MAD010 cohort did not passed quality control and were not included in any analyses.

**Figure 15:** Change from baseline in total cartilage volume ($mm^3$) for subjects having the genotype corresponding to the non-sensitives. Lines correspond to the mean change from baseline and error bars correspond to standard error of mean. MRI data from the MAD010 cohort showed aberrant variability and were not included in any analyses.

**Figure 16(a)-(h):** Sets out the full length amino acid and nucleic acid sequences corresponding to the "SEQ ID NOs" referenced in the instant patent application.

**Description of the sequences:**

SEQ ID NO.1: Amino acid sequence of the native human FGF-18.
SEQ ID NO.2: Amino acid sequence of the recombinant truncated FGF-18 (trFGF-18).
SEQ ID NO.3: IL1RN gene
SEQ ID NO.4: IL1RN rs9005 locus
SEQ ID NO.5: IL1RN rs315952 locus
SEQ ID NO.6: Specific region from IL1RN rs9005 locus (corresponding to nucleotide 415 to nucleotide 466 of SEQ ID NO.4), wherein N is A or G
SEQ ID NO.7: Specific region from IL1RN rs315952 locus (corresponding to nucleotide 415 to nucleotide 466 of SEQ ID NO.5), wherein N is C or T
SEQ ID NO.8: rs315952 primer 1
SEQ ID NO.9: rs315952 primer 2
SEQ ID NO.10: rs9005 primer 1
SEQ ID NO.11: rs9005 primer 2

**Examples**

**1. Genotyping background:**

The level of cartilage volume growth and the associated risk of adverse events in response to a drug treatment (such as sprifermin treatment) in cartilage disorders, such as osteoarthritis, cartilage injury, fractures affecting joint cartilage or surgical procedures with impact on joint cartilage (e.g. Microfracture), may each be associated with a specific genetic variation in one or several genes. In the present study, the search for associations between genes containing variations and disease or response to treatment was focused on candidate genes that were selected based on the physiological role of the proteins they encode and their potential implication in the cartilage disorders, or in the response to sprifermin treatment. The list of selected candidate SNPs that have been tested is given in Table 1.

Response to sprifermin treatment was measured by change in cartilage volume from baseline 1 year after the beginning of treatment with sprifermin.

It is noted that candidate and whole genome scan SNP markers were not kept for further analysis if any of the following criteria was met:

- Rare variant SNP in the PGx ITT population: Minor Allele Frequency (MAF) < 10% for both candidate SNP and whole genome scan SNPs.
- Questionable genotyping quality, as measured by a high rate (≥5%) of missing data.
- Significant deviation from the Hardy-Weinberg equilibrium (Bonferroni adjusted p value less than 5% for candidate SNPs or FDR (i.e. Benjamini-Hochberg adjusted p value) less than 20% for whole genome scan SNPs).
- Subjects with gender discrepancy between the clinical database and the predicted gender from whole genome scan SNP data (chromosome X) are excluded.

The candidate genes selected have been previously implicated in cartilage disorder, such as osteoarthritis. The purpose of the study was to investigate whether the level of response, i.e. cartilage volume growth and/or occurrence of adverse events in response to sprifermin treatment in cartilage disorder is correlated with a specific DNA variant or pattern of variants. The existence of such a correlation would indicate that either the gene(s) carrying the identified variant(s) or one or more genes lying in the vicinity of the variants may be (a) susceptibility gene(s).

**2 Materials and methods**

*2.1. FGF-18 compound*

The FGF-18 compound used as a treatment in the present examples is sprifermin. It is a truncated form of FGF-18, as defined in the section "definitions".

*2.2. Sample reception and double coding*

Blood samples were received from patients participating in study 28980 (A randomized, double blind, placebo-controlled, multicenter, single and multiple ascending dose study of sprifermin, administered intra-articularly in patients with primary osteoarthritis of the knee who are not expected to require knee surgery within one year)

In order to comply with the Pharmacogenomics (PGx) Informed Consent Form (ICF), which covered the DNA analysis, all samples were double-coded by the Biobank (Merck Serono, Geneva) to ensure an additional level of subject anonymity. The Biobank provided the Biomarker Data Management group with the double key coding as a flat file containing both the PGx ID and the Subject ID for each subject. Additional verifications were performed to ensure that no DNA analyses are performed on subjects who did not consent to the PGx study.

*2.3. DNA samples extraction, amplification, fragmentation and labeling*

The analysis was performed on DNA extracted from blood. A total of 140 blood samples were received. Out of these 140, 3 samples were destroyed by the genomic laboratory as the patients withdrew their consent during the course of study; resulting in 137 DNAs analyzed corresponding to 137 patients. Thus 137 patients were genotyped and eligible for the association studies.

Genomic DNA was extracted from EDTA blood samples using a Qiagen extraction kit (QIAamp DNA Blood Maxi Kit). After extraction, measures of sample absorbance at wavelengths of 260 nm and 280 nm using a spectrophotometer and electrophoresis on agarose gels were performed to estimate the quality and quantity of genomic DNA samples.

For each plate, genomic DNA samples were digested with Nsp I and Sty I restriction endonucleases, ligated with specific adaptors (Nsp or Sty), processed in parallel until the Polymerase Chain Reactions (PCR). PCR amplified the product of ligation in triplicate for StyI reactions and in quadruplicate for NspI reactions, to product a large efficiency. All the PCR products were pooled, purified, quantified, fragmented and labeled.

PCR amplification step was evaluated using electrophoresis agarose gel. DNA quantification step was measured using spectrophotometer and DNA fragmentation step was evaluated using electrophoresis agarose gel. The average of DNA fragment size should be lower than 180 bp.

*2.4. DNA microarray technology (Whole Genome Scan)*

The Affymetrix Genome Wide SNP 6.0 Assays were used to perform the Whole Genome Scan (hypothesis free approach). The Affymetrix technology is based on a DNA chip allowing the genotyping of approximately 906 600 single nucleotide polymorphisms (SNPs) per patient. SNPs are randomly distributed in all the chromosomes and are used as tagging markers of the corresponding genomic area. The details of process and protocol followed the PGX Affymetrix wide-genome SNP 5.0/6.0 technology.

For each sample, the labeled product was hybridized into the Affymetrix Genome Wide SNP 6.0 GeneChip. Two lots of chips were used for both sets.

After hybridization and staining, the Affymetrix Gene Chips were scanned to create image data (DAT) files. After that, AGCC Software aligned automatically a grid on the DAT files and computed the Cell Intensity data (CEL) file. Afterwards the CEL data passed on to Genotyping Console software that generated Probe Analysis (CHP) data.

Analysis quality control was performed using Genotyping Console Software assessing the Dynamic Model QC (DM) call rate analysis of a subset of 3022 SNPs following chip scanning. DM call rates measure the consistency of intensities within each SNP, with four possible genotyping states (Null, AA, AB and BB). It provides an estimate of the overall quality for a data sample prior to performing full clustering analysis. It is based on QC Call Rate.

The QC Call Rate (QC CR) is well correlated with clustering performance and is an effective single-sample metric for deciding what samples should be used in downstream clustering. The fixed threshold for Genome wide SNP6.0 arrays is >=86 %. In addition to QC CR, another algorithm has been developed for SNP 6.0 arrays. This new algorithm is the Contrast QC. The contrast QC is a metric that captures the ability of an experiment to resolve SNP signals into three genotype clusters. It measures the separation of allele intensities into three clusters in "contrast space". Contrast space is a projection of the two-dimensional allele intensity space into an informative single dimension. The default threshold is >= 0.4 for each sample. The results of QC are automatically displayed in the Intensity QC Table. Samples, which pass the QC threshold, (call rate > 86% and contrast QC > 0.4) are noted "bound in", and those, which did not pass the QC (call rate < 86% or contrast QC < 0.4) are noted "bound out". The genomic DNA samples of study passed all QC.

Genome-wide scan were carried to identify SNPs marginally associated with acute inflammatory reactions (AIRs); change in cartilage volume and change in WOMAC total scores. The list of SNPs with marginal association (empirical p-value < 5% after n=1000 permutations) can be consulted from Table 1. No SNPs were marginally associated with change in total cartilage volume.

*2.5. TaqMan SNP genotyping (Candidate gene)*

TaqMan SNP Genotyping was performed to detect selected markers based on literature information. A total of 19 SNPs distributed onto 8 candidate genes were selected and carried out in two periods (see Tables 2a and 2b). In a TaqMan® SNP Genotyping assay, two locus-specific PCR primers surrounding the SNP are used to amplify a fragment of about 100 bp (see for instance Table 3). Two allele-specific probes are then hybridized to their specific SNP sequence. Each probe was labeled at its 5' extremity with either a fluorescent reporter dye (FAM), either the VIC reporter dye. Each probe also has a non-fluorescent quencher dye, MGB, at the 3' end. In each PCR cycle, if the target sequence of the allele-specific probe is amplified, the probe will hybridize to the DNA during the annealing step and extend. When the DNA polymerase comes into contact with this hybridized probe, the reporter dye of the probe is cleaved from the probe leaving the quencher dye behind. In each cycle of the PCR, cleavage of the reporter dyes from one or both of the allele-specific probes causes an exponential increase in the fluorescent intensity. At PCR completion, the total fluorescence of each sample is read on the ABI 9700 (384-well format). If fluorescence is observed from only one probe, the sample is homozygous for this allele. If fluorescence is observed for both allele-specific probes, the sample is heterozygous for both alleles. If the probe does not hybridize, the fluorescence of the dye is "quenched" or reduced by the quencher dye, and thus minimal fluorescence is observed, indicating a failed genotype.

Protocol is detailed in the datasheet of TaqMan® SNP Genotyping.

Period 1: DNA samples were genotyped with 17 TaqMan® SNP assays (see Table 2a).
Period 2: DNA samples were genotyped with 2 further TaqMan® SNP assays (see table 2b).

For each TaqMan® SNP assays, the NTC cluster was specific and all NTCs were undetermined, the three distinct sample clusters were present and genotyping was automatically assigned and the call rate was specified to be above 85 percent.

For each of the 19 TaqMan® SNP assays in the three parts, acceptance criteria were reached.

*2.6. SNP filtering*

Candidate and whole genome scan SNP markers were not kept for analysis if any of the following criteria was met:

- Rare variant SNP in the PGx ITT population: Minor Allele Frequency (MAF) < 10% for both candidate SNP and whole genome scan SNPs.
- Questionable genotyping quality, as measured by a high rate ≥5%) of missing data.
- Significant deviation from the Hardy-Weinberg equilibrium (Bonferroni adjusted p value less than 5% for candidate SNPs or FDR (i.e. Benjamini-Hochberg adjusted p value) less than 20% for whole genome scan SNPs).
- Subjects with gender discrepancy between the clinical database and the predicted gender from whole genome scan SNP data (chromosome X) are excluded.

*2.7. Association tests*

For association tests, genotype data were coded as presence/absence of the SNP minor allele (i.e. homozygous for major allele compared to at least one copy of the minor allele).

2.7.1. Association with Acute Inflammatory Reactions (AIRs)

In these analyses, only subjects treated with 100mcg FGF18 dose were used. For single marker analysis, two approaches were used: Fisher's exact test and a multivariate linear model (i.e. AIR status ~ SNP + Kellgren Lawrence grade [2; 3] + Gender [Female; Male] + Age [< 65; ≥ 65] + BMI [<30, ≥30]. In this model, significance of each term in the model was assessed with a type III anova).

2.7.2. Association with WOMAC total scores and total cartilage volume

Association between change from baseline at week 52 (termination date), both for WOMAC total scores and total cartilage volume, was assessed using the following linear model:

$$\text{Rank(change in endpoint)} \sim \text{Arm [Placebos, Treated subjects for.e.g. with FGF-18 100 mcg dose]} + \text{genotype group} + \text{Kellgren Lawrence grade [2; 3]} + \text{Gender [Female; Male]} + \text{Age} [< 65; \geq 65] + \text{BMI} [<30, \geq 30].$$

Significance of each term in the model was assessed with a type III anova and significance threshold was set at alpha=5%.

2.7.3. Association between a given genotype group and Kellgren-Lawrence grade

To test whether a given genotype group (for e.g. subjects with the 'IL-1 RN rs9005 G/G and IL-1RN rs315259 T/T' genotype) had a significant enrichment or paucity in subjects with severe osteoarthritis (i.e. Kellgren-Lawrence grade 3) independence tests were performed using a Fisher's exact test and from the following contingency table:

| | Grade 3 | Grade 2 |
|---|---|---|
| # of subjects from a given genotype group | | |
| # of subjects from the remaining genotype groups | | |

All available subjects from any dose regimen (including placebos) were included in this analysis. P-values were computed using a two-sided test and significance was set at alpha = 5%. Odds ratio and their 95% confidence intervals were also computed.

*2.8. Haplotype analyses*

Genotype data from SNPs rs419598, rs315952, rs9005 were phased (using the MACH software, version 1.0.18.c, Li Y et al., 2010) to infer presence or absence of the C-T-A haplotype in subjects. The following MACH parameters were used: "--rounds 50 --states 200 --phase". Association with AIRs was tested using a Fisher's exact test (significance threshold set at alpha=5%).

*2.9. Combinatorial analyses between candidate SNPs*

In initial association analyses (data not shown), the rs9005 SNP was found significantly associated with AIRs. Combinatorial analyses (i.e. epistasis) were performed to test whether IL-1RN rs9005 in combination of another SNP, from a list of about 120 candidate SNPs, would be a better AIR predictor (see Table 1). Such analysis was performed using a logistic regression with the following model:

$$\text{AIR status} \sim \text{rs9005} * \text{another SNP} + \text{Kellgren Lawrence grade [2; 3]} + \text{Gender [Female; Male]} + \text{Age} [< 65; \geq 65] + \text{BMI} [<30, \geq 30].$$

Significance of each term in the model was assessed with a type III anova. Interaction p-values were adjusted for multiple-testing using the Benjamini-Hochberg procedure (Benjamini and Hochberg, 1995, J. of the Royal Statistical Society Series B(57):289) and significance threshold was set at FDR=5%. Epistasis effects were confirmed using the statistical approach described in (Wirapati et al., 2011).

*2.10. Performance metrics at predicting AIRs*

Performance metrics at predicting AIRs were derived from the corresponding contingency table. These metrics included sensitivity, specificity, accuracy, precision, negative predictive value and F1 score (i.e. harmonic mean of precision and recall).

## 3. Results

*3.1. Predictive analyses*

Combinatorial analyses identified only one combination (IL-1RN rs9005 and IL-1RN rs315259) as significantly associated with AIRs (FDR from multivariate linear model = 0.0187, Fisher's exact test p-value = 0.0018, odds ratio = 18.82 [2.25-260.03]). Contingency table and prediction performance metrics are shown respectively in Table 5 and Table 6. The combination of rs9005 and rs315259 (Table 6) has a better performance at predicting AIRs, compared to the C-T-A haplotype (Table 8 see also contingency table in Table 7). The combination of IL-1RN rs9005 and IL-1RN rs315259 has a very strong specificity (94.44%) and negative predictive value (89.47%), i.e. these biomarkers have a very strong performance at identifying subjects that will not have AIRs. In addition, this combination reveals stratification on total cartilage volume (Figure 4) and WOMAC total scores (Figure 5). By contrast, the C-T-A haplotype does not allow such clinical outcome stratification (Figures 2 and 3). Indeed, the C-T-A haplotype did not allow stratifying subjects for change in total cartilage volume (Figure 2) nor change in WOMAC total score (Figure 3). Thus the C-T-A haplotype was not identified as a good predictor of the response to drug therapy, preferably an anabolic drug such as sprifermin.

*3.2. Prognostic analyses*

Placebo subjects with the 'IL-1RN rs9005 G/G and IL-1RN rs315259 T/T' genotype were identified as having significantly higher total cartilage volume than treated subjects from the same genotype group. To follow-up on this result, change in WOMAC total score and change in total cartilage volume were modeled in placebo subjects with the following formula:

$$\text{Rank(change in endpoint)} \sim \text{genotype group} + \text{Kellgren Lawrence grade } [2;\, 3] + \text{Gender [Female; Male]} + \text{Age } [< 65;\, \geq 65] + \text{BMI } [<30,\, \geq 30].$$

No significant difference in WOMAC total score was found between subjects from the four different genotype groups (p-value = 0.63, Table 10). However significant differences were found in change in total cartilage volume (p-value = 0.02, Table 9). Subjects from the 'IL-1RN rs9005 G/G and IL-1RN rs315259 T/T' genotype group have significantly higher total cartilage volume increase compared to subjects from the remaining genotype groups.

Independence test between the Kellgren-Lawrence grade and subjects from a given genotype group demonstrated that the 'IL-1RN rs9005 G/G and IL-1RN rs315259 T/T' genotype group has a significant paucity in subjects from Kellgren-Lawrence grade 3 (Fisher's exact test p-value = 0.0179, Table 11). The corresponding odds ratio is 0.306 (with 95% confidence intervals [0.096, 0.885]). This demonstrates that subjects from the 'IL-1RN rs9005 G/G and IL-1RN rs315259 T/T' genotype group are classified with a less severe osteoarthritis condition than subjects from other genotype groups. Lending support to this result, subjects from the 'IL-1RN rs9005 G/G and IL-1RN rs315259 T/T' genotype group have marginally smaller baseline WOMAC total scores than subjects from other genotype groups (ranksum p-value = 0.0927, see Figure 6). In addition, subjects from the 'IL-1RN rs9005 GG and IL-1RN rs315259 TT' genotype group have significantly higher baseline total cartilage volume than subjects from other genotype groups (ranksum p-value = 0.0204, see Figure 8).

Interestingly, there was no difference in the proportion of subjects with Kellgren-Lawrence grade 3 between the 'IL-1 RN rs9005 A carriers and IL-1RN rs315259 C carriers' genotype group (aka super-sensitives) and subjects from the remaining genotype groups (Fisher's exact test p-value = 0.2736, odds ratio=1.693 [0.637, 4.769], Table 12). Thus the super-sensitive group is not enriched in subjects with severe osteoarthritis condition. This is further enforced with the fact that both baseline WOMAC total scores and baseline total cartilage volume are comparable between super-sensitives subjects and other subjects (see Figures 7 and 9).

Analysis with the C-T-A haplotype, did not reveal difference in the proportion of subjects with Kellgren-Lawrence grade 3 and bearing at least one copy of the C-T-A haplotype (Fisher's exact test p-value = 1).

*3.3. Clinical outcome using the proposed genetic diagnostic test*

Without any genetic stratification, the clinical outcomes of the FGF18 therapy are the following: 1) significant increase in total cartilage volume in treated subjects (MAD100) compared to placebos (p-value = 0.0157); 2) marginally smaller improvement in WOMAC total scores in treated subjects (MAD100) compared to placebos (p-value = 0.1044); 3) 20% of AIRs in treated subjects. These results are summarized in Table 13 and detailed results are presented in Table 14 and Table 15. Figures 10 and 13 are also provided for data visualization. It is understood that figures 10 to 15 do not correspond to the multivariate linear model used for the analyses. These figures are only provided to facilitate results interpretation.

The proposed diagnostic test (Table 4) aims at:

1. Identifying sensitives and treat them with the proposed FGF18 dose (e.g. 100 mcg)
2. Identifying super-sensitives and treat them with a lower FGF18 dose (e.g. 30 mcg)
3. Identifying non-sensitives and exclude them from FGF18 therapy.

Retrospectively, the clinical outcomes, for subjects elected for FGF18 therapy, are

1. Significant increase in total cartilage volume in treated subjects (sensitives from MAD100 cohort + super-sensitives from MAD030 cohort) compared to matched placebos (p-value = 0.0016 Table 18, Figure 14). Simulation studies (bootstrap) showed that this cartilage volume improvement is significantly higher than the improvement obtained when no diagnostic test is used (p-value < 1E-4)
2. Comparable improvement in WOMAC total scores between treated subjects and placebos (p-value = 0.6603, Table 17, Figure 11)
3. 11.43% of AIRs in treated subjects (Table 16)

By contrast, subjects identified as non-sensitives have the following clinical outcomes:

1. Significantly lower improvement in total cartilage volume in treated subjects (non-sensitives from MAD100 cohort) compared to matched placebos (p-value = 0.0289, Table 21) Subjects from the MAD030 cohort had similar outcome than subjects from the MAD100 cohort (Figure 15). Thus none of the investigated dose showed an improvement with respect to placebos.
2. Although the p-value from the multivariate linear model is not significant (p-value= 0.3068, Table 20), there is no improvement in WOMAC total score for treated subjects (median change = -1) while there is some improvement for placebos (median change = -39). Subjects from the MAD010 and MAD030 cohorts had similar outcome than subjects from the MAD100 cohort (Figure 12). Thus none of the investigated dose showed an improvement with respect to placebos.
3. 22.22% of AIRs in treated subjects (Table 19)

It is anticipated that similar results will be achieved with other anabolic drugs such as BMP-2, BMP-7, GDF-5, FGFβ, FGF-8, FGF-9, SOX-9 enhancers, TGFβ, and any variants thereof.

**Tables**

**Table 1: List of candidate SNPs**

| Gene / description | Tested SNPs |
|---|---|
| FGF18 | rs3806929, rs4073716, rs9313543, rs4076077, rs4073717, rs6555956, rs10065728, rs4620037, rs11553493 |
| FGFR1 | rs2288696, rs2978073, rs11777067, rs6983315, rs7012413, rs6996321 |
| FGFR2 | rs3135810, rs2278202, rs1649200, rs7090018, rs2912759, rs2912787, rs2981449, rs2981432, rs10736303, rs1078806, rs2981575, rs1219648, rs1219643, rs2912774, rs2162540, rs2981582, rs3135715, rs3750819, rs755793 |
| FGFR3 | rs17880763, rs17881656, rs17882190, rs17884368 |
| FGFR4 | rs442856, rs422421, rs2011077 |
| FGFRL1 | rs4647934 |
| IL10 | rs1878672, rs3024493, rs1554286, rs3024491, rs3024490 |
| IL1A | rs1304037, rs3783550, rs3783525, rs1800587 |
| IL1B | rs1143627, rs1143634, rs1143633, rs3136558 |
| IL1RN | rs9005, rs315952, rs444413, rs3181052, rs419598, rs423904, rs442710, rs447713, rs451578, rs432014, rs431726, rs452204, rs3087266, rs579543 |

(continued)

| Gene / description | Tested SNPs |
|---|---|
| IL6 | rs1800795, rs1800797, rs1474347, rs2069840, rs1800796 |
| marginal association with AIRs (from whole-genome scan) | rs5934659, rs12407610, rs1344049, rs10954969, rs1522844, rs2685592, rs6697273, rs887071, rs1105227, rs6846033, rs871746, rs11815080, rs6949763, rs897718, rs7651624, rs6989732, rs7786717, rs10093384, rs11737974, rs3122569, rs12453065, rs1992509, rs2202731, rs6897534, rs747159, rs4342357, rs2447011, rs4770271, rs10430746, rs7032155, rs10948190, rs7073333, rs6495812, rs946120, rs1047813, rs2032790, rs3865404, rs11040899, rs1968294, rs723077 |
| marginal association with WOMAC total score (from whole-genome scan) | rs12410403, rs587505, rs9902708, rs734397, rs894013, rs932241 |
| TNFRS1B | rs1061622 |
| VDR region | rs731236, rs7975232, rs1544410 |

**Table 2a: Details of TaqMan SNP Id screened in period 1**

| Gene Symbol | rs Id | Assay Id | NCBI alleles | Assay type |
|---|---|---|---|---|
| FGF18 | rs4073716 | C__27537611_10 | C/T | Functionally Tested |
| FGF18 | rs11553493 | NA | G/T | Custom |
| FGF18,NPM1 | rs3806929 | C__11274941_10 | C/T | Functionally Tested |
| FGFR2 | rs755793 | C___2414603_10 | C/T | Validated |
| FGFR3 | rs17881656 | NA | C/T | Custom |
| FGFR3,LETM1 | rs17880763 | C__58182661_10 | A/T | Functionally Tested |
| FGFR3,LETM1 | rs17882190 | C__58182657_10 | A/G | Functionally Tested |
| IL1B | rs1143627 | C___1839944_10 | C/T | Validated |
| IL-6 | rs1800795 | hCV1839697 | C/G | Custom/SNPlex system |
| IL6,LOC541472 | rs1800796 | C__11326893_10 | C/G | Functionally Tested |
| LETM1,FGFR3 | rs17884368 | C__58182646_10 | A/G | Functionally Tested |
| LOC100131885,FGFR2 | rs3750819 | C__27511529_10 | C/G | Functionally Tested |
| LOC541472,IL6 | rs1800797 | C___1839695_20 | A/G | Functionally Tested |
| TNFRSF1B | rs1061622 | C___8861232_20 | G/T | Functionally Tested |
| VDR | rs7975232 | C__28977635_10 | A/C | Functionally Tested |
| VDR | rs731236 | C___2404008_10 | C/T | Functionally Tested |
| VDR | rs1544410 | C___8716062_10 | A/G | Validated |

**Table 2b: Details of TaqMan SNP Id screened in period 2**

| Gene Symbol | rs Id | Assay Id | NCBI alleles | Assay type |
|---|---|---|---|---|
| IL1RN | rs9005 | C_3133528_10 | A/G | Functionally Tested |
| IL1RN | rs315952 | C_11512470_10 | C/T | Validated |

**Table 3: Taqman primer sequences**

| SNP Reference | Applied Biosystems assay ID | Primer sequences |
|---|---|---|
| rs315952 | C_11512470_10 | Primer 1: GCTTCGCCTTCATCCGCTCAGACAG or complementary sequence<br>Primer 2: GGCCCCACCACCAGTTTTGAGTCTG or complementary sequence |
| rs9005 | C_3133528_10 | Primer 1: TGTGCCTCTGCCTGTCTCCCCCACC or complementary sequence<br>Primer 2: GGCTGGGAGCTCTGCAGAGCAGGAA or complementary sequence |

**Table 4: Identified genotype categories in the Multiple Ascending Dose cohort (100 mcg)**

| | | **rs9005 (A/G)** | |
|---|---|---|---|
| | | **GG** | **A carriers** |
| **rs315952 (T/C)** | **TT** | **group A:**<br>non-sensitives (20% of MAD100) | **group B:**<br>**Sensitives (27% of** MAD100) |
| | **C carriers** | **group C:**<br>Sensitives (38% of MAD100) | **group D:**<br>super-sensitives (15% of MAD100) |

**Table 5: Contingency table: AIR predictions based on rs9005 and rs315952 genotypes with subjects from the FGF18 MAD100 arm (n=45)**

| | | **True AIR status** | |
|---|---|---|---|
| | | **Subjects with AIRs** | **Subjects without AIRs** |
| **Predicted status** | **Predicted with AIRs** | 5 | 2 |
| | **Predicted without AIRs** | 4 | 34 |

**Table 6: Performance at predicting AIRs based on rs9005 and rs315952 genotypes with subjects from the FGF18 MAD100 arm (n=45)**

| **Performance metrics** | **value** |
|---|---|
| Sensitivity | 55.56% |
| Accuracy | 86.67% |
| Specificity | 94.44% |
| Precision | 71.43% |
| Negative predictive value | 89.47% |
| Sensitivity and precision (F1 score) | 62.50% |

**Table 7: Contingency table: AIR predictions based on presence/absence of the C-T-A haplotype with subjects from the FGF18 MAD100 arm (n=48)**

| | | True AIR status | |
|---|---|---|---|
| | | Subjects with AIRs | Subjects without AIRs |
| Predicted status | Predicted with AIRs | 6 | 7 |
| | Predicted without AIRs | 4 | 31 |

**Table 8: Performance at predicting AIRs based on presence/absence of the C-T-A haplotype with subjects from the FGF18 MAD100 arm (n=48)**

| Performance metrics | value |
|---|---|
| Sensitivity | 60% |
| Accuracy | 77.08% |
| Specificity | 81.58% |
| Precision | 46.15% |
| Negative predictive value | 88.57% |
| Sensitivity and precision (F1 score) | 52.17% |

**Table 9: Multivariate linear modeling for change in total cartilage volume with placebo subjects only**

| model term | regression coefficient | Standard Error | Z-score | p-value |
|---|---|---|---|---|
| **Intercept** | 78.44 | 23.68 | 3.31 | 0.0035 |
| **group [B-C-D; A only]** | 83.11 | 33.85 | 2.46 | 0.0234 |
| **Kellgren-Lawrence grade [2; 3]** | -12.93 | 22.36 | -0.58 | 0.5695 |
| **Aqe [< 65; >= 65]** | -15.83 | 20.86 | -0.76 | 0.4569 |
| **BMI [<30; >= 30]** | 4.02 | 21.61 | 0.19 | 0.8545 |
| **Gender [Female; Male]** | -15.01 | 20.31 | -0.74 | 0.4683 |

**Table 10: Multivariate linear modeling for change in WOMAC total score with placebo subjects only**

| model term | regression coefficient | Standard Error | Z-score | p-value |
|---|---|---|---|---|
| **Intercept** | 63.76 | 20.40 | 3.13 | 0.0051 |
| **group [B-C-D; A only]** | -13.98 | 28.71 | -0.49 | 0.6313 |
| **Kellgren-Lawrence grade [2; 3]** | -25.47 | 18.83 | -1.35 | 0.1906 |
| **Age [< 65; >= 65]** | 0.34 | 17.66 | 0.02 | 0.9847 |
| **BMI [<30; >= 30]** | 46.97 | 18.08 | 2.60 | 0.0168 |
| **Gender [Female; Male]** | 29.74 | 17.01 | 1.75 | 0.0950 |

**Table 11: Contingency table: Kellgren-Lawrence grade (3 or 2) based on presence/absence of the 'rs9005 GG rs315952 TT' genotype** - Analysis was performed using all subjects from all dose regimen (including placebos). Fisher's exact test p-value is 0.0179, odds ratio is 0.306 with 95% confidence interval [0.096, 0.885].

| genotype | Grade 3 | Grade 2 |
|---|---|---|
| rs9005 GG rs315952 TT | 7 | 15 |

(continued)

| genotype | Grade 3 | Grade 2 |
|---|---|---|
| other | 60 | 39 |

**Table 12: Contingency table: Kellgren-Lawrence grade (3 or 2) based on presence/absence of the 'rs9005 A carriers rs315952 C carriers' genotype** - Analysis was performed using all subjects from all dose regimen (including placebos). Fisher's exact test p-value is 0.2736, odds ratio is 1.693 with 95% confidence interval [0.637, 4.769].

| genotype | Grade 3 | Grade 2 |
|---|---|---|
| rs9005 A carriers rs315952 C carriers | 17 | 9 |
| other | 50 | 45 |

**Table 13: Clinical outcome without diagnostic test (45 subjects treated with FGF18 100mcg and 27 placebos)** - Delta corresponds to the difference between the median change in placebos and the median change in treated subjects. P-value corresponds to the p-value from a multivariate linear model adjusting for gender, age, BMI and KL grade.

| Groups A, B, C, D | median change in placebos | median change in treated subjects (MAD100) | delta | p-value |
|---|---|---|---|---|
| **Change in WOMAC total score** | -19 | -10 | 9 | 0.1044 |
| **Change in total cartilage volume** | -44.68 | 102.25 | 146.93 | 0.0157 |
| **%AIRs** | 3.7 | 20 | 16.3 | NA |

**Table 14: Multivariate linear modeling for change in WOMAC total score with all placebos and all MAD100 treated subjects**

| model term | regression coefficient | Standard Error | Z-score | p-value (GLM) | LR Chi-square (anova) | p-value (anova) |
|---|---|---|---|---|---|---|
| Intercept | 59.51 | 14.86 | 4.00 | 0.0002 | NA | NA |
| Age [< 65; >= 65] | 11.07 | 12.61 | 0.88 | 0.3834 | 0.77 | 0.3802 |
| Arm (dose 100mcg) | 19.51 | 12.02 | 1.62 | 0.1091 | 2.64 | 0.1044 |
| BMI [<30; >= 30] | 13.24 | 12.09 | 1.10 | 0.2774 | 1.20 | 0.2734 |
| Gender [Female; Male] | 20.13 | 11.84 | 1.70 | 0.0937 | 2.89 | 0.0890 |
| Kellgren-Lawrence grade [2;3] | 0.14 | 11.79 | 0.01 | 0.9902 | 0.00 | 0.9902 |

**Table 15: Multivariate linear modeling for change in total cartilage volume with all placebos and all MAD100 treated subjects**

| model term | regression coefficient | Standard Error | Z-score | p-value (GLM) | LR Chi-square (anova) | p-value (anova) |
|---|---|---|---|---|---|---|
| Intercept | 79.10 | 13.37 | 5.92 | 0.0000 | NA | NA |
| Age [< 65; >= 65] | -15.10 | 11.35 | -1.33 | 0.1878 | 1.77 | 0.1832 |
| Arm [placebos; treated] | 26.12 | 10.81 | 2.42 | 0.0185 | 5.84 | 0.0157 |
| BMI [<30; >= 30] | -14.60 | 10.87 | -1.34 | 0.1841 | 1.80 | 0.1795 |
| Gender [Female; Male] | 1.05 | 10.65 | 0.10 | 0.9216 | 0.01 | 0.9213 |
| Kellgren-Lawrence grade [2;3] | -1.25 | 10.61 | -0.12 | 0.9065 | 0.01 | 0.9061 |

**Table 16: Clinical outcome for subjects classified as 1) sensitives (groups B and C, n=29, treated with FGF18 100 mcg) or 2) super-sensitives (group D, n=6, treated with a lower FGF18 dose: 30 mcg). 24 placebos, with genotypes from either group B, C or D, were included in the analysis** - Delta corresponds to the difference between the median change in placebos and the median change in treated subjects. P-value corresponds to the p-value from a multivariate linear model adjusting for gender, age, BMI and KL grade.

| Groups B, C, D | median change in placebos | median change in treated subjects (MAD100+MAD30) | delta | p-value |
|---|---|---|---|---|
| **Change in WOMAC total score** | -16.5 | -13 | 3.5 | 0.6603 |
| **Change in total cartilage volume** | -114.91 | 102.25 | 217.16 | 0.0016 |
| **%AIRs** | 0 | 11.43 | 11.43 | NA |

**Table 17: Multivariate linear modeling for change in WOMAC total score with subjects classified as 1) sensitives (groups B and C, n=29, treated with FGF18 100 mcg) or 2) super-sensitives (group D, n=6, treated with a lower FGF18 dose: 30 mcg). 24 placebos, with genotypes from either group B, C or D, were included in the analysis.**

| model term | regression coefficient | Standard Error | Z-score | p-value (GLM) | LR Chi-square (anova) | p-value (anova) |
|---|---|---|---|---|---|---|
| Intercept | 67.09 | 16.25 | 4.13 | 0.0001 | NA | NA |
| Age [< 65; >= 65] | 7.23 | 13.33 | 0.54 | 0.5900 | 0.29 | 0.5877 |
| Arm [placebos; treated] | 5.82 | 13.24 | 0.44 | 0.6621 | 0.19 | 0.6603 |
| BMI [<30; >= 30] | 3.87 | 12.82 | 0.30 | 0.7641 | 0.09 | 0.7629 |
| Gender [Female; Male] | 16.54 | 13.68 | 1.21 | 0.2322 | 1.46 | 0.2268 |
| Kellgren-Lawrence grade [2;3] | 6.67 | 13.09 | 0.51 | 0.6124 | 0.26 | 0.6103 |

**Table 18: Multivariate linear modeling for change in total cartilage volume with subjects classified as 1) sensitives (groups B and C, n=29, treated with FGF18 100 mcg) or 2) super-sensitives (group D, n=6, treated with a lower FGF18 dose: 30 mcg). 24 placebos, with genotypes from either group B, C or D, were included in the analysis.**

| model term | regressio n coefficient | Standard Error | Z-score | p-value (GLM) | LR Chi-square (anova) | p-value (anova) |
|---|---|---|---|---|---|---|
| Intercept | 64.94 | 14.46 | 4.49 | 0.0000 | NA | NA |
| Age [< 65; >= 65] | -15.89 | 11.86 | -1.34 | 0.1860 | 1.79 | 0.1803 |
| Arm [placebos; treated] | 37.14 | 11.78 | 3.15 | 0.0027 | 9.94 | 0.0016 |
| BMI [<30; >= 30] | -5.47 | 11.40 | -0.48 | 0.6332 | 0.23 | 0.6312 |
| Gender [Female; Male] | 1.14 | 12.18 | 0.09 | 0.9258 | 0.01 | 0.9254 |
| Kellgren-Lawrence grade [2;3] | 1.27 | 11.65 | 0.11 | 0.9137 | 0.01 | 0.9133 |

**Table 19: Clinical outcome for subjects classified as non-sensitives by the diagnostic test (MAD100 n=9, MADPL n=3) -** Delta corresponds to the difference between the median change in placebos and the median change in treated subjects. P-value corresponds to the p-value from a multivariate linear model adjusting for gender, age, BMI and KL grade.

| Group A only | median change in placebos | median change in treated subjects (MAD100) | delta | p-value |
|---|---|---|---|---|
| **Change in WOMAC total score** | -39 | -1 | 38 | 0.3068 |
| **Change in total cartilage volume** | 224.56 | 117.92 | -106.64 | 0.0289 |
| **%AIRs** | 33.33 | 22.22 | -11.11 | NA |

**Table 20: Multivariate linear modeling for change in WOMAC total score, with subjects classified as non-sensitives by the diagnostic test (MAD100 n=9, MADPL n=3)**

| model term | regressio n coefficient | Standard Error | Z-score | p-value (GLM) | LR Chi-square (anova) | p-value (anova) |
|---|---|---|---|---|---|---|
| Intercept | 38.99 | 40.18 | 0.97 | 0.3693 | NA | NA |
| Age [< 65; >= 65] | 1.62 | 45.92 | 0.04 | 0.9730 | 0.00 | 0.9718 |
| Arm [placebos; treated] | 43.10 | 42.18 | 1.02 | 0.3462 | 1.04 | 0.3068 |
| BMI [<30; >= 30] | 18.24 | 38.93 | 0.47 | 0.6558 | 0.22 | 0.6393 |
| Gender [Female; Male] | 43.01 | 33.47 | 1.29 | 0.2461 | 1.65 | 0.1987 |
| Kellgren-Lawrence grade [2;3] | -19.53 | 34.44 | -0.57 | 0.5911 | 0.32 | 0.5706 |

**Table 21: Multivariate linear modeling for change in total cartilage volume with subjects classified as non-sensitives by the diagnostic test (MAD100 n=9, MADPL n=3)**

| model term | regression coefficient | Standard Error | Z-score | p-value (GLM) | LR Chi-square (anova) | p-value (anova) |
|---|---|---|---|---|---|---|
| Intercept | 128.67 | 15.26 | 8.43 | 0.0002 | NA | NA |
| Age [< 65; >= 65] | 47.00 | 17.44 | 2.70 | 0.0358 | 7.27 | 0.0070 |
| Arm [placebos; treated] | -35.00 | 16.02 | -2.19 | 0.0715 | 4.78 | 0.0289 |
| BMI [<30; >= 30] | 30.67 | 14.78 | 2.07 | 0.0834 | 4.30 | 0.0380 |
| Gender [Female; Male] | -39.00 | 12.71 | -3.07 | 0.0220 | 9.42 | 0.0022 |
| Kellgren-Lawrence grade [2;3] | 7.00 | 13.08 | 0.54 | 0.6117 | 0.29 | 0.5925 |

**Table 22: Summary of clinical outcome and potential therapeutic options based on rs9005 and rs315952 genotypes**

| | Group A | Groups B & C | Group D | |
|---|---|---|---|---|
| | **100mcg** | **100mcg** | **100mcg** | **30mcg** |
| **Change in WOMAC total score** | Significant WOMAC worsening compared to placebo | Change in WOMAC comparable to placebo | Change in WOMAC higher than placebo | Change in WOMAC comparable to placebo |
| **Change in total cartilage volume** | No improvement | Significant cartilage volume improvement | Significant cartilage volume improvement (highest gain among all groups treated at 100mcg) | Highest cartilage volume improvement (significantly better than 100 mcg) |
| **AIRs** | 2/9 treated subjects (1/3 in placebos) | 2/29 treated subjects (0/17 in placebos) | 5/7 treated subjects (0/7 in placebos) | 2/6 treated subjects (0/7 in placebos) |
| **Potential therapeutic option** | Do not benefit from FGF18 therapy | Treat up to 100mcg | Treat at 30mcg | |

**References**

1) WO2008/023063
2) WO2004/032849
3) WO2006/063362
4) WO2009/135218
5) WO 92/15712
6) US 5,679,524
7) WO 91/02087
8) WO 90/09455
9) WO 95/17676
10) US 5,302,509
11) US 5,945,283

12) US5,605,798
13) WO 89/10414
14) http://www.cartilage.org/_files/contentmanagement/ICRS_evaluation.pdf
15) Lotz, 2010, Arthritis research therapy, 12:211
16) Ellsworth et al., 2002, Osteoarthritis and Cartilage, 10: 308-320
17) Shimoaka et al., 2002, J. Bio. Chem. 277(9):7493-7500
18) The Merck Manual, 17th edition, page 449
19) Bellamy et al., 1988, J.Rheumatology, 15:1833-1840
20) Wolfe, 1999, Rheumatology, 38:355-361
21) Attur et al., Ann. Rheum. Dis. 2010, 69:856-861
22) Li et al., 2010, Genet Epidemiol 34:816-834
23) Benjamini and Hochberg, 1995, J. of the Royal Statistical Society Series B(57):289
24) Wirapati et al., 2011, Ann. Hum. Genet. 75(1):133-45
25) Bateson W. and Mendel G., 1909, "G. Mendel's principles of heredity". Cambridge University Press; 1909. Available: http://archive.org/details/mendelsprinciple00bate
26) Phillips PC. ,1998, Genetics. 7;149(3):1167-71.
27) Cordell HJ., 2002, Hum. Mol. Genet. 11(20):2463-8.
28) Fisher RA, 1918, "The Correlation Between Relatives on the Supposition of Mendelian Inheritance". Available: http://digital.library.adelaide.edu.au/dspace/handle/2440/15097.
29) Browning SR and Browning BL, 2011, Nature Reviews Genetics. 12(10):703-14.

## Claims

**1.** A method of prognosing disorder severity in a subject having osteoarthritis, the method comprising the steps of:

a. Determining, from a nucleic acid sample, the presence of the genotype G/G at IL-1RN rs9005 and T/T at IL-1RN rs315952; and
b. Prognosing from the result of step a. less severe form of ostheoarthritis.

**2.** A method of prognosing disorder severity in a subject having osteoarthritis, the method comprising the steps of:

a. Determining, from a nucleic acid sample, the absence of the genotype G/G at IL-1RN rs9005 and T/T at IL-1RN rs315952; and
b. Prognosing from the result of step a. more severe form of ostheoarthritis.

## Patentansprüche

**1.** Verfahren zum Prognostizieren der Erkrankungsschwere bei einem Probanden mit Osteoarthritis, wobei das Verfahren die Schritte umfasst:

a. Bestimmen, aus einer Nukleinsäureprobe, des Vorhandenseins des Genotyps G/G bei IL-1RN rs9005 und T/T bei IL-1RN rs315952; und
b. Prognostizieren aus dem Ergebnis von Schritt a. einer weniger schweren Form von Osteoarthritis.

**2.** Verfahren zum Prognostizieren der Erkrankungsschwere bei einem Probanden mit Osteoarthritis, wobei das Verfahren die Schritte umfasst:

a. Bestimmen, aus einer Nukleinsäureprobe, der Abwesenheit des Genotyps G/G bei IL-1RN rs9005 und T/T bei IL-1RN rs315952; und
b. Prognostizieren aus dem Ergebnis von Schritt a. einer schwereren Form von Osteoarthritis.

## Revendications

**1.** Procédé pour la pose d'un pronostic de la gravité d'une affection chez un sujet ayant une arthrose, le procédé comprenant les étapes consistant à :

a. déterminer, à partir d'un échantillon d'acide nucléique, la présence du génotype G/G au niveau du locus IL-1RN rs9005 et T/T au niveau du locus IL-1RN rs315952 ; et
b. poser d'après le résultat de l'étape a. un pronostic de forme moins grave d'arthrose.

**2.** Procédé pour la pose d'un pronostic de la gravité d'une affection chez un sujet ayant une arthrose, le procédé comprenant les étapes consistant à :

a. déterminer, à partir d'un échantillon d'acide nucléique, l'absence du génotype G/G au niveau du locus IL-1RN rs9005 et T/T au niveau du locus IL-1RN rs315952 ; et
b. poser d'après le résultat de l'étape a. un pronostic de forme plus grave d'arthrose.

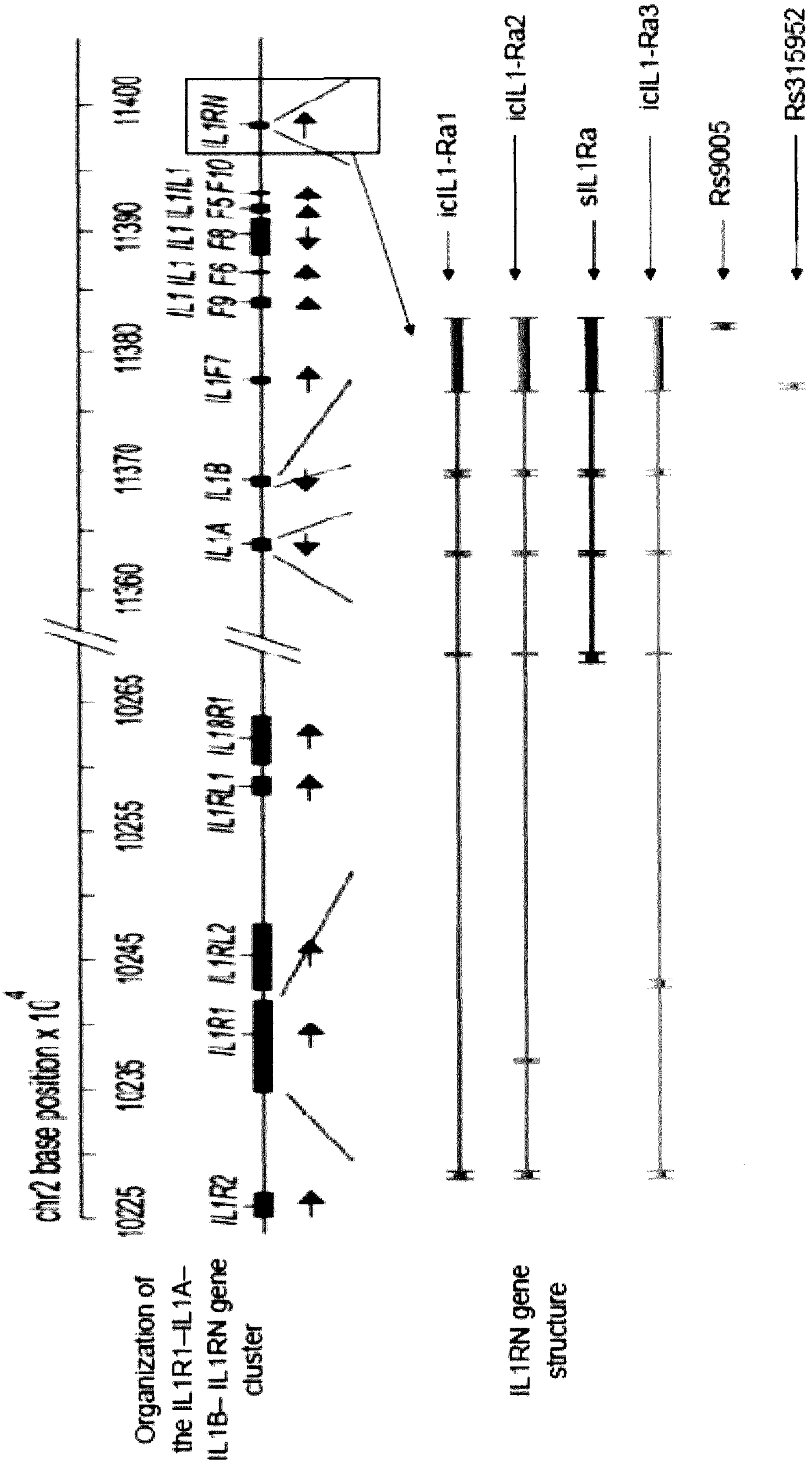
chr2 base position x 10^4
10225
10235
10245
10255
10265
11360
11370
11380
11390
11400
IL1R2
IL1R1
IL1RL2
IL1RL1
IL18R1
IL1A
IL1B
IL1F7
IL1F9
IL1F6
IL1F8
IL1F5
IL1F10
IL1RN
Organization of the IL1R1–IL1A–IL1B– IL1RN gene cluster
IL1RN gene structure
icIL1-Ra1
icIL1-Ra2
sIL1Ra
icIL1-Ra3
Rs9005
Rs315952

Fig. 1

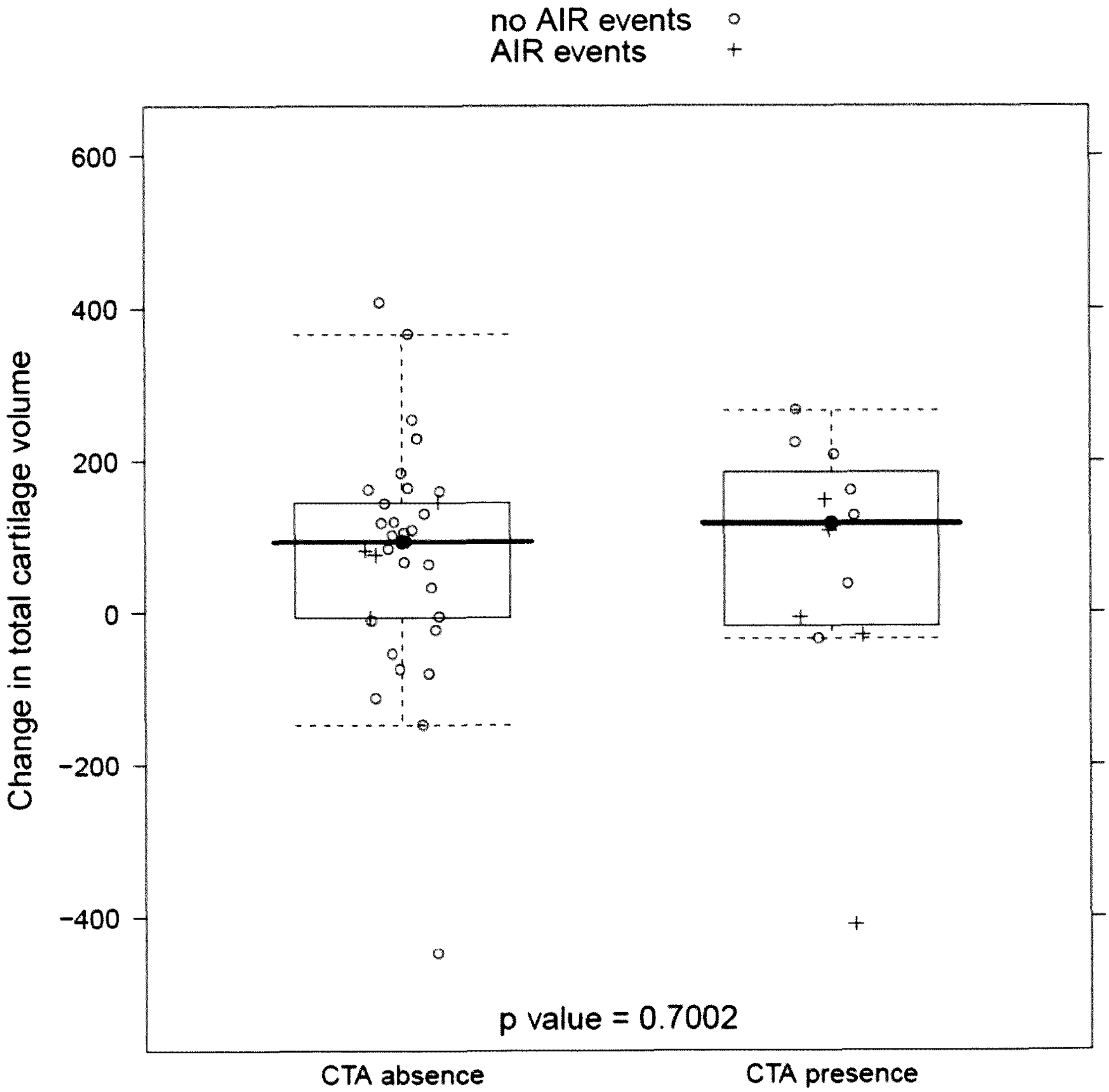
no AIR events
AIR events
600
400
200
0
-200
-400
Change in total cartilage volume
p value = 0.7002
CTA absence
CTA presence

Fig. 2

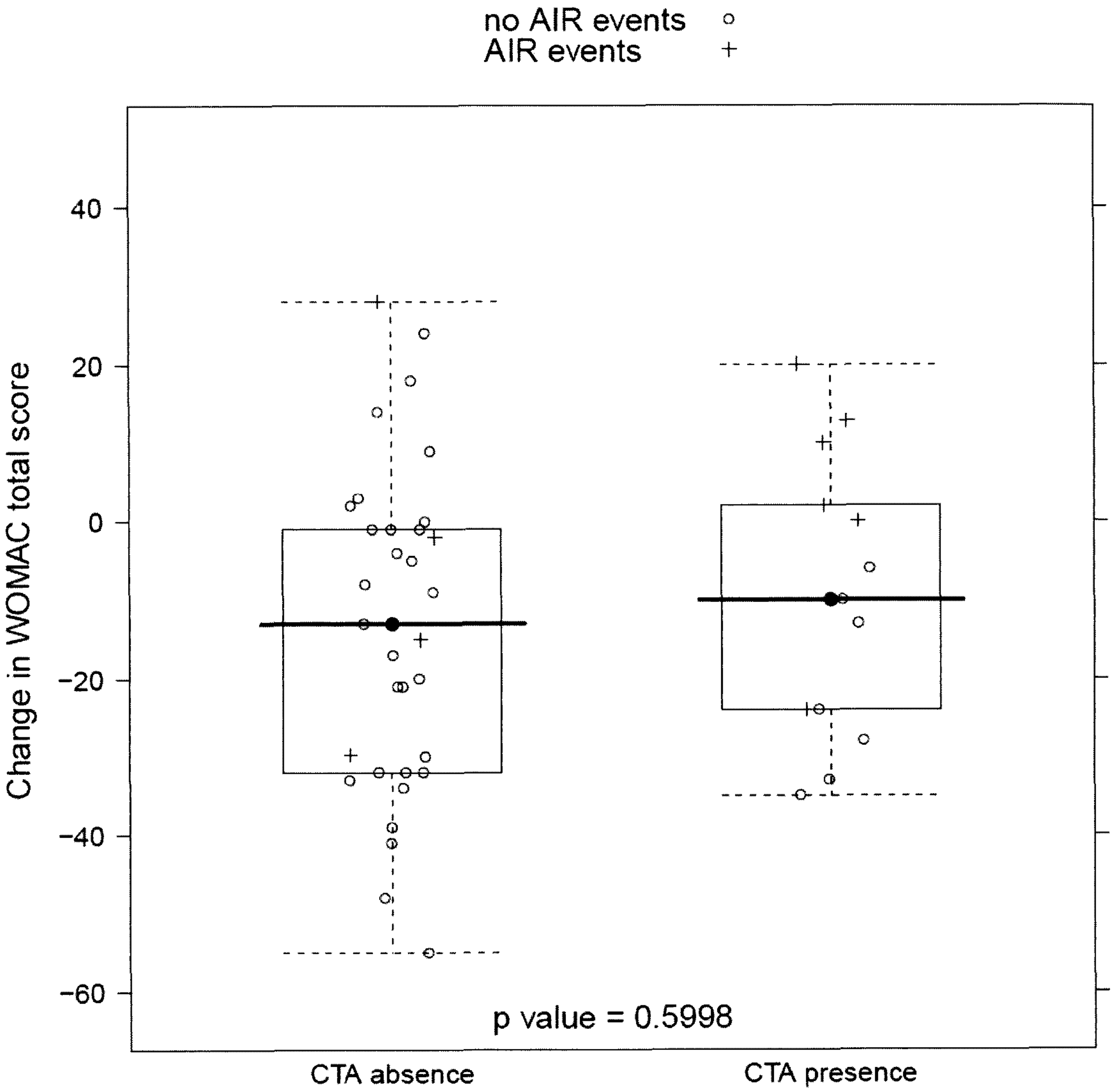
no AIR events
AIR events
40
20
0
-20
-40
-60
Change in WOMAC total score
p value = 0.5998
CTA absence
CTA presence

Fig. 3

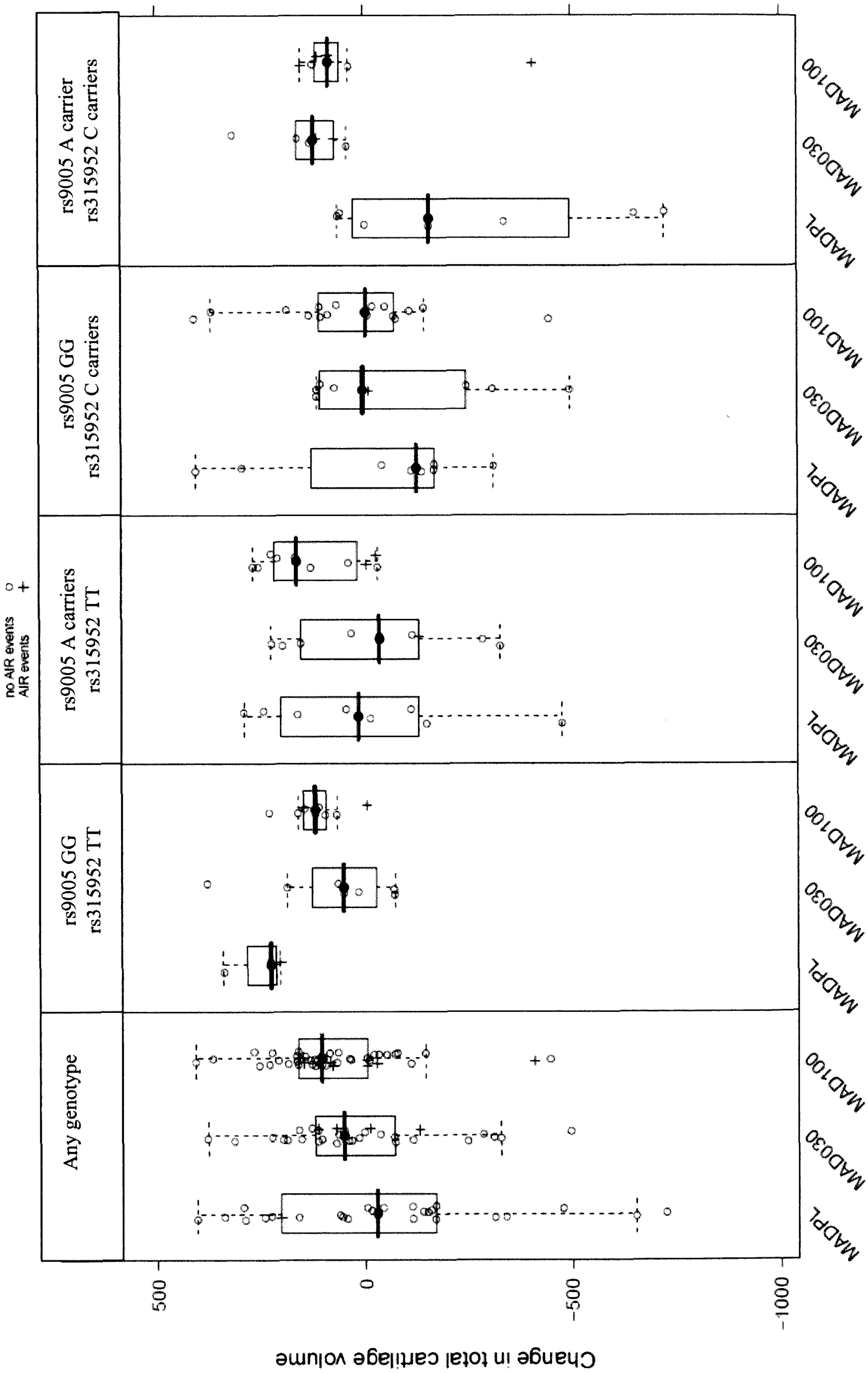
Any genotype
rs9005 GG
rs315952 TT
rs9005 A carriers
rs315952 TT
rs9005 GG
rs315952 C carriers
rs9005 A carrier
rs315952 C carriers
no AIR events
AIR events
MADPL
MAD030
MAD100
500
0
-500
-1000
Change in total cartilage volume

Fig. 4

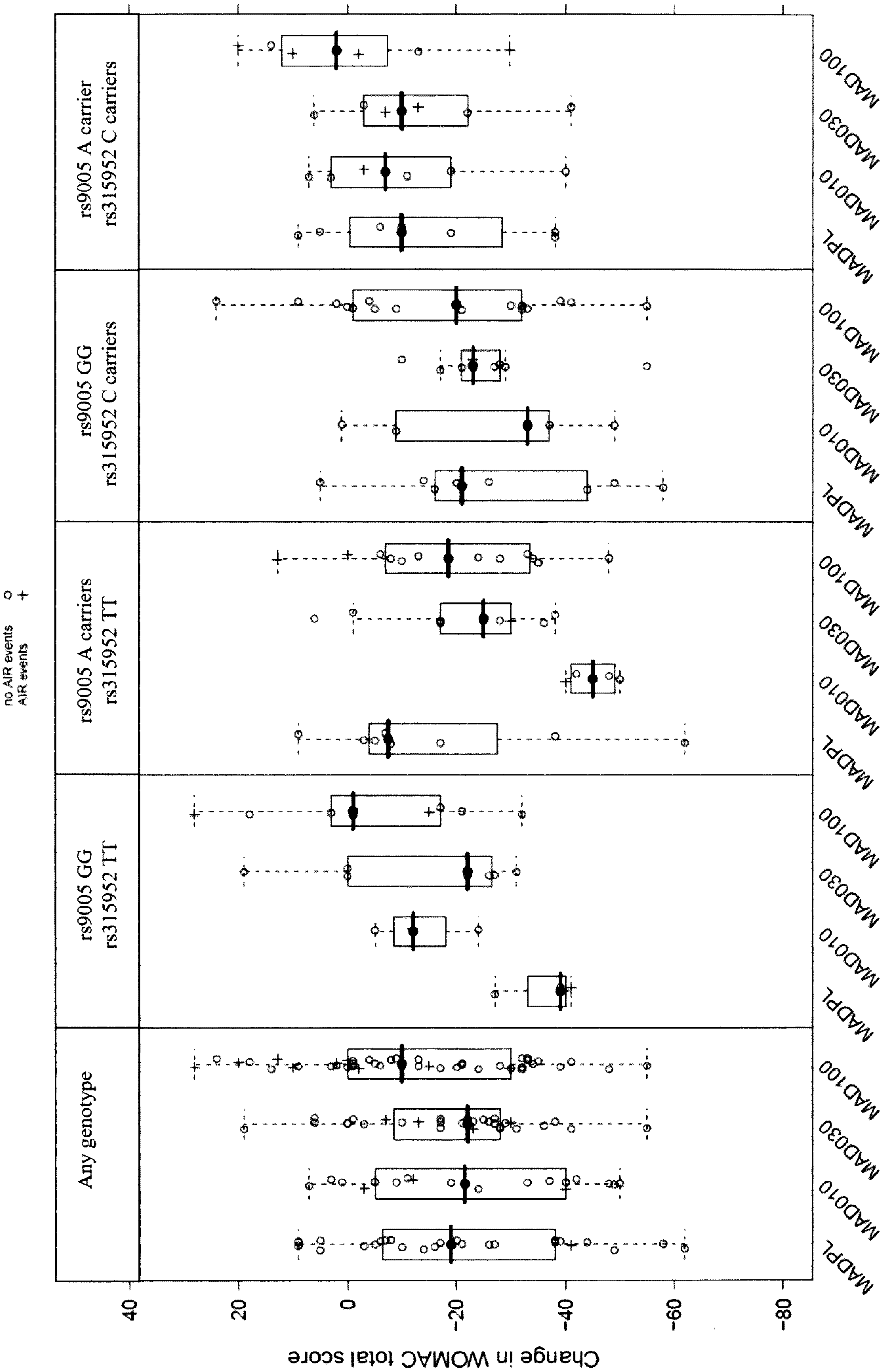
no AIR events
AIR events
Any genotype
rs9005 GG
rs315952 TT
rs9005 A carriers
rs315952 TT
rs9005 GG
rs315952 C carriers
rs9005 A carrier
rs315952 C carriers
MADPL
MAD010
MAD030
MAD100
40
20
0
-20
-40
-60
-80
Change in WOMAC total score

Fig. 5

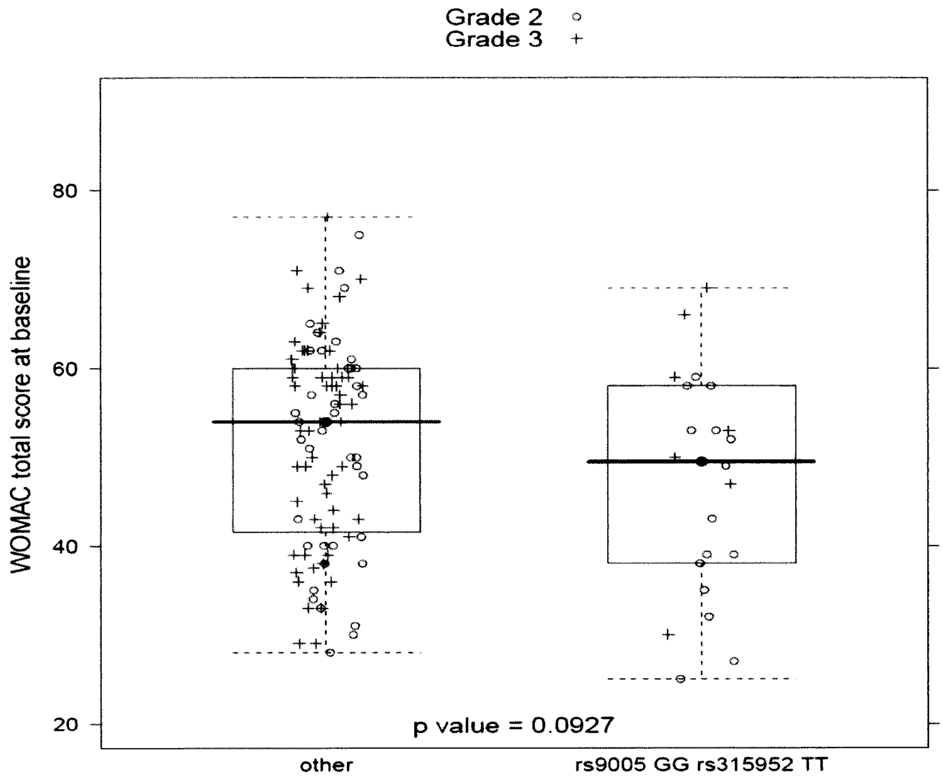
Grade 2
Grade 3
WOMAC total score at baseline
80
60
40
20
p value = 0.0927
other
rs9005 GG rs315952 TT

Fig. 6

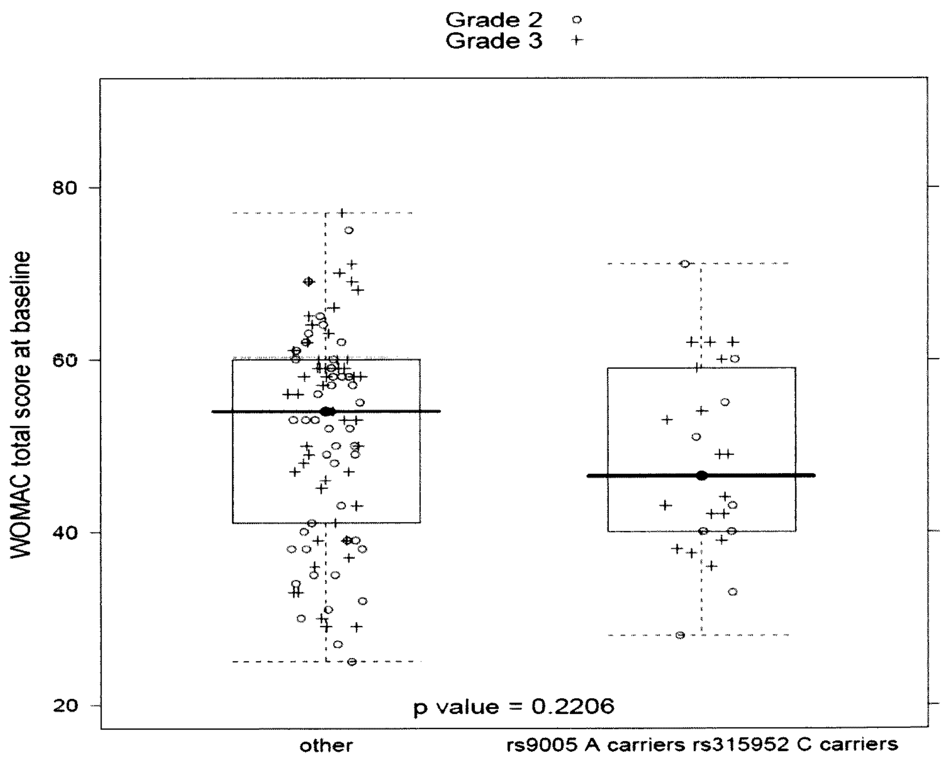
Grade 2
Grade 3
WOMAC total score at baseline
80
60
40
20
p value = 0.2206
other
rs9005 A carriers rs315952 C carriers

Fig. 7

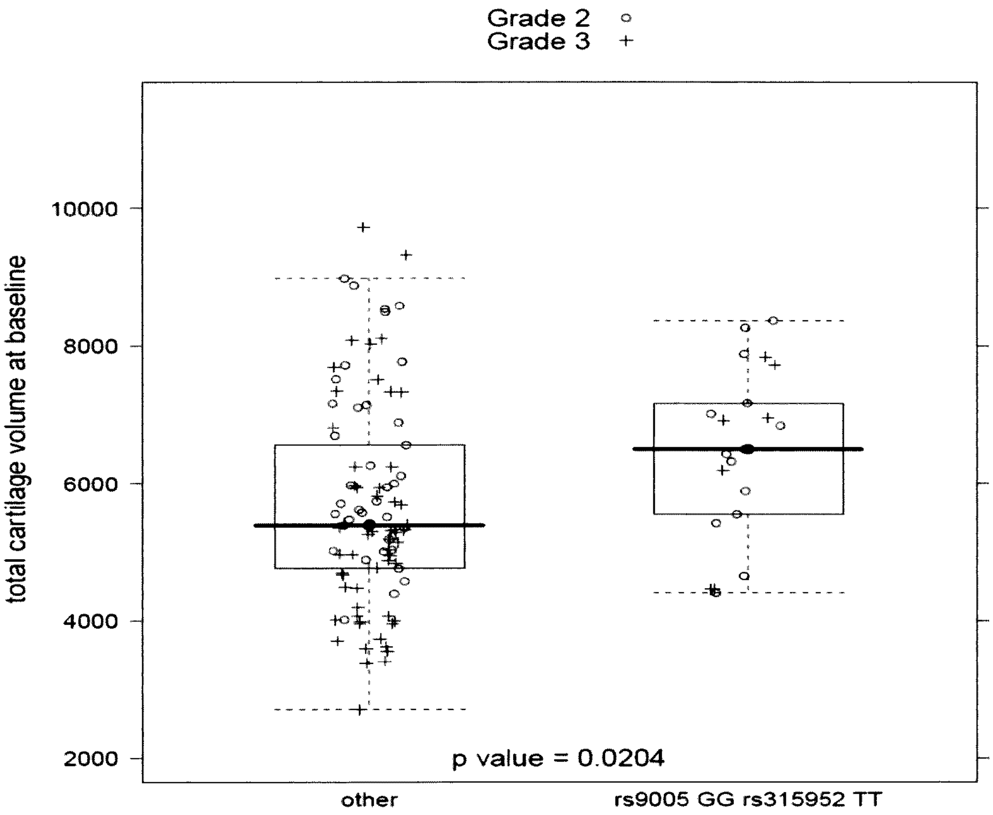
Grade 2
Grade 3
total cartilage volume at baseline
10000
8000
6000
4000
2000
p value = 0.0204
other
rs9005 GG rs315952 TT

Fig. 8

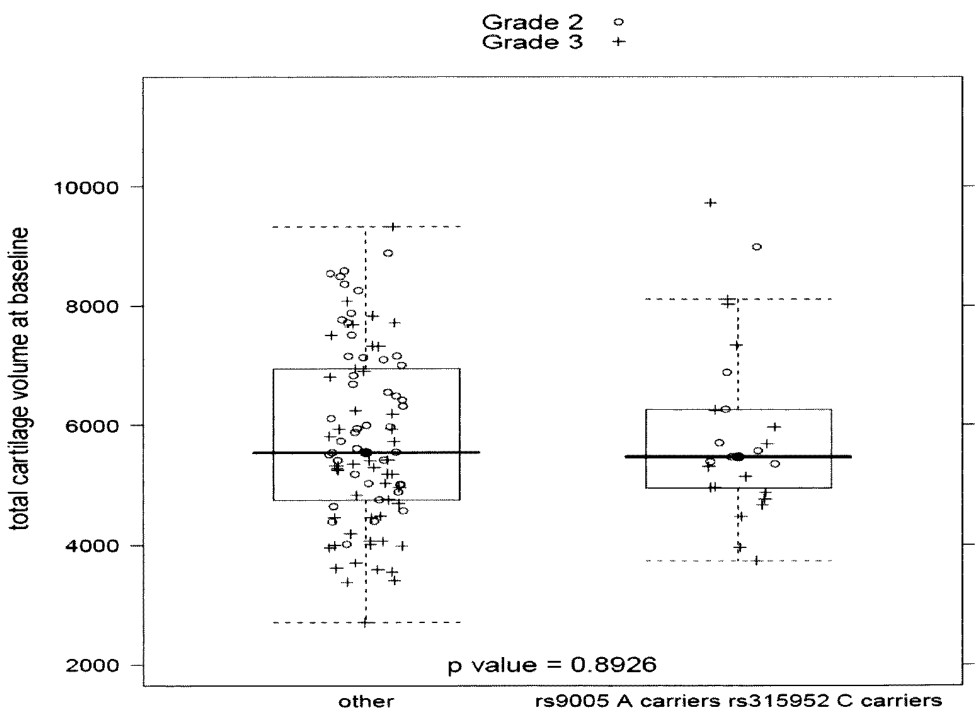
Grade 2
Grade 3
total cartilage volume at baseline
10000
8000
6000
4000
2000
p value = 0.8926
other
rs9005 A carriers rs315952 C carriers

Fig. 9

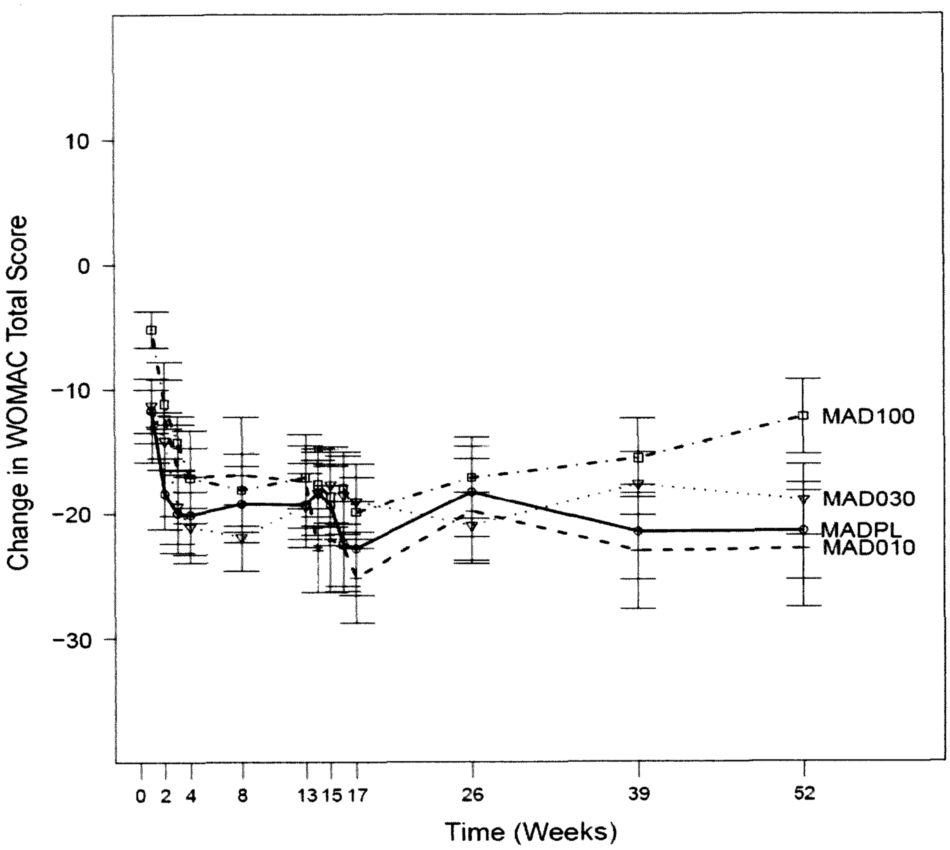
10
0
-10
-20
-30
Change in WOMAC Total Score
0 2 4 8 13 15 17 26 39 52
Time (Weeks)
MAD100
MAD030
MADPL
MAD010

Fig. 10

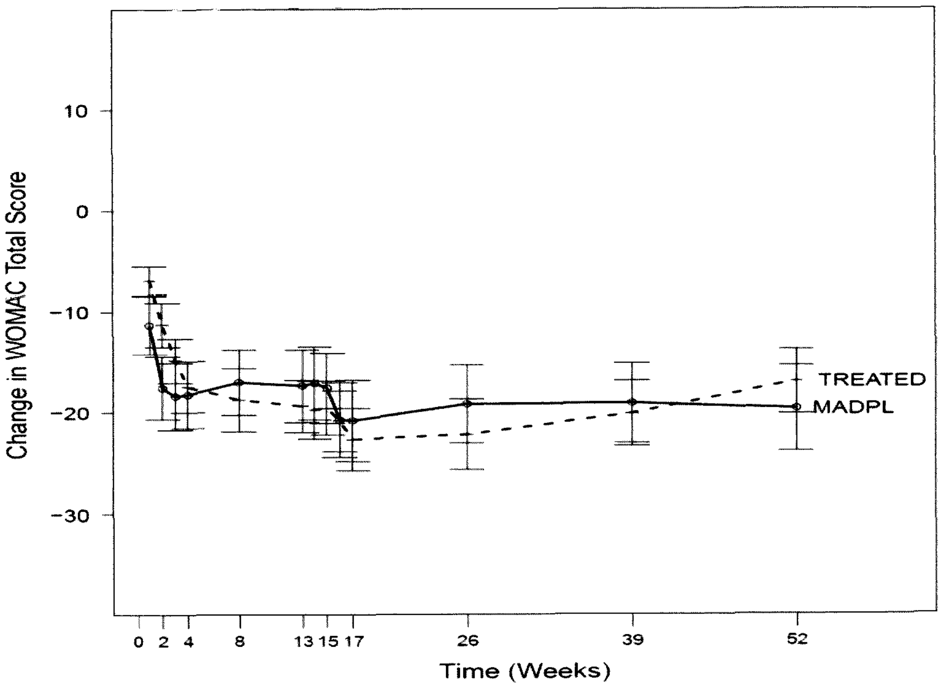
10
0
-10
-20
-30
Change in WOMAC Total Score
0 2 4 8 13 15 17 26 39 52
Time (Weeks)
TREATED
MADPL

Fig. 11

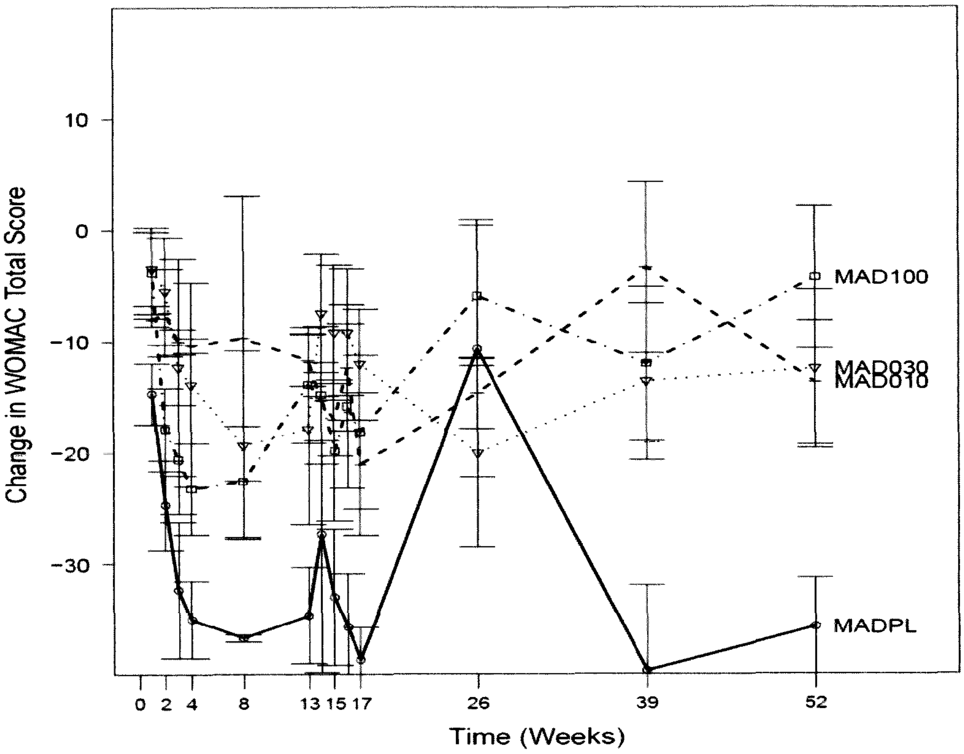
10
0
−10
−20
−30
Change in WOMAC Total Score
MAD100
MAD030
MAD010
MADPL
0 2 4 8 13 15 17 26 39 52
Time (Weeks)

Fig. 12

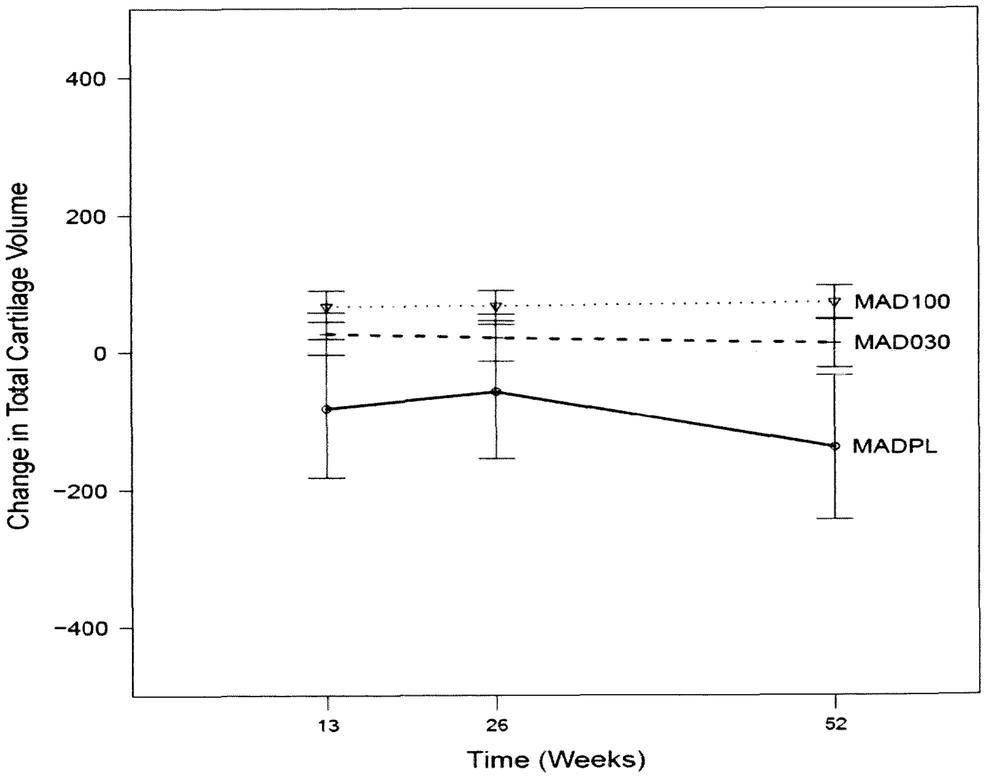
400
200
0
−200
−400
Change in Total Cartilage Volume
MAD100
MAD030
MADPL
13 26 52
Time (Weeks)

Fig. 13

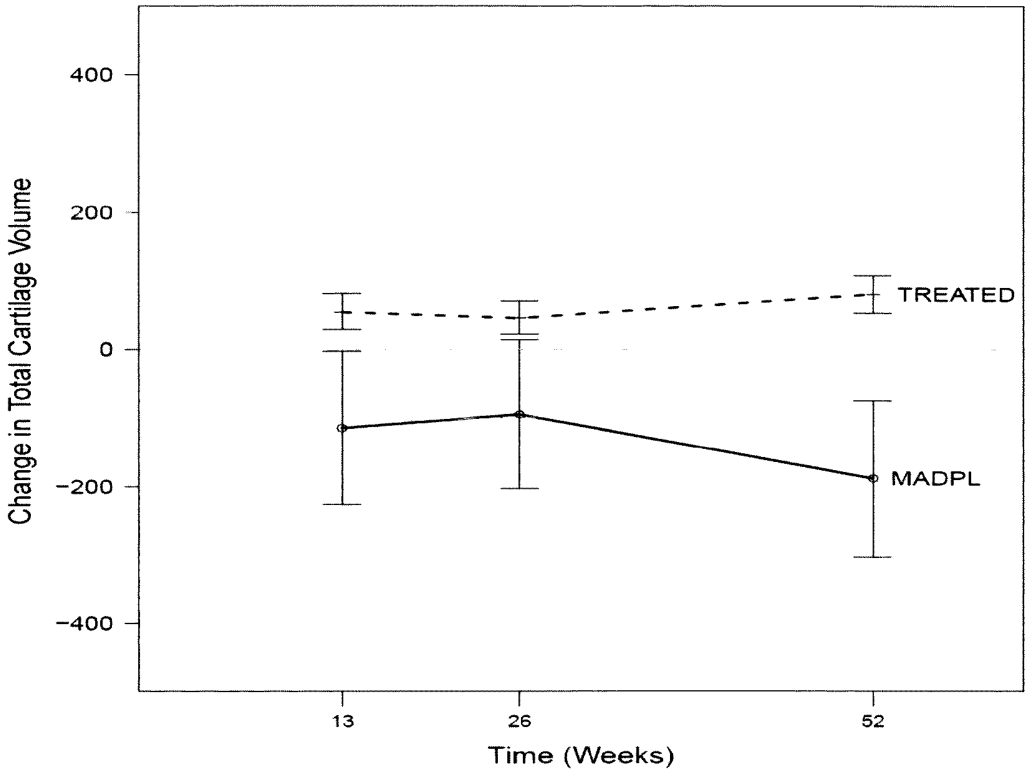
400
200
0
−200
−400
Change in Total Cartilage Volume
TREATED
MADPL
13
26
52
Time (Weeks)

Fig. 14

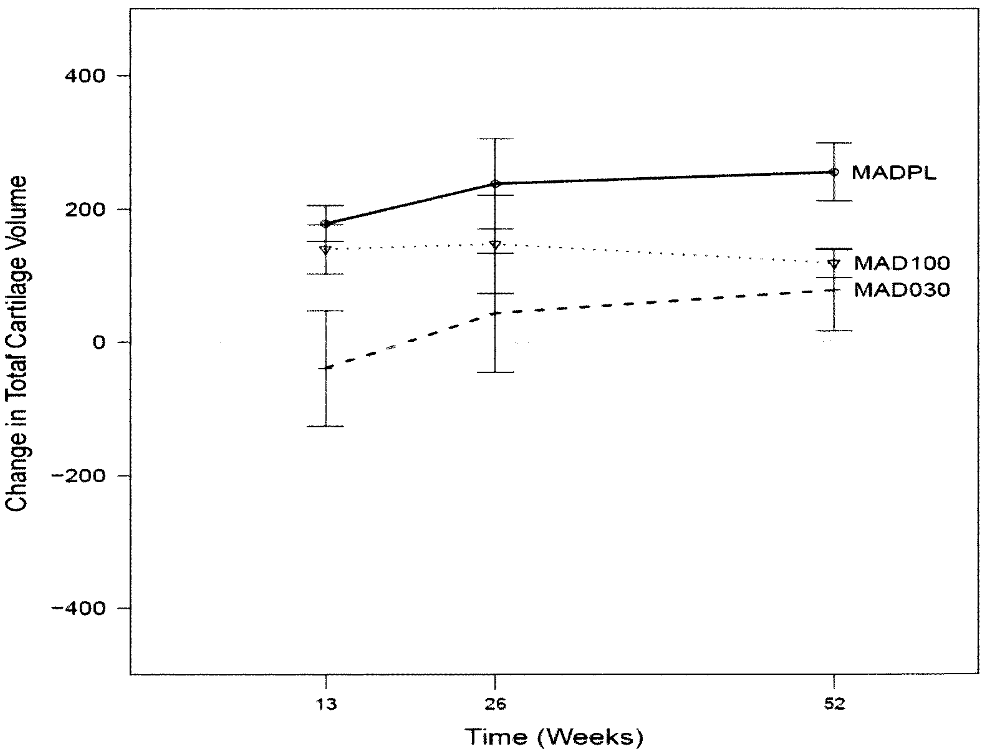
400
200
0
−200
−400
Change in Total Cartilage Volume
MADPL
MAD100
MAD030
13
26
52
Time (Weeks)

Fig. 15

## SEQ ID NO.1: Amino acid sequence of the native human FGF-18

```
Met Tyr Ser Ala Pro Ser Ala Cys Thr Cys Leu Cys Leu His Phe Leu
Leu Leu Cys Phe Gln Val Gln Val Leu Val Ala Glu Glu Asn Val Asp
Phe Arg Ile His Val Glu Asn Gln Thr Arg Ala Arg Asp Asp Val Ser
Arg Lys Gln Leu Arg Leu Tyr Gln Leu Tyr Ser Arg Thr Ser Gly Lys
His Ile Gln Val Leu Gly Arg Arg Ile Ser Ala Arg Gly Glu Asp Gly
Asp Lys Tyr Ala Gln Leu Leu Val Glu Thr Asp Thr Phe Gly Ser Gln
Val Arg Ile Lys Gly Lys Glu Thr Glu Phe Tyr Leu Cys Met Asn Arg
Lys Gly Lys Leu Val Gly Lys Pro Asp Gly Thr Ser Lys Glu Cys Val
Phe Ile Glu Lys Val Leu Glu Asn Asn Tyr Thr Ala Leu Met Ser Ala
Lys Tyr Ser Gly Trp Tyr Val Gly Phe Thr Lys Lys Gly Arg Pro Arg
Lys Gly Pro Lys Thr Arg Glu Asn Gln Gln Asp Val His Phe Met Lys
Arg Tyr Pro Lys Gly Gln Pro Glu Leu Gln Lys Pro Phe Lys Tyr Thr
Thr Val Thr Lys Arg Ser Arg Arg Ile Arg Pro Thr His Pro Ala
```

## SEQ ID NO.2: Amino acid sequence of the recombinant truncated FGF-18 (trFGF-18)

```
Met Glu Glu Asn Val Asp Phe Arg Ile His Val Glu Asn Gln Thr Arg
Ala Arg Asp Asp Val Ser Arg Lys Gln Leu Arg Leu Tyr Gln Leu Tyr
Ser Arg Thr Ser Gly Lys His Ile Gln Val Leu Gly Arg Arg Ile Ser
Ala Arg Gly Glu Asp Gly Asp Lys Tyr Ala Gln Leu Leu Val Glu Thr
Asp Thr Phe Gly Ser Gln Val Arg Ile Lys Gly Lys Glu Thr Glu Phe
Tyr Leu Cys Met Asn Arg Lys Gly Lys Leu Val Gly Lys Pro Asp Gly
Thr Ser Lys Glu Cys Val Phe Ile Glu Lys Val Leu Glu Asn Asn Tyr
Thr Ala Leu Met Ser Ala Lys Tyr Ser Gly Trp Tyr Val Gly Phe Thr
Lys Lys Gly Arg Pro Arg Lys Gly Pro Lys Thr Arg Glu Asn Gln Gln
Asp Val His Phe Met Lys Arg Tyr Pro Lys Gly Gln Pro Glu Leu Gln
Lys Pro Phe Lys Tyr Thr Thr Val Thr Lys
```

Fig. 16(a)

## SEQ ID NO.3: IL1RN gene

```
gggcagctcc accctgggag ggactgtggc ccaggtactg cccgggtgct actttatggg     60
cagcagctca gttgagttag agtctggaag acctcagaag acctcctgtc ctatgaggcc    120
ctccccatgg ctttaggtaa gctccttcca ctctcatttt ttcacctgag aaatgagaga    180
ggaaaatgtc tacaattggt gtttatcaaa tgctttcagg ctctggtgag caagcgtcca    240
ggaaaatgtc aagcgcatgg agctccaggc ctgtctgggg gatctgggca cggggaggca    300
tccatgggag accatgcagg cactctgagg caggggctgc aagcctagtg cctgctgggg    360
cagcaggtga acagagaggt gtaactgctg tgacagaagt catggagtcc ttggagtgtg    420
agggtcattt tccactgttg atagaatagg gaaattggtg aaatagccct gttaaatgag    480
agaaagaaca gtgtgagctc aatgagaaat actaatagaa tgtggcactg agccacaagg    540
tctgagggtt gattgataag gaagggtggg gactgtggag aattaagggc ttggcacagt    600
cagttccacc agttgtcaca agagaatgca ggctcaggtg gccagaactt ctcgcttttc    660
cagaagagtc cgatattctg atttcattat atatagtatt ctgattaaac cagacaataa    720
agcaagcaga taaaatattt aaattataag ctgccagttt gcaacctccg gttaggattt    780
gtgtggggca aagaaaaaaa ctctcaggat cattggtatg tagactctaa ttttaagttt    840
ctaatttaaa attggcccct gaggctgggc gtggtggctc acacctgtaa tcccagcatt    900
ttgggaggcc aaggtgggtg gatctcttga ggtcaagagt tcaaggcctg cctggccaac    960
atggtgaaac cctgtctcta ttaaaaatac aaaaattagc tgggcatggt ggtgcatgtc   1020
tgcaatctta gctacttggg tagctaaggc aggagaattg ctggaacccg ggaggtagag   1080
gttgcagtga atggagatca caccactgca ctccagtctg ggcaatagag agagacgctc   1140
tctctaaaaa aaaatatgta aagataaata aaatgaaata aaataggcct ctaatgagca   1200
ggccattctc ctttctgggt cttactttcc ttgcactcct ttctgggtgt taagaggagg   1260
tctagaggaa gctggacaac tcttagcttg tagtaagcac agtggaagtg tcagctctta   1320
atgggtcatg gacacgttac aagctaggcg ccttgctgag cactttacat ggtttatccc   1380
actgaaccct ctcaataacc ctatgaggaa gggctattat tgctcacatt ttcagaagag   1440
gaaatggata tagagagatt agataatttg cccatggcca gacagctagt ataagaggag   1500
gaggtggatt gactgcagac attctgtctt caaaccacta cactatgcta tgggggcaca   1560
gagacttaat gaaatcatgg agaggggaat tgctttgtca accacaagca gttattccgg   1620
gggcagcaga tcctcccctg tcccccagtg ggtacaatgg tccctggtgg gttgtgctac   1680
aatgttagcc catggtctta tgtgtttttc aaatgtgtaa agtaggatgc tggaaccact   1740
cttagaacca gataccaata cattgtgaag aaataaatct ctgtgcttaa aactggttca   1800
tcccaaaata ttttgaactg acacacaata ggtgctaaat aaatgtgtgt taacttgaat   1860
tggattgaat tcgggaaaaa agtgcaataa gcttagtgaa gacaccatgt tccctgggta   1920
gaggaaccac attctccatc taaggccagg agtatgggag gtatcaatgt ttgcccagca   1980
cagaacaggg tgccaagaag agaaaagttg acggggtgca tactcggact ggaaactgga   2040
agggtgagaa cagagggtaa aggatagaga tggaaccatg tgcatacact ttgtgttacc   2100
ttggacaagt cattcatttc tctggacctc tgctttctct ctacacaatg gggtcccacc   2160
acttccctta cagctgactt gtatgaagaa ggaggtggag gaggaggaga aggtgaagac   2220
aatgctgact caaagggtaa attattttta ggatccaagt ttgaaaacaa ttttaggcta   2280
ctagatatga acaacatctt gattatgtag ttgaaggaaa ttaaagatga atggtttaat   2340
taaaaattaa tcagaatgaa aacgattgat tactaatata tctgcaatgg tttattttcc   2400
tgagtggcag actcactaag gtttttgaat actcctgtgt gattgctcta tgtatgtatg   2460
tatgtatgta tgtatgcatg tatctatcta tctgttgtct aataaaatgg atcacatctc   2520
tgctaataaa aacactacac tggcagggta caattataat cattaactgt gcctggaatt   2580
tgcagcagca gccaccagag gtaccagtgc cctttaaggg ttcataattt agaataatcc   2640
aattatctga gtttttcagg gactgagggg tttggcaagg tgtagaactt tcagtaataa   2700
agtcaagaaa gtcctggaca aaccaaggta gttggtcact ctagtccata accaggtaaa   2760
gagctttccc tgtaacctgt gtaaggtttt agaatcattt ctttccttat taccaaaaat   2820
cctccccaaa ttttcaagaa attatgaact aaatagttac tctatgagat aggagttcag   2880
cccaaaagaa acaccataag aacaaatata attcttgctt atgttaacca tgcaatgaag   2940
cagagagaaa aagtcagtgg cctctttagg aggactgtag tgtgggaaga aataactaaa   3000
ctgggtttca atcctggcct ggccaggatc tggagcaagt gagttaatct ttctaagcct   3060
tgagtagttt cttcttcttc ttcttcttct tcctccccct tctcctcttc ttcttcctcc   3120
tccttctcct cttcttcttc ttcttcttct tcctcttctt cttcttcctc ctcctcctcc   3180
tcctcttctt cttcttcttc ttcctcttcc tcttcctctt cttcctcttc ctcttattct   3240
tcttcatcgt cttcgtcttc gtcttctttt tattttcaaa gtgaaagcaa gtttattaag   3300
aaagtaaagg aataaaagaa tggccactcc atagacagag tagcctgaac cttgagttct   3360
tctataaagt cactatgaat ttatactcat tttgaaagtg ggtgtcaata tgtctgtcca   3420
ctttgcacag ctgttatgtg gacaaaagga gatctgtgtg aaagtgtaac acagagccta   3480
aactataaca ggtaagcaac acagttgtcc ccttccccat ggtgtctgtt cttctccatt   3540
tcctcctgtc tgcaggggga ttataaaact aatcatcaaa gccaagaagg caagagcaag   3600
catgtaccgc tgaaaacaca agataactgc ataagtaatg actttcagtg cagattcata   3660
gctaacccat aaactgctgg ggcaaaaatc atcttggaag gctctgaacc tcagaaagga   3720
ttcacagtaa gttaaccatg tagatctgag aggagagtag cttcttgtag ataacagttg   3780
```

Fig. 16(b)

gattatatac catgtcctga tccccttcat catccaggag agcagaggtg gtcaccctga 3840
tagcagcaag cctgggggct gcagcttggt gggtagaggt actcaggggt acagatgtct 3900
ccaaacctgt cctgctgcct tagggagctt ctaataagtt gatggatttg gttaaaatta 3960
acttggctac ttggcaggac tgggtcagtg aggaccaaca aaaagaagac atcagattat 4020
accctggggg tttgtatttc ttgtgtttct ttctcttctt tgtactaaaa tatttaccca 4080
tgactgggaa agagcaactg gagtctttgt agcattatct tagcaaaaat ttacaaagtt 4140
tggaaaacaa tattgcccat attgtgtggt gtgtcctgtg acactcagga ttcaagtgtt 4200
ggccgaagcc actaaatgtg agatgaagcc attacaaggc agtgtgcaca tctgtccacc 4260
caagctggat gccaacattt cacaaatagt gcttgcgtga cacaaatgca gttccaggag 4320
gcccaaatga aaatgtttgt actgaaattt gttaaagctt cccgacaaac tagatttatc 4380
agtaaggatt gttttctgca agggggatga aacttgtggg gtgagccatt tgggctgagg 4440
aggagggagg ttggagctga gaaatgtgga gacaatttcc ctttagaagg actgaatctc 4500
cctgcctctc tggggtgcgg cagccagcag gatccaatgg tgtatatgtc tccccagctc 4560
cccattcagt gatatcatgt cagtagcttg aaattatccg tggtgggagt attatgtcat 4620
ggaaattggc aaatggaaac ttttattgga gattcaattg ttaaactttt accagcacaa 4680
cactgccctg ccttcagagt caatgaccct atccaagttt aatccatctg tccactgtct 4740
ccaacacgat ctttataaaa cacacctgac aacattaccc ttttattcag tttttaaaa 4800
gataagtttc cagctcatcg ggctggcttt aaaggccatt tctcctctgg acctcaccca 4860
acttttcaaa tcacttttcc tacccctacc tctaaatgct actcaaactc cagccatcct 4920
gaataataag acttttgaaa agtagattat gggctgggca cagtggctca cacctgtaat 4980
cccagcactt tgggaggcca agatgggtgg atcacctgag gtcgggagtt cgagaccagc 5040
ctgactaaca tagtgaaacc ctgtctctac taaaaataca aaattagttg gggtggtgg 5100
cacaagcctg taatcccagc tactcaggag gttgaggcag gggaattgct tgaacctggg 5160
aggcggaggt tgcggtgagc ctagattgct ccactgcact ccagcctggg caacaagagc 5220
gaaactccat ctcaaaaaaa taaataaata aataaagtag attacatcag atacctctgg 5280
cctaggttgt ttatgaccaa ctctcctgct gagaataact agaaaagcta gacaaaacat 5340
atttccaaaa gatctctttg gaggcatcag agaatggcca aggctgtaag gaactgcctg 5400
agcccagaga ggtggagccc agcactggtg ccctttactc ctggggacat gtgctggttt 5460
caaaaacttc agctgagttt ttgagcattc atggaacttg gtgggggaga tgaaatttgt 5520
accttaaatc ctgcctacag ggagggtccc tgataatccc cacccaattt ggaaatctgg 5580
gtcagccttc acaggtactg aagccctcct ctgaatgatc tcaagtcctg ctagggtaga 5640
ggttacctgc ttttgaaagg ctcctggcct acctgtgcag caggagcaaa agtgaaccat 5700
ctcagggtac agataacaat catccagagc cttgaatgac ctctactgtg cttaatatat 5760
agtattcagc agtcagtaaa aaggatttag gcacatgcaa gatgacctgt gtatcaggga 5820
gaaataggca ataaattgag atccagcagg gatttgaatc atggatttga atcaggggca 5880
gccttcgaaa gaactgtgga gaatatactc agatttaaaa cataagattg gaatttttgg 5940
cagagaacta acaactgtac aaaaaaggaa ccaaatggaa atcctagaac tgaaagatgc 6000
aattaaccga tgttgagaaa tagccaacat ctattgaaca cttcccatgt ggacagctgt 6060
gctaaacact ttacaggcat caacataaga tgtgtcccct tacagcagtg cagtgtccct 6120
cctaagacat ggacagcctg gtttccctat ctctctgctt catcaaaacc cctttacgtg 6180
gggcttagac actcctgttg tctctagtgt ctagtagcac agggctcagc acatggaagc 6240
cactagatac aatttgatga ccaggacctc cgatgaaagc catgggtgct gattgggaag 6300
gcattgtctt ttatgtgcta tggtcttaaa gcttcatcca ggaagcagaa ctcggggggt 6360
gctgaggacc cagaaccgag aataagatta gtcagagatt tcctgtgggc agaaatcata 6420
aggacgccaa ctgtttgggt gagataagac gaaaccaaga gtggacttgt ggccagaagc 6480
gtgaggaaga gggagagagc ttcccttgtc ccctttcttc ctctccctaa gccacagtga 6540
ttgacagccc cccccctttg gagtcagagc aggcttgaga ctggactggg aaaggagggt 6600
gggtcaggat acagagcagg aaggctggga gtgcagggca ggagcaaggg gctggggcat 6660
tcattgtgcc tgatctctcc cactttacct ggggtaaaga agcatatgca aaagccacgg 6720
tgtgagtatt tcccaagtgc cagggtcagg gcatgattca tcacgtgcag catttcattc 6780
aatccttata gtaaccgatg atgtggcttc tattattagc tctatcagat aatgaaactg 6840
agaccaagac aggctctgca cattgtgtgg ggtaatgaca caggggggatt cagacctaga 6900
ctccataact cctgccccag ggaccacccc caccctcacc ctgtgcatgt cgacaaagga 6960
cagactgggc cacttctcag gacacagcgg ggaaatgaca cagagcaggg aggttccagg 7020
agccccgagc gtcttttctc caggagaata ctctctgaat tcagactggg gtcagagaaa 7080
catttaccca ggagccgcag tgtgggtggg gctttttact tgaaacgctg tctgaaggca 7140
gtggccagga tggaactctc caccctacct tggcaagcca cttctcttct gcaatctgta 7200
aggacattgt tgagagaatt atggtcttcc aattccggag ggttgaagaa agacaaatag 7260
gagagaacct atcatagtca ggtgctagct gccttctctt tcagagagtg tgagaataaa 7320
gtgatacact tgattattag caaatacttt ggaaatttta aacgctaata ttcaacacac 7380
tctggaagag gcaaataagt agacaggttc atatacatca tctccttcag ctagtcctca 7440
caaaaacaaa caaatgaata aacaaaattc ttctttggcc ctcataggaa gacactgttt 7500
cttgaacgtg tttcaaaaag gatgggtgac tcactcaagg tcacactgtt tatgaggaca 7560
gtacaggaat acagacatgc cattttgcct gaaaaaatcc atcacccagg gaggtgacac 7620

Fig. 16(c)

aattttgcag aaatgttcta tttcctctga aggatacatt ctttaaacct ttgggaaatt 7680
cattcatagt cttcctcctt tgaaggatta actctctgga cacaaagtgt ttgattctga 7740
tttgttggtt ggaagatgtg ttggttgaga gaaagattct gatttgttgg ttgaaaatag 7800
actcatcaag atcaactgct gtagtagtaa atattttgac attttgtctg tattcctgtg 7860
ctgccctcac aagctgcatc accttgagtg agtcattcat actttttgt ttgtttttgt 7920
tttggagatg gagtcttact ctgttgccta ggctggagtg cggtggcgtg atcttggctc 7980
actgcgacct ccatctcctg ggttcaagtg atcctcctgc ctcagcctcc cgagtagctg 8040
ggattacagg cacatgccac catccctgct aatttttgca ttttcagtag agacggagtt 8100
tcaccatgtt ggtcaggttg gtcttgaact cctgacctca ggtgatccgc ccacctcagc 8160
ctccccaagt gctgggatta caggtgtgag ccaccgtgcc cagcccagcc atcatttttg 8220
aaacacgttt gagaaatagt gtcttccttt gagggccaag gagacatttt ttttgtttat 8280
ttgtttgttt ttgtgaggac tagctgaagg gggtgatgta tattaacctg cctacttatt 8340
tgcctcttcc cagagtgtga tgaatattag ggtttaaagt ttctgaagca tttgttaata 8400
aagcccgggg ctggaggtca gaagacctgg atttctctgc atacttttgc catcagcaag 8460
ctgtgtgacc ttggacagat cccttttttg tctaaatctt tctgagtctt cttgaaaaca 8520
atgccaggtt gggacaggat gattgccaag ctcccgtcca gctctaaaac actgcaacgt 8580
atgcttctgc accagcactg tccatcctgt agatcatgca gaaattctct tcaacttttt 8640
cctacccata aaataggagc atgcttacct ttttcctaat gttccaggcc ccgggtctag 8700
aatattgtaa gtaaggaagt taatgtgtat cagagcccat tatgggccag aagttctcct 8760
cttccttcct acacctgctt cctccctccc tccctccctc tttcccttcc ttccttccat 8820
ccatttgtga agaagacatg atcaccctca ttctgagagt gaagagacag aggctcaact 8880
aatgaaatga tttgttcaag gtcacacggg tggcacaagg caagtggcag aggttgaatt 8940
tagacccatt cctgtccaaa tgctgagttt atgtcatcgt cccgagacca taactttaaa 9000
gatgtaagat agtgggaaaa gagttgattt caaagcacct ctcagaagga ctcactttac 9060
atcaggggtc agcagactca ggccaaatcc ggtccattcc ccgcttttgc aaagaaagtt 9120
gtagtggaac acagctaggc ttattgattt atggattgcc aacgtccttt tgtgaaacag 9180
acagctgagc tgagtaatcg tggcgcacaa aacctaaaat atttactatc tcgtccttta 9240
cagaatgttt gccaatctat ggtccggagt ccaaggctgt ccatttttca aagaacacaa 9300
agtgacatga gactgtccca tgtgcaggga gccctatcat tttattatga aaaaacggcc 9360
tttctgctca aatctgtttt ttaaaaagtc aacaaacaga ctctgggtac ctgtcaggaa 9420
cagtagggag tttggtttcc attgtgctct tcttcccagg aactcaatga aggggaaata 9480
gaaatcttaa ttttggggaa attgcacagg ggaaaaaggg gagggaatca gttacaacac 9540
tccattgcga cacttagtgg ggttgaaagt gacaacagca agggtttctc tttttggaaa 9600
tgcgaggagg gtatttccgc ttctcgcagt ggggcagggt ggcagacgcc tagcttgggt 9660
gagtgactat ttctttataa accacaactc tgggcccgca atggcagtcc actgccttgc 9720
tgcagtcaca gaatggaaat ctgcagaggc ctccgcagtc acctaatcac tctcctcctc 9780
ttcctgttcc attcagagac gatctgccga ccctctggga gaaaatccag caagatgcaa 9840
gccttcaggt aaggctaccc caaggaggag aaggtgaggg tggatcagct ggagactgga 9900
aacatatcac agctgccagg ggctgccagg ccccagaggg cctgagaact gggtttgggc 9960
tggagaggat gtccattatt caagaaagag gctgttacat gcatgggctt caggacttgt 10020
gtttcaaaat atcccagatg tggatagtgc gaccggaggg ctgtcttact ttcccagaga 10080
ctcaggaacc cagtgagtaa tagatgcatg ccaaggagtg ggactgcgat tcaggcctag 10140
ttgaatgtgc tgacagagaa gcagagaggg gcaccagggg cacagcccga aggcccagac 10200
tgatatgggc aaggcctgtc tgtgctgaca tgtcggaggg tcccactctc cagggacctt 10260
ggtttccccg tctgtgacat ctgtgacatg agagtcacga taactccttg tgtgccttac 10320
agggttgttg tgaaaattaa atgcacagat aatagcgtaa cagtattccg tgcattgtaa 10380
agagcctgaa aaccattatg atttgaaaat ggaatcggct ttgtgagacc atcactattg 10440
taaagatgtg atgctgatag aaatgacagg actgcttgtg catgccctct gcagtgtgac 10500
attccagcag tgaaatcatg ttggggtgac ttctccccca ctctgacctt tatgtttgtc 10560
tgggccgagg ctgcaagtcg ggctctgtgg gtgtatgagt gacaagtctc tcccttccag 10620
atatggggac tgtctgcttc cctaggttgc ctctccctgc tctgatcagc tagaagctcc 10680
aggagatcct cctggaggcc ccagcaggtg atgtttatcc ctccagactg aggctaaatc 10740
tagaaactag gataatcaca aacaggccaa tgctgccata tgcaaagcac tttggtttgc 10800
ctggccaccc ctcgtcgagc atgtgggctc ttcagagcca cctgatgagg tgggtacagt 10860
tagccacact tcacaggtga agaggtgagg cacaggtccc aggtcaggct ggccagagct 10920
ctgtttatta cgtctcacag ctttgagtcc tgctctcaac cagagaggcc ctttaccaag 10980
aagaaaggat tgggacccag aatcaggtca ctggctgagg tagagaggaa gccgggttgt 11040
tcccaagggt agctgctcct gcaggactct gagcaggtca ccagctaatg gaggaaaggc 11100
tctagggaaa gacccttctg gtctcagact cagagcgagt tagctgcaag gtgttccgtc 11160
tcttgaaact tctacctagg tgctatggta gccactagtc tcaggtggct atttaaattt 11220
atacttaaat gaatgaaaat agaagaaaat ttaaaatcca gacccttggt cacactatcc 11280
acatttaaag aggtcaatag ccacatgtgg ttagtggcca ccctattggg cagtgcagct 11340
acagaacatt tttgcatccc agaaagttct tttggatgtt gctgctctac agcatgcttt 11400
gctgaaacag aagtgccttc cctgggaatc tcagatggga agcaagtaag gaggggagtc 11460
aaatgtgggc tcactgctca ccagctgtga gggttgggcc tgcctcttaa ccattgtcag 11520

Fig. 16(d)

cctcagtctt ctcatccatg catgccgtgg gtatactaaa atactatacc cctggaagag 11580
ctggatgcaa atttgacaag ttctggggga cacaggaagg tgccaagcac aaggctgggc 11640
acatggtggc tgtgcactac agctgagtcc ttttcctttt cagaatctgg gatgttaacc 11700
agaagacctt ctatctgagg aacaaccaac tagttgctgg atacttgcaa ggaccaaatg 11760
tcaatttaga aggtgagtgg ttgccaggaa agccaatgta tgtgggcatc acgtcacttt 11820
gcccgtctgt ctgcagcagc atggcctgcc tgcacaaacc ctaggtgcaa tgtcctaatc 11880
cttgttgggt ctttgtattc aagtttgaag ctgggagggc ctggctactg aagggcacat 11940
atgagggcag cctgaagagg gtgtggagag gtagagtcta ggtcagaggt cagtgcctat 12000
aggcacagtg gtcccagggc cacagctggg aagggcaaat accagaaggc aaggttgacc 12060
attcccttcc tcaagtgcct attaaggctc catgttccta tgttgttcaa accctaactc 12120
aatcccaaat taatccacca tgtataaggt tgagctatgt ctcttattcc tggacaccat 12180
actcagccat attctggtcc acacattaaa caagctggat gaccttgaag aagcttcacc 12240
cactctgttc ctcagctttc ccttcagtgg gatgatatca actggacaac aggatgtgcg 12300
attcttttag ttccagcctt ccaggatgtt ttcactcccc tgtttgttgt tgtaggatgg 12360
tattacctcc accttcccac cttccctatg ccctggttct gtctcctgtg cctcgctctg 12420
aaagtggatg agacctacaa ttcctgtcct ggtagttctc ctaatgaaca cactgaagca 12480
cgaggaagct gagatttttg ttgctacatg agagcatgga ggcctcttag ggagagagga 12540
ggttcagaga ctcctaggct cctgtggagc cccactcatg gccttgttca ttttccctgc 12600
ccctcagcaa cactcctatt gacctggagc acaggtatcc tggggaaagt gagggaaata 12660
tggacatcac atggaacaac atccaggaga ctcaggcctc taggagtaac tgggtagtgt 12720
gcatcctggg gaaagtgagg gaaatatgga catcacatgg aacaacatcc aggagactca 12780
ggcctctagg agtaactggg tagtgtgcat cctggggaaa gtgagggaaa tatggacatc 12840
acatggaaca acatccagga gactcaggcc tctaggagta actgggtagt gtgcatcctg 12900
gggaaagtga gggaaatatg gacatcacat ggaacaacat ccaggagact caggcctcta 12960
ggagtaactg ggtagtgtgc ttggtttaat cttctattta cctgcagacc aggaagatga 13020
gacctctctg cccttctgac ctcgggattt tagttttgtg gggaccaggg gagatagaaa 13080
aatacccggg gtctcttcat tattgctgct tcctcttcta ttaacctgac cctcccctct 13140
gttcttcccc agaaaagata gatgtggtac ccattgagcc tcatgctctg ttcttgggaa 13200
tccatggagg gaagatgtgc ctgtcctgtg tcaagtctgg tgatgagacc agactccagc 13260
tggaggtaaa aacatgcttt ggatctcaaa tcaccccaaa acccagtggc ttgaaacaac 13320
caaaattttt tcttatgatt ctgtgggttg accaggatta gctgggtagt tctgttccat 13380
gtggtggaac atgctggggt cactttggaa gctgcattca gcagagtgcc tggcttgcgc 13440
tgggcatcca aggtggtccc tcatcctcca ggctctcttt ccatgtgatc tctcagtgtt 13500
taagagttag ttggagcttc cttacagcat ggcggctgac ttccaaaagg gattattcca 13560
aaaagagcct caacatgcag gcgcttatta tgacttctgc ttgcatcatc ctattggcca 13620
aagccagtca cgtggctaag tctagccccc tgtgagagga gactgcataa gagtgtgaac 13680
accaggagac acggtcactg ggggccacca ctgtaaccat ctaccacagg acctgaatct 13740
ctgtgtgcta ctcccttgct caagggcccc cctacccacg cagacctgct gtcttctagc 13800
aaagcccatc ctcaggacct ttctcttcca atccttattg actcaaattg attagttggt 13860
gctccaccca gagccctgtg ctcctttatc tcatgtaatg ttaatgggtt tcccagccct 13920
gggaaaacat ggctttgtct caggggcttg ctggatgcaa gcttaacctc aatgtgagtg 13980
gccatactgt ggcactgtcc catccctcac cagggacact gttctggagg gtgactgcct 14040
gttctgtgag gagtggggat ggctaggaca ttgcatggaa cacaccacca ccccatcttc 14100
tcagagctca aaccctgaca gaacaccagc tccacaggcc ttggcttctg ctgatggtgc 14160
cgtgtattta ccagacttag tggtccaagg ccagagtggc cagatttccc aaagtcaagg 14220
tgtgacagtg ggacagcctc tttgtgtctt tgctgtccta agaaacctgg gccaggccag 14280
gcgcagtggc tcacgcctgt aatcccagca ctttgagaag ccaaggtggg cagatcacga 14340
ggtcaggagt ttgagaccag cctggccaac atggtgaaac cctgtctcta ttaaaaatag 14400
aaaacattag acaggtgtgg tggtgcatgc ctgtaatccc agctactcag gaggctgagg 14460
caggagaatc gcttgaaccc aggaggtgga ggttgcagtg agccgagatt gtgccactgc 14520
actccagcct aggcgacaga gcaagactcc gtctcgggaa aattaattaa taaataaata 14580
aacctaggtc ccagagtccc acagaatggc agacaggagc acctgggggc ttttagggta 14640
tggcatttcc cctgtactaa ctctgggctg tccagagggc catttcatgg cgtggagtgg 14700
agagggaggc agcacaggac ttcctaggcc tcagctctca cctgcccatc ttttgatttc 14760
caggcagtta acatcactga cctgagcgag aacagaaagc aggacaagcg cttcgccttc 14820
atccgctcag acagtggccc caccaccagt tttgagtctg ccgcctgccc cggttggttc 14880
ctctgcacag cgatggaagc tgaccagccc gtcagcctca ccaatatgcc tgacgaaggc 14940
gtcatggtca ccaaattcta cttccaggag gacgagtagt actgcccagg cctgcctgtt 15000
cccattcttg catggcaagg actgcaggga ctgccagtcc ccctgcccca gggctcccgg 15060
ctatgggggc actgaggacc agccattgag gggtggaccc tcagaaggcg tcacaacaac 15120
ctggtcacag gactctgcct cctcttcaac tgaccagcct ccatgctgcc tccagaatgg 15180
tctttctaat gtgtgaatca gagcacagca gcccctgcac aaagcccttc catgtcgcct 15240
ctgcattcag gatcaaaccc cgaccacctg cccaacctgc tctcctcttg ccactgcctc 15300
ttcctccctc attccacctt cccatgccct ggatccatca ggccacttga tgacccccaa 15360

Fig. 16(e)

```
ccaagtggct cccacaccct gttttacaaa aaagaaaaga ccagtccatg agggaggttt    15420
ttaagggttt gtggaaaatg aaaattagga tttcatgatt tttttttttc agtccccgtg    15480
aaggagagcc cttcatttgg agattatgtt ctttcgggga gaggctgagg acttaaaata    15540
ttcctgcatt tgtgaaatga tggtgaaagt aagtggtagc ttttcccttc tttttcttct    15600
ttttttgtga tgtcccaact tgtaaaaatt aaaagttatg gtactatgtt agccccataa    15660
tttttttttt ccttttaaaa cacttccata atctggactc ctctgtccag gcactgctgc    15720
ccagcctcca agctccatct ccactccaga ttttttacag ctgcctgcag tactttacct    15780
cctatcagaa gtttctcagc tcccaaggct ctgagcaaat gtggctcctg gggttcttt     15840
cttcctctgc tgaaggaata aattgctcct tgacattgta gagcttctgg cacttggaga    15900
cttgtatgaa agatggctgt gcctctgcct gtctccccca ccgggctggg agctctgcag    15960
agcaggaaac atgactcgta tatgtctcag gtccctgcag ggccaagcac ctagcctcgc    16020
tcttggcagg tactcagcga atgaatgctg tatatgttgg gtgcaaagtt ccctacttcc    16080
tgtgacttca gctctgtttt acaataaaat cttgaaaatg ccta                      16124
```

Fig. 16(f)

## SEQ ID NO.4: IL1RN rs9005 locus

```
aattaggatt tcatgatttt ttttttcag tccccgtgaa ggagagccct tcatttggag      60
attatgttct ttcggggaga ggctgaggac ttaaaatatt cctgcatttg tgaaatgatg    120
gtgaaagtaa gtggtagctt ttcccttctt tttcttcttt ttttgtgatg tcccaacttg    180
taaaaattaa aagttatggt actatgttag ccccataatt tttttttcc ttttaaaaca     240
cttccataat ctggactcct ctgtccaggc actgctgccc agcctccaag ctccatctcc    300
actccagatt ttttacagct gcctgcagta ctttacctcc tatcagaagt ttctcagctc    360
ccaaggctct gagcaaatgt ggctcctggg ggttctttct tcctctgctg aaggaataaa    420
ttgctccttg acattgtaga gcttctggca cttggagact tgtatgaaag atggctgtgc    480
ctctgcctgt ctccccccacc rggctgggag ctctgcagag caggaaacat gactcgtata   540
tgtctcaggt ccctgcaggg ccaagcacct agcctcgctc ttggcaggta ctcagcgaat    600
gaatgctgta tatgttgggt gcaaagttcc ctacttcctg tgacttcagc tctgttttac    660
aataaaatct tgaaaatgcc tatattgttg actatgtcct tggccttgac aggctttggg    720
tatagagtgc tgaggaaact gaaagaccaa tgtgtctttc ttaccccaga ggctggcgcc    780
tggcctcttc tctgagagtt cttttcttcc ttcagcctca ctctccctgg ataacatgag    840
agcaaatctc tctgcaaaaa agatatgggg cagcactgtc cacaacagcc tctgctggaa    900
acaacccaag cacccatcac agaatgaatt agtacatcat gtatctgcac acaacacagt    960
gctccttggc aaagaaaatg aatgaattac agccagctgc a                       1001
```

## SEQ ID NO.5: IL1RN rs315952 locus

```
tcacgaggtc aggagtttga gaccagcctg gccaacatgg tgaaaccctg tctctattaa     60
aaatagaaaa cattagacag gtgtggtggt gcatgcctgt aatcccagct actcaggagg    120
ctgaggcagg agaatcgctt gaacccagga ggtggaggtt gcagtgagcc gagattgtgc    180
cactgcactc cagcctaggc gacagagcaa gactccgtct cgggaaaatt aattaataaa    240
taaataaacc taggtcccag agtcccacag aatggcagac aggagcacct gggggctttt    300
agggtatggc atttcccctg tactaactct gggctgtcca gagggccatt tcatggcgtg    360
gagtggagag ggaggcagca caggacttcc taggcctcag ctctcacctg cccatctttt    420
gatttccagg cagttaacat cactgacctg agcgagaaca gaaagcagga caagcgcttc    480
gccttcatcc gctcagacag yggccccacc accagttttg agtctgccgc ctgccccggt    540
tggttcctct gcacagcgat ggaagctgac cagcccgtca gcctcaccaa tatgcctgac    600
gaaggcgtca tggtcaccaa attctacttc caggaggacg agtagtactg cccaggcctg    660
cctgttccca ttcttgcatg gcaaggactg cagggactgc cagtcccccct gccccagggc   720
tcccggctat gggggcactg aggaccagcc attgaggggt ggaccctcag aaggcgtcac    780
aacaacctgg tcacaggact ctgcctcctc ttcaactgac cagcctccat gctgcctcca    840
gaatggtctt tctaatgtgt gaatcagagc acagcagccc ctgcacaaag cccttccatg    900
tcgcctctgc attcaggatc aaaccccgac cacctgccca acctgctctc ctcttgccac    960
tgcctcttcc tccctcattc caccttccca tgccctggat c                       1001
```

Fig. 16(g)

**SEQ ID NO.6: Specific region from IL1RN rs9005 locus (corresponding to nucleotide 415 to nucleotide 466 of SEQ ID NO.4), wherein N is A or G**

```
<221>  variation
<222>  (27)..(27)
<223>  n is a or g
ctgtgcctct gcctgtctcc cccaccnggc tgggagctct gcagagcagg aa          52
```

**SEQ ID NO.7: Specific region from IL1RN rs315952 locus (corresponding to nucleotide 415 to nucleotide 466 of SEQ ID NO.5), wherein N is C or T**

```
<221>  variation
<222>  (27)..(27)
<223>  n is c or t
cgcttcgcct tcatccgctc agacagnggc cccaccacca gttttgagtc tg          52
```

**SEQ ID NO.8: rs315952 primer 1**

```
gcttcgcctt catccgctca gacag                                        25
```

**SEQ ID NO.9: rs315952 primer 2**

```
ggccccacca ccagttttga gtctg                                        25
```

**SEQ ID NO.10: rs9005 primer 1**

```
tgtgcctctg cctgtctccc ccacc                                        25
```

**SEQ ID NO.11: rs9005 primer 2**

```
ggctgggagc tctgcagagc aggaa                                        25
```

Fig. 16(h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008023063 A
- WO 2004032849 A
- WO 9816644 A
- WO 2006063362 A
- WO 2009135218 A
- US 2012115137 A
- WO 9215712 A
- US 5679524 B
- WO 9102087 A
- WO 9009455 A
- WO 9517676 A
- US 5302509 A
- US 5945283 A
- US 5605798 A
- WO 8910414 A


### Non-patent literature cited in the description


- The Merck Manual. 449
- **LI Y et al.** *MACH software,* 2010
- **BENJAMINI ; HOCHBERG.** *J. of the Royal Statistical Society Series,* 1995, vol. B (57), 289
- **LOTZ.** *Arthritis research therapy,* 2010, vol. 12, 211
- **ELLSWORTH et al.** *Osteoarthritis and Cartilage,* 2002, vol. 10, 308-320
- **SHIMOAKA et al.** *J. Bio. Chem.,* 2002, vol. 277 (9), 7493-7500
- **BELLAMY et al.** *J.Rheumatology,* 1988, vol. 15, 1833-1840
- **WOLFE.** *Rheumatology,* 1999, vol. 38, 355-361
- **ATTUR et al.** *Ann. Rheum. Dis.,* 2010, vol. 69, 856-861
- **LI et al.** *Genet Epidemiol,* 2010, vol. 34, 816-834
- **WIRAPATI et al.** *Ann. Hum. Genet.,* 2011, vol. 75 (1), 133-45
- **BATESON W. ; MENDEL G.** G. Mendel's principles of heredity. Cambridge University Press, 1909
- **PHILLIPS PC.** *Genetics,* 1998, vol. 7;149 (3), 1167-71
- **CORDELL HJ.** *Hum. Mol. Genet.,* 2002, vol. 11 (20), 2463-8
- **FISHER RA.** *The Correlation Between Relatives on the Supposition of Mendelian Inheritance,* 1918, http://digital.library.adelaide.edu.au/dspace/handle/2440/15097
- **BROWNING SR ; BROWNING BL.** *Nature Reviews Genetics,* 2011, vol. 12 (10), 703-14